# EUROPEAN PATENT SPECIFICATION

(11) **EP 1 230 345 B1**
(45) Date of publication and mention of the grant of the patent: **11.06.2008**
(21) Application number: 00980418.8
(22) Date of filing: 14.11.2000
(51) Int. Cl.: C12N 15/82, C07K 14/415, A01H 5/00

(54) **ENVIRONMENTAL STRESS TOLERANCE GENES**
TOLERANZGENE FÜR UMWELTSTRESS
GENES RESISTANT AU STRESS ENVIRONNEMENTAL

(30) Priority: 17.11.1999 US 166228 P; 17.04.2000 US 197899 P; 22.08.2000 US 227439 P
(43) Date of publication of application: 14.08.2002
(60) Divisional application: 08006255.7
(73) Proprietor: Mendel Biotechnology, Inc., Hayward, CA 94545-3720 (US)
(72) Inventor: PINEDA, Omaira, Vero Beach, FL 32960 (US); YU, Guo-Liang, Berkeley, CA 94705 (US); CREELMAN, Robert, Castro Valley, CA 94546 (US); RIECHMANN, Jose, Luis, Pasadena, CA 91101 (US); HEARD, Jacqueline, Stonington, CT 06378-1722 (US); RATCLIFFE, Oliver, Oakland, CA 94606 (US); REUBER, Lynne, San Mateo, CA 94402 (US); KEDDIE, James, San Mateo, CA 94402 (US)
(74) Representative: Brasnett, Adrian Hugh
(86) International application number: PCT/US2000/031458
(87) International publication number: WO 2001/036598

(56) References cited:
- EP-A- 0 803 572
- EP-A- 1 033 405
- WO-A-99/53016
- WO-A1-01/35727
- WO-A1-98/37184
- WO-A1-98/37755
- WO-A1-98/58069
- WO-A2-00/53724
- DE-C1- 19 503 359
- US-A- 5 892 009
- DATABASE EMBL 4 August 1997 (1997-08-04), ROUNSLEY S D ET AL.: "Arabidopsis thaliana chromosome II BAC T13E15 genomic sequence, complete sequence." XP002303794 Database accession no. AC002388
- KASUGA MIE ET AL: "Improving plant drought, salt, and freezing tolerance by gene transfer of a single stress-inducible transcription factor" NATURE BIOTECHNOLOGY, NATURE PUB. CO, NEW YORK, NY, US, vol. 17, no. 3, March 1999 (1999-03), pages 287-291, XP002173128 ISSN: 1087-0156
- LIU QIANG ET AL: "Two transcription factors, DREB1 and DREB2, with an EREBP/AP2 DNA binding domain separate two cellular signal transduction pathways in drought-and low-temperature-responsive gene expression, respectively, in Arabidopsis" PLANT CELL, AMERICAN SOCIETY OF PLANT PHYSIOLOGISTS, ROCKVILLE, MD, US, vol. 10, no. 8, August 1998 (1998-08), pages 1391-1406, XP002145075 ISSN: 1040-4651
- OHME-TAKAGI M AND SHINSHI H: "Ethylene-inucible DNA binding proteins that interact with an ethylene-responsive element" PLANT CELL, AMERICAN SOCIETY OF PLANT PHYSIOLOGISTS, ROCKVILLE, MD, US, vol. 7, no. 2, February 1995 (1995-02), pages 173-182, XP002108954 ISSN: 1040-4651
- BÜTTNER M AND SINGH K: "Arabidopsis thaliana ethylene-responsive element binding protein (AtEBP), an ethylene-inducible, GCC box DNA-binding protein interacts with an ocs element binding protein" PROCEEDINGS OF THE NATIONAL ACADEMY OF SCIENCES OF USA, NATIONAL ACADEMY OF SCIENCE. WASHINGTON, US, vol. 94, no. 11, 27 May 1997 (1997-05-27), pages 5961-5966, XP002108953 ISSN: 0027-8424
- WINICOV I: "New molecular approaches to improving salt tolerance in crop plants" ANNALS OF BOTANY, ACADEMIC PRESS, LONDON, GB, vol. 82, no. 6, December 1998 (1998-12), pages 705-710, XP001007288 ISSN: 0305-7364
- URAO T ET AL: "AN ARABIDOPSIS MYB HOMOLOG IS INDUCED BY DEHYDRATION STRESS AND ITSGENE PRODUCT BINDS TO THE CONSERVED MYB RECOGNITION SEQUENCE" PLANT CELL, AMERICAN SOCIETY OF PLANT PHYSIOLOGISTS, ROCKVILLE, MD, US, vol. 5, November 1993 (1993-11), pages 1529-1539, XP002938159 ISSN: 1040-4651
- DATABASE EMBL [Online] 9 April 1999 (1999-04-09), "Arabidopsis thaliana genomic DNA, chromosome 5, P1 clone:MNJ7." XP002315550 retrieved from EBI accession no. EM_PRO:AB025628 Database accession no. AB025628
- DATABASE EMBL [Online] 12 September 1997 (1997-09-12), "Arabidopsis thaliana mRNA for Hap3b transcription factor" XP002315551 retrieved from EBI accession no. EM_PRO:Y13724 Database accession no. Y13724 -& EDWARDS DAVID ET AL: "Multiple genes encoding the conserved CCAAT-box transcription factor complex are expressed in arabidopsis" PLANT PHYSIOLOGY, AMERICAN SOCIETY OF PLANT PHYSIOLOGISTS, ROCKVILLE, MD, US, vol. 117, no. 3, July 1998 (1998-07), pages 1015-1022, XP002136431 ISSN: 0032-0889
- DATABASE EMBL [Online] 5 February 1992 (1992-02-05), "Z.mays mRNA for CAAT-box DNA binding protein subunit B (NF-YB)" XP002315552 retrieved from EBI accession no. EM_PRO:X59714 Database accession no. X59714
- WINICOV ILGA ET AL: "Transgenic overexpression of the transcription factor Alfin1 enhances expression of the endogenous MsPRP2 gene in alfalfa and improves salinity tolerance of the plants" PLANT PHYSIOLOGY, AMERICAN SOCIETY OF PLANT PHYSIOLOGISTS, ROCKVILLE, MD, US, vol. 120, no. 2, June 1999 (1999-06), pages 473-480, XP002179695 ISSN: 0032-0889
- LEE J H ET AL: "DEREPRESSION OF THE ACTIVITY OF GENETICALLY ENGINEERED HEAT SHOCK FACTOR CAUSES CONSTITUTIVE SYNTHESIS OF HEAT SHOCK PROTEINS AND INCREASED THERMOTOLERANCE IN TRANSGENIC ARABIDOPSIS" PLANT JOURNAL, BLACKWELL SCIENTIFIC PUBLICATIONS, OXFORD, GB, vol. 8, no. 4, 1 October 1995 (1995-10-01), pages 603-612, XP002003021 ISSN: 0960-7412
- PRAENDL R ET AL: "HSF3, a new heat shock factor from Arabidopsis thaliana, derepresses the heat shock response and confers thermotolerance when overexpressed in transgenic plants" MOLECULAR AND GENERAL GENETICS, SPRINGER VERLAG, BERLIN, DE, vol. 258, May 1998 (1998-05), pages 269-278, XP002135096 ISSN: 0026-8925
- DATABASE EMBL [Online] 9 November 1999 (1999-11-09), "Arabidopsis thaliana heme activated protein (HAP5c) mRNA, complete cds." XP002315554 retrieved from EBI accession no. EM_PRO:AF193440 Database accession no. AF193440
- DATABASE EMBL [Online] 27 January 1997 (1997-01-27), "Sequence of BAC F7G19 from Arabidopsis thaliana chromosome 1, complete sequence." XP002315553 retrieved from EBI accession no. EM_PRO:AC000106 Database accession no. AC000106
- HOLLUNG KRISTIN ET AL: "Developmental, stress and ABA modulation of mRNA levels for bZip transcription factors and Vp1 in barley embryos and embryo-derived suspension cultures" PLANT MOLECULAR BIOLOGY, vol. 35, no. 5, 1997, pages 561-571, XP002315582 ISSN: 0167-4412
- DATABASE GENEMBL, GENBANK [Online] USPTO (ARLINGTON, VA, USA) LIN ET AL.: 'Sequence and analysis of chromosome 2 of the plant arabidopsis thaliana', XP002938725 Retrieved from stic Database accession no. AC002388 & NATURE vol. 402, 1999, pages 761 - 768
- DATABASE GENBANK [Online] USPTO (ARLINGTON, VA, USA) ASAMIZU ET AL.: 'A large scale analysis of cDNA in arabidopsis thaliana: generation of 12,028 non-redundant expressed sequence tags from normalized and size-selected cDNA libraries', XP002938726 Retrieved from stic Database accession no. AV552445 & DNA RESEARCH vol. 7, 2000, pages 175 - 180
- DATABASE GENBANK [Online] USTPO (ARLINGTON, VA, USA) 08 September 1999 CHEN ET AL., XP002938727 Retrieved from STIC Database accession no. AI997809
- DATABASE GENBANK [Online] USPTO (ARLINGTON, VA, USA) NEWMAN ET AL.: 'Genes galore: A summary of methods for accessing results from large-scale partial sequencing of anonymous arabidopsis cDNA clones', XP002938728 Retrieved from STIC Database accession no. N97133 & PLANT PHYSIOLOGY vol. 106, 1994, pages 1241 - 1255
- DATABASE GENBANK [Online] ASPTO (ARLINGTON, VA, USA) NEWMAN ET AL.: 'Genes galore: A summary of methods for accessing results from large-scale partial sequencing of anonymous arabidopsis cDNA clones', XP002938729 Retrieved from STIC Database accession no. AA598183 & PLANT PHYSIOLOGY vol. 106, 1994, pages 1241 - 1255
- DATABASE PIR_66 [Online] USPTO (ARLINGTON, VA, USA) 01 February 1999 ROUNSLEY ET AL., XP002938730 Retrieved from STIC Database accession no. T00409
- DATABASE SPTREMBL_15 [Online] USPTO (ARLINGTON, VA, USA) 01 January 1998 ROUNSLEY ET AL., XP002938743 Retrieved from STIC Database accession no. 022167
- RIECHMANN ET AL.: 'Arabidopsis transcription factors: genome-wide comparative analysis among eukaryotes' SCIENCE vol. 290, 15 December 2000, pages 2105 - 2110, XP002938731
- SUNG ET AL.: 'Developmentally regulated expression of two MADS-box genes, MdMADS3 and MdMADS4, in the morphogenesis of flower buds and fruits in apple' PLANTA vol. 210, March 2000, pages 519 - 528, XP002938732
- RIECHMANN ET AL.: 'A genomic perspective on plant transcription factors' CURRENT OPINION IN PLANT BIOLOGY vol. 3, October 2000, pages 423 - 434, XP002938733
- RATCLIFFE ET AL.: 'Separation of shoot and floral identity in arabidopsis' DEVELOPMENT vol. 126, March 1999, pages 1109 - 1120, XP002938734
- SUNG ET AL.: 'Characterization of MdMADS2, a member of the AQUAMOSA subfamily of genes, in apple' PLANT PHYSIOLOGY vol. 120, August 1999, pages 969 - 978, XP002938735
- RIECHMANN ET AL.: 'The AP2/EREBP family of plant transcription factors' BIOL. CHEM. vol. 379, June 1998, pages 633 - 646, XP002938736
- RIECHMANN ET AL.: 'Determination of floral organ identity by arabidopsis MADS domain homeotic proteins AP1, AP3, PI and AG is independent of their DNA-binding specificity' MOLECULAR BIOLOGY OF THE CELL vol. 8, July 1997, pages 1243 - 1259, XP002938737
- HEARD ET AL.: 'Evolutionary diversity of symbiotically induced nodule MADS box genes: characterization of nmhC5, a member of a novel subfamily' MOLECULAR PLANT-MICROBE INTERACTIONS vol. 10, no. 5, July 1997, pages 665 - 676, XP002938738
- RIECHMANN ET AL.: 'MADS domain proteins in plant development' BIOL. CHEM. vol. 378, October 1997, pages 1079 - 1101, XP002938739
- RIECHMANN ET AL.: 'DNA-binding properties of arabidopsis MADS domain homeotic proteins APETALA1, APETALA3, PISTILLATA and AGAMOUS' NUCLEIC ACIDS RESEARCH vol. 24, no. 16, August 1996, pages 3134 - 3141, XP002938740
- RIECHMANN ET AL.: 'Dimerization specificity of arabidopsis MADS domain homeotic proteins APETALA1, APETALA3, PISTILLATA and AGAMOUS' PROC. NATL. ACAD. SCI. USA vol. 93, May 1996, pages 4793 - 4798, XP002938741
- HEARD ET AL.: 'Symbiotic induction of a MADS-box gene during development of alfalfa root nodules' PROC. NATL. ACAD. SCI. USA vol. 92, June 1995, pages 5273 - 5277, XP002938742

## Description

### FIELD OF THE INVENTION

This invention relates to the field of plant biology. More particularly, the present invention pertains to compositions and methods for phenotypically modifying a plant.

### BACKGROUND OF THE INVENTION

Transcription factors can modulate gene expression, either increasing or decreasing (inducing or repressing) the rate of transcription, This modulation results in differential levels of gene expression at various developmental stages, in different tissues and cell types, and in response to different exogenous (e.g., environmental) and endogenous stimuli throughout the life cycle of the organism.

Kasuga et al., 1999, Nature Biotechnology, Vol. 17, pp. 287-291, describes gene transfer of a single stress-inducible transcription factor.

Because transcription factors are key controlling elements of biological pathways, altering the expression levels of one or more transcription factors can change entire biological pathways in an organism. For example, manipulation of the levels of selected transcription factors may result in increased expression of economically useful proteins or metabolic chemicals in plants or to improve other agriculturally relevant characteristics. Conversely, blocked or reduced expression of a transcription factor may reduce biosynthesis of unwanted compounds or remove an undesirable trait. Therefore, manipulating transcription factor levels in a plant offers tremendous potential in agricultural biotechnology for modifying a plant's traits.

Described herein are novel transcription factors useful for modifying a plant's phenotype in desirable ways, such as modifying a plant's environmental stress tolerance.

### SUMMARY OF THE INVENTION

In one aspect, the invention is a transgenic plant as defined in the claims.

The plant may be a soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, or vegetable brassicas plant.

Furthermore, the invention relates to a method for producing a plant having improved environmental stress tolerance, the method being as defined in the claims.

### BRIEF DESCRIPTION OF THE DRAWINGS

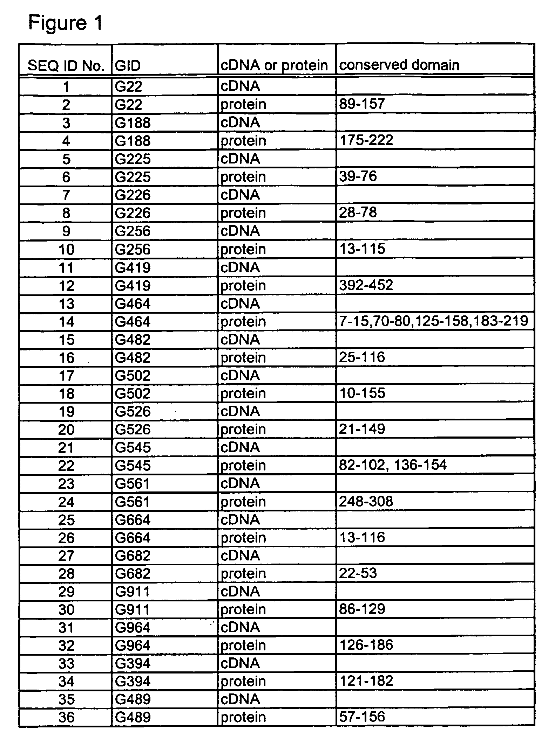

Figure 1 provides a table of exemplary polynucleotide and polypeptide sequences. The table includes from left to right for each sequence: the SEQ ID No., the internal code reference number (GID), whether the sequence is a polynucleotide or polypeptide sequence, and identification of any conserved domains for the polypeptide sequences.

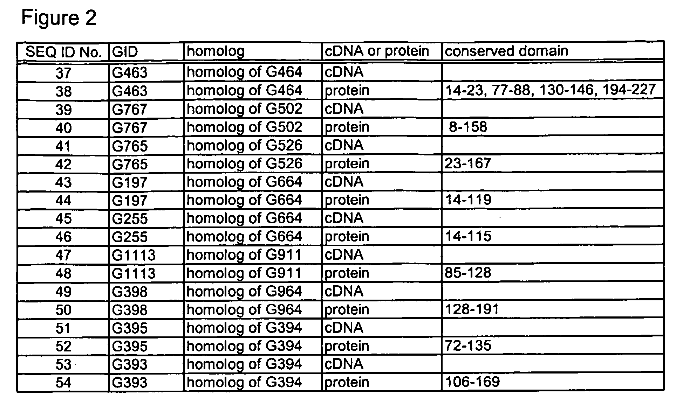

Figure 2 provides a table of exemplary sequences that are homologous to other sequences provided in the Sequence Listing and that are derived from *Arabidopsis thaliana.* The table includes from left to right: the SEQ ID No., the internal code reference number (GID), identification of the homologous sequence, whether the sequence is a polynucleotide or polypeptide sequence, and identification of any conserved domains for the polypeptide sequences.

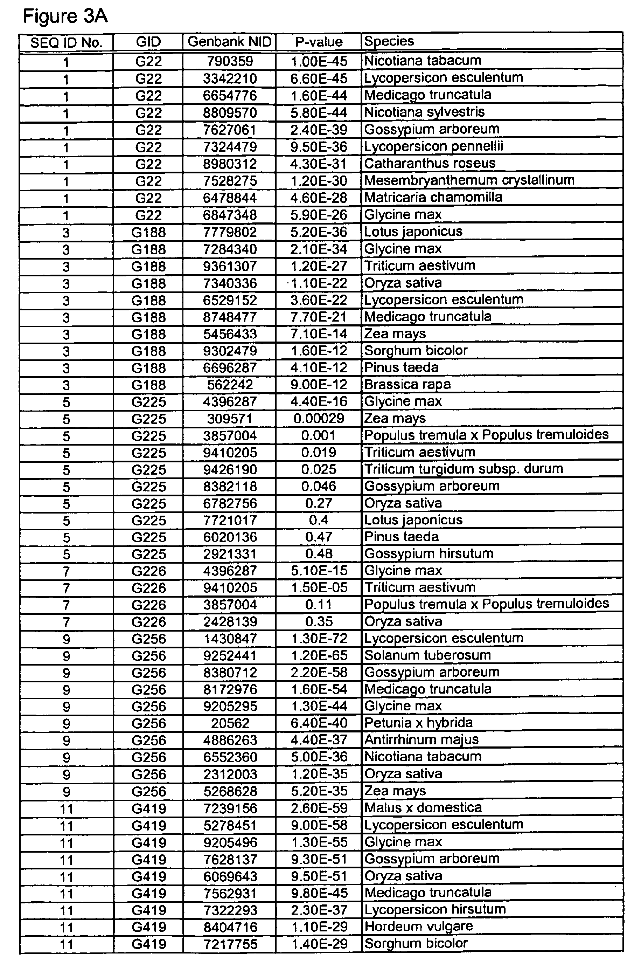

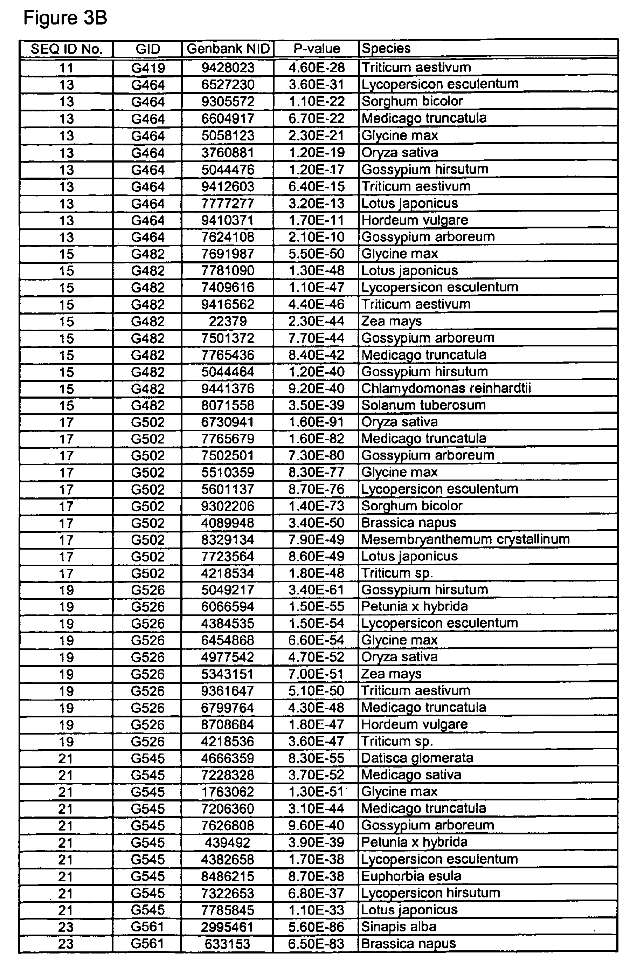

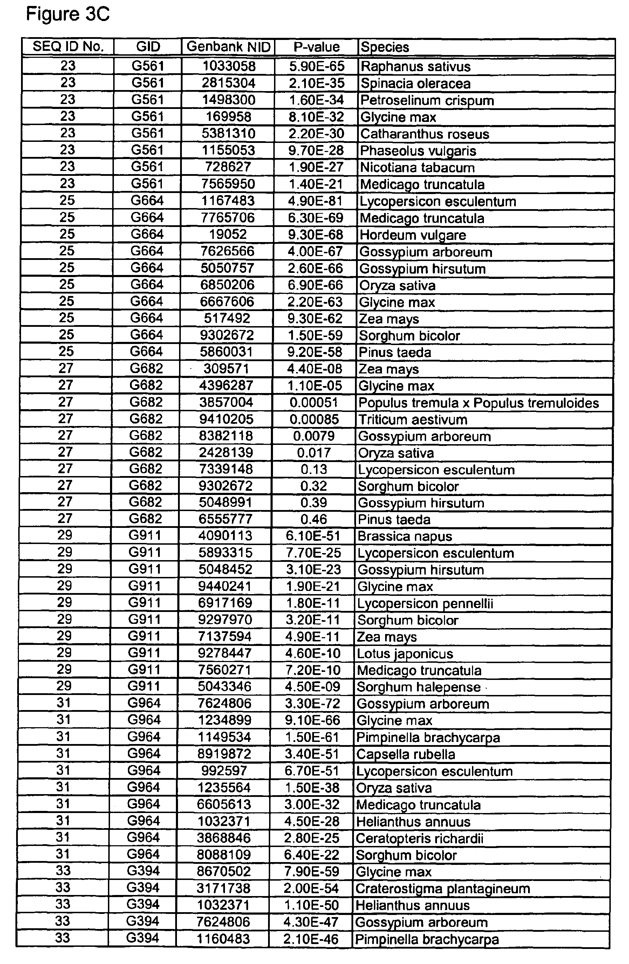

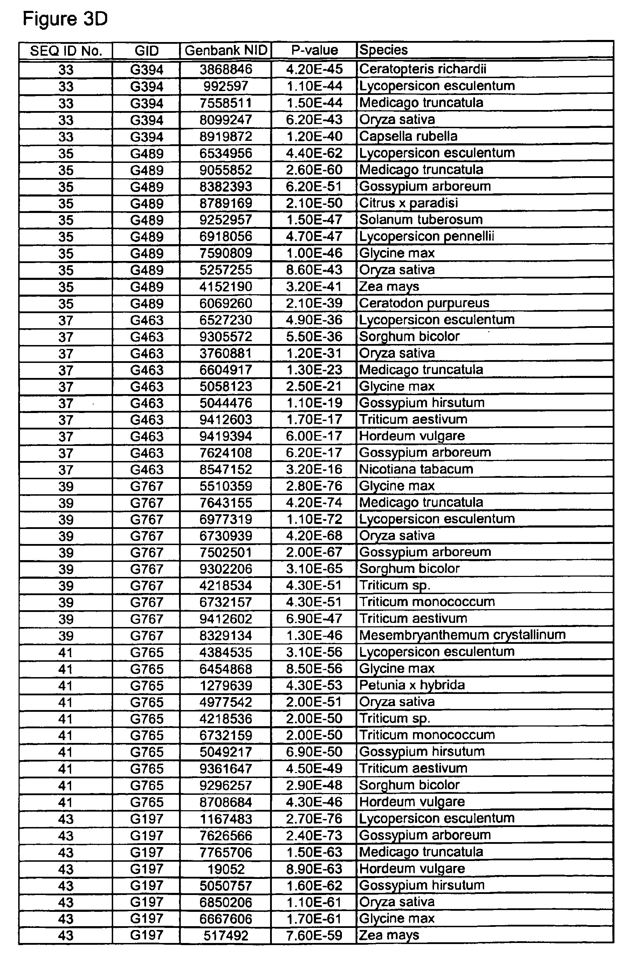

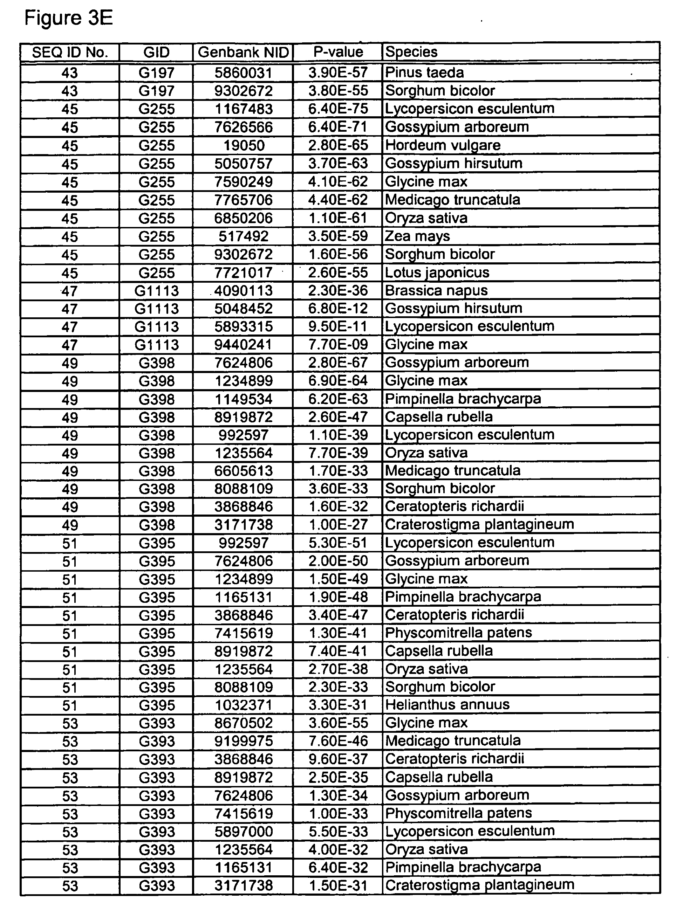

Figure 3 provides a table of exemplary sequences that are homologous to the sequences provided in Figures 1 and 2 and that are derived from plants other than *Arabidopsis thaliana*. The table includes from left to right: the SEQ ID No., the internal code reference number (GID), the unique GenBank sequence ID No. (NID), the probability that the comparison was generated by chance (P-value), and the species from which the homologous gene was identified.

### DETAILED DESCRIPTION

The present invention relates to polynucleotides and polypeptides, e.g. for modifying phenotypes of plants.

In particular, the polynucleotides or polypeptides are useful for modifying traits associated with a plant's environmental stress tolerance when the expression levels of the polynucleotides or expression levels or activity levels of the polypeptides are altered. Specifically, the polynucleotides and polypeptides are useful for modifying traits associated with a plant's environmental stress tolerance, such as freezing, chilling, heat, drought, water saturation, salt, photoconditions, radiation and ozone, or the like. Plants with altered expression of the polynucleotides or polypeptides of the invention are more tolerant to these environmental stresses compared with plants without altered expression levels.

The polynucleotides disclosed herein encode plant transcription factors. The plant transcription factors are derived, e.g., from *Arabidopsis thaliana* and can belong, e.g., to one or more of the following transcription factor families: the AP2 (APETALA2) domain transcription factor family (Riechmann and Meyerowitz (1998) J. Biol. Chem. 379:633-646); the MYB transcription factor family (Martin and Paz-Ares (1997) Trends Genet. 13:67-73); the MADS domain transcription factor family (Ritchmann and Meyerowitz (1997) J. Biol. Chem 378:1079-1101); the WRKY protein family (Ishiguro and Nakamura (1994) Mol. Gen. Genet. 244:563-571); the ankyrin-repeat protein family (Zhang et al. (1992) Plant Cell 4:1575-1588); the miscellaneous protein (MISC) family (Kim et al. (1997) Plant J. 11:1237-1251); the zinc finger protein (Z) family (Klug and Schwabe (1995) FASEB J. 9: 597-604); the homeobox (HB) protein family (Duboule (1994) Guidebook to the Homeobox Genes, Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 1996 250:7-16), the NAM protein family; the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the BPF-I protein (Box P-binding factor) family (da Costa e Silva et al. (1993) Plant J. 4:125-135); and the golden protein (GLD) family (Hall et al. (1998) Plant Cell 10:925-936).

In addition to methods for modifying a plant phenotype by employing one or more polynucleotides and polypeptides described herein, the polynucleotides and polypeptides have a variety of additional uses. These uses include their use in the recombinant production (i.e, expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like, of as substrates for cloning e.g., including digestion or ligation reactions, and for identifying exogenous or endogenous modulators of the transcription factors.

### DEFINITIONS

A "polynucleotide" is a nucleic acid sequence comprising a plurality of polymerized nucleotide residues, e.g., at least about 15 consecutive polymerized nucleotide residues, optionally at least about 30 consecutive nucleotides, at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material, not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

The phrase "ectopically expression or altered expression" in reference to a polynucleotide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides:

The term "fragment" or "domain," with respect to a polypeptide, refers to a subsequence of the polypeptide. In some cases, the fragment or domain, is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA binding domain that binds to a DNA promoter region, an activation domain or a domain for protein-protein interactions. Fragments can vary in size from as few as 6 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically, of at least consecutive about 15 nucleotides, preferably at least about 30 nucleotides, more preferably at least about 50, of any of the sequences provided herein.

The term "trait" refers to a physiological, morphological, biochemical or physical characteristic of a plant or particular plant material or cell. In some instances, this characteristic is visible to the human eye, such as seed or plant size, or can be measured by available biochemical techniques, such as the protein, starch or oil content of seed or leaves or by the observation of the expression level of genes, e.g., by employing Northern analysis, RT-PCR, microarray gene expression assays or reporter gene expression systems, or by agricultural observations such as stress tolerance, yield or pathogen tolerance.

"Trait modification" refers to a detectable difference in a characteristic in a plant ectopically expressing a polynucleotide or polypeptide of the present invention relative to a plant not doing so, such as a wild type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference. It is known that there can be a natural variation in the modified trait. Therefore, the trait modification observed entails a change of the normal distribution of the trait in the plants compared with the distribution observed in wild type plant.

Trait modifications of particular interest include those to seed ( such as embryo or endosperm), fruit, root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that can be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, internode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

### POLYPEPTIDES AND POLYNUCLEOTIDES

Described herein are among other things, transcription factors (TFs), and transcription factor homologue polypeptides, and isolated or recombinant polynucleotides encoding the polypeptides. These polypeptides and polynucleotides may be employed to modify a plant's environmental stress tolerance.

Exemplary polynucleotides encoding the polypeptides were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides were ectopically expressed in overexpressor or knockout plants and changes in the environmental stress tolerance of the plants was observed. Therefore, the polynucleotides and polypeptides can be employed to improve the environmental stress resistance of plants.

### Making polynucleotides

The polynucleotides include sequences that encode transcription factors and transcription factor homologue polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides. Procedures for identifying and isolating DNA clones are well known to those of skill in the art, and are described in, e.g., Berger and Kimmel, Guide to Molecular Cloning Techniques, Methods in Enzymology volume 152 Academic Press, Inc., San Diego, CA ("Berger"); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 2000) ("Ausubel").

Alternatively, polynucleotides can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids of the invention are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See*, e.g., Ausubel, Sambrook and Berger, *all supra.*

Alternatively, polynucleotides and oligonucleotides can be assembled from fragments produced by solid-phase synthesis methods. Typically, fragments of up to approximately 100 bases are individually synthesized and then enzymatically or chemically ligated to produce a desired sequence, e.g., a polynucletotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5, According to such methods, oligonucleotides are synthesized, purified, annealed to their complementary strand, ligated and then optionally cloned into suitable vectors. And if so desired, the polynucleotides and polypeptides of the invention can be custom ordered from any of a number of commercial suppliers.

### HOMOLOGOUS SEQUENCES

Sequences homologous, i.e., that share significant sequence identity or similarity, to those provided in the Sequence Listing, derived from *Arabidopsis thaliana* or from other plants of choice are also described herein. Homologous sequences can be derived from any plant including monocots and dicots and in particular agriculturally important plant species, including but not limited to, crops such as soybean, wheat, corn, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet, sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus.

Transcription factors that are homologous to the listed sequences will typically share at least about 30% amino acid sequence identity. More closely related transcription factors can share at least about 50%, about 60%, about 65%, about 70%, about 75% or about 80% or about 90% or about 95% or about 98% or more sequence identity with the listed sequences. Factors that are most closely related to the listed sequences share, e.g., at least about 85%, about 90% or about 95% or more % sequence identity to the listed sequences. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least 65% sequence identity including conservative substitutions, and preferably at least 80% sequence identity.

### Identifying Nucleic Acids by Hybridization

Polynucleotides homologous to the sequences illustrated in the Sequence Listing can be identified, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C, for example 0.2 x SSC, 0.1% SDS at 65° C. For identification of less closely related homologues washes can be performed at a lower temperature, e.g., 50° C. In general, stringency is increased by raising the wash temperature and/or decreasing the concentration of SSC.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x, 25x, 35x, 50x or more. The particular signal will depend on the label used in the relevant assay, e.g., a fluorescent label, a colorimetric label, a radio active label, or the like.

Alternatively, transcription factor homologue polypeptides can be obtained by screening an expression library using antibodies specific for one or more transcription factors. With the provision herein of the disclosed transcription factor, and transcription factor homologue nucleic acid sequences, the encoded polypeptide(s) can be expressed and purified in a heterologous expression system (e.g., *E. coli*) and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### SEQUENCE VARIATIONS

It will readily be appreciated by those of skill in the art, that any of a variety of polynucleotide sequences are capable of encoding the transcription factors and transcription factor homologue polypeptides described herein. Due to the degeneracy of the genetic code, many different polynucleotides can encode identical and/or substantially similar polypeptides in addition to those sequences illustrated in the Sequence Listing.

For example, Table 1 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

**Table 1**

| Amino acids | | | Codon | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art.

In addition to silent variations, other conservative variations that alter one, or a few amino acids in the encoded polypeptide, can be made without altering the function of the polypeptide.

For example, substitutions, deletions and insertions introduced into the sequences provided in the Sequence Listing are also envisioned. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser;Val |
| Trp | Tyr |
| Tyr | Trp; Phe |
| Val | Ile; Leu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### FURTHER MODIFYING SEQUENCES MUTATION/FORCED EVOLUTION

In addition to generating silent or conservative substitutions as noted, above, described herein are methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, given nucleic acid sequences are modified, e.g., according to standard mutagenesis or artificial evolution methods to produce modified sequences. For example, Ausubel, *supra,* provides additional details on mutagenesis methods. Artificial forced evolution methods are described, e.g., by Stemmer (1994) Nature 370:389-391, and Stemmer (1994) Proc. Natl. Acad. Sci. USA 91:10747-10751. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, the specification provides for modification of any given nucleic acid by mutation, evolution, chemical or enzymatic modification, or other available methods, as well as for the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *S*. *cerevisiae* and mammals are TAA and TGA, respectively. The preferred stop codon for monocotyledonous plants is TGA, whereas insects and *E. coli* prefer to use TAA as the stop codon.

The polynucleotide sequences can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides described herein can be combined with domains derived from other transcription factors or synthetic domains to modify the biological activity of a transcription factor. For instance, a DNA binding domain derived from a transcription factor described herein can be combined with the activation domain of another transcription factor or with a synthetic activation domain. A transcription activation domain assists in initiating transcription from a DNA binding site. Examples include the transcription activation region of VP16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### EXPRESSION AND MODIFICATION OF POLYPEPTIDES

Typically, polynucleotide sequences are incorporated into recombinant DNA (or RNA) molecules that direct expression of polypeptides of the invention in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

### Vectors, Promoters and Expression Systems

Described herein are recombinant constructs comprising one or more of the nucleic acid sequences disclosed herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence of the invention has been inserted, in a forward or reverse orientation. In a preferred aspect the construct further comprises regulatory sequences, including, for example, a promoter, operably linked to the sequence. Large numbers of suitable vectors and promoters are known to those of skill in the art, and are commercially available.

General texts which describe molecular biological techniques useful herein, including the use and production of vectors, promoters and many other relevant topics, include Berger, Sambrook and Ausubel, *supra.* Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucl Acid Res. 12: 8711-8721, Klee (1985) Bio/Technology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technology 10: 667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see*, e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide of the invention in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flower-specific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plant Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI*, Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Plant Mol Biol 48: 89-108). In addition, the timing of the expression can be controlled by using promoters such as those acting at senescence (An and Amazon (1995) Science 270: 1986-1988); or late seed development (Odell et al. (1994) Plant Physiol 106:447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

### Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present invention also relates to plant cells which are transduced with vectors described herein and the production of polypeptides (including fragments thereof) by recombinant techniques. Host cells are genetically engineered (i.e, nucleic acids are introduced, e.g., transduced, transformed or transfected) with the vectors disclosed herein, which may be, for example, a cloning vector or an expression vector comprising the relevant nucleic acids herein. The vector is optionally a plasmid, a viral particle, a phage, a naked nucleic acids, *etc*. The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be a plant cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small beads or particles, or on the surface (Klein et al., (1987) Nature 327, 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens,* and a portion is stably integrated into the plant genome (Horsch et al. (1984) Science 233:496-498; Fraley et al. (1983) Proc. Natl. Acad. Sci. USA 80, 4803).

The cell can include a nucleic acid which encodes a polypeptide, wherein the cells expresses a polypeptide described herein. The cell can also include vector sequences, or the like. Furthermore, cells and transgenic plants which include any polypeptide or nucleic acid above or throughout this specification, e.g., produced by transduction of a vector of the invention, are an additional feature of the invention.

For long-term, high-yield production of recombinant proteins, stable expression can be used. Host cells transformed with a nucleotide sequence encoding a polypeptide of the invention are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins described herein can be designed with signal sequences which direct secretion of the mature polypeptides through a prokaryotic or eukaryotic cell membrane.

### Modified Amino Acids

Polypeptides may contain one or more modified amino acids. The presence of modified amino acids may be advantageous in, for example, increasing polypeptide half-life, reducing polypeptide antigenicity or toxicity, increasing polypeptide storage stability, or the like. Amino acid(s) are modified, for example, co-translationally or post-translationally during recombinant production or modified by synthetic or chemical means.

Non-limiting examples of a modified amino acid include incorporation or other use of acetylated amino acids, glycosylated amino acids, sulfated amino acids, prenylated (e.g., farnesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acids are replete throughout the literature.

### IDENTIFICATION OF ADDITIONAL FACTORS

A transcription factor can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor to modify a phenotype as desired. For example, the transcription factors can be employed to identify one or more downstream gene with which is subject to a regulatory effect of the transcription factor. In one approach, a transcription factor or transcription factor homologue is expressed in a host cell, e.g, a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor can be used to identify promoter sequences (i.e., binding sites) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnology 17:573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien, et al., (1991), Proc. Natl. Acad. Sci. USA 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be preformed.

### IDENTIFICATION OF MODULATORS

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northerns, quantitative PCR, or any other technique for monitoring changes in mRNA expression. These techniques are exemplified in Ausubel et al. (eds) Current Protocols in Molecular Biology, John Wiley & Sons (1998). Such changes in the expression levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on microtiter plates in robotic assays).

In one embodiment, high throughput screening methods involve providing a combinatorial library containing a large number of potential compounds (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnology, 14(3):309-314 and PCT/US96/10287), carbohydrate libraries (see, e.g., Liang et al. Science (1996) 274:1520-1522 and U.S. Patent 5,593,853), peptide nucleic acid libraries (see, e.g., U.S. Patent 5,539,083), and small organic molecule libraries (see, e.g., benzodiazepines, Baum C&EN Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337) and the like.

Preparation and screening of combinatorial or other libraries is well known to those of skill in the art. Such combinatorial chemical libraries include, but are not limited to, peptide libraries (see, e.g., U.S. Patent 5,010,175, Furka, Int. J. Pept. Prot. Res. 37:487-493 (1991) and Houghton et al. Nature 354:84-88 (1991)). Other chemistries for generating chemical diversity libraries can also be used.

In addition, as noted, compound screening equipment for high-throughput screening is generally available, e.g., using any of a number of well known robotic systems that have also been developed for solution phase chemistries useful in assay systems. These systems include automated workstations including an automated synthesis apparatus and robotic systems utilizing robotic arms. Any of the above devices are suitable for use with the present invention, e.g., for high-throughput screening of potential modulators. The nature and implementation of modifications to these devices (if any) so that they can operate as discussed herein will be apparent to persons skilled in the relevant art.

Indeed, entire high throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provides technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides according to the present invention.

In some assays it is desirable to have positive controls to ensure that the components of the assays are working properly. At least two types of positive controls are appropriate. That is, known transcriptional activators or inhibitors can be incubated with cells/plants/ etc. in one sample of the assay, and the resulting increase/decrease in transcription can be detected by measuring the resulting increase in RNA/ protein expression, etc., according to the methods herein. It will be appreciated that modulators can also be combined with transcriptional activators or inhibitors to find modulators which inhibit transcriptional activation or transcriptional repression. Either expression of the nucleic acids and proteins herein or any additional nucleic acids or proteins activated by the nucleic acids or proteins herein, or both, can be monitored.

Described herein is a method for identifying compositions that modulate the activity or expression of a polynucleotide or polypeptide of the invention. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide of the invention.

### SUBSEQUENCES

Also contemplated are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra- high stringent conditions) conditions to a polynucleotide sequence described above. The polynucleotides may be used as probes, primers, sense and antisense agents, and the like, according to methods as noted *supra*.

Subsequences of the polynucleotides described herein including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologues of the invention, including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook and Ausubel, *supra.*

In addition, described herein are isolated or recombinant polypeptides including a subsequence of at least about 15 contiguous amino acids encoded by the recombinant or isolated polynucleotides disclosed herein. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

### PRODUCTION OF TRANSGENIC PLANTS

### Modification of Traits

The polynucleotides disclosed herein are favorably employed to produce transgenic plants with various traits, or characteristics, that have been modified in a desirable manner, e.g., to improve the environmental stress resistance of a plant. For example, alteration of expression levels or patterns (e.g., spatial or temporal expression patterns) of one or more of the transcription factors (or transcription factor homologues) described herein, as compared with the levels of the same protein found in a wild type plant, can be used to modify a plant's traits. An illustrative example of trait modification, improved environmental stress tolerance, by altering expression levels of a particular transcription factor is described further in the Examples and the Sequence Listing.

### Antisense and Cosuppression Approaches

In addition to expression of the nucleic acids disclosed herein as gene replacement or plant phenotype modification nucleic acids, the nucleic acids are also useful for sense and anti-sense suppression of expression, e.g., to down-regulate expression of a nucleic acid, e.g., as a further mechanism for modulating plant phenotype. That is, the nucleic acids of the invention, or subsequences or anti-sense sequences thereof, can be used to block expression of naturally occurring homologous nucleic acids. A variety of sense and anti-sense technologies are known in the art, e.g., as set forth in Lichtenstein and Nellen (1997) Antisense Technology: A Practical Approach IRL Press at Oxford University, Oxford, England. In general, sense or anti-sense sequences are introduced into a cell, where they are optionally amplified, e.g., by transcription. Such sequences include both simple oligonucleotide sequences and catalytic sequences such as ribozymes.

For example, a reduction or elimination of expression (i.e., a "knock-out") of a transcription factor or transcription factor homologue polypeptide in a transgenic plant, e.g., to modify a plant trait, can be obtained by introducing an antisense construct corresponding to the polypeptide of interest as a cDNA. For antisense suppression, the transcription factor or homologue cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using a ribozyme. Ribozymes are RNA molecules that possess highly specific endoribonuclease activity. The production and use of ribozymes are disclosed in U.S. Patent No. 4,987,071 and U.S. Patent No. 5,543,508. Synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a transcription factor or transcription factor homologue cDNA is over-expressed can also be used to obtain co-suppression of a corresponding endogenous gene, e.g., in the manner described in U.S. Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating it's activity and modifying one or more traits. Methods for producing such constructs are described in U.S. Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141).

Another method for abolishing the expression of a gene is by insertion mutagenesis using the T-DNA of *Agrobacterium tumefaciens.* After generating the insertion mutants, the mutants can be screened to identify those containing the insertion in a transcription factor or transcription factor homologue gene. Plants containing a single transgene insertion event at the desired gene can be crossed to generate homozygous plants for the mutation (Koncz et al. (1992) Methods in Arabidopsis Research, World Scientific).

Alternatively, a plant phenotype can be altered by eliminating an endogenous gene, such as a transcription factor or transcription factor homologue, e.g., by homologous recombination (Kempin et al. (1997) Nature 389:802).

A plant trait can also be modified by using the cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides described herein can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide of the invention (*See*, e.g., PCT Publications WO 96/06166 and WO 98/53057 which describe the modification of the DNA binding specificity of zinc finger proteins by changing particular amino acids in the DNA binding motif).

The transgenic plant can also include the machinery necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides and/or expressing the polypeptides described herein can be produced by a variety of well established techniques as described above. Following construction of a vector, most typically an expression cassette, including a polynucleotide, e.g., encoding a transcription factor or transcription factor homologue, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melon and cucumber), *Gramineae* (wheat, com, rice, barley, millet, etc.), *Solanaceae* (potato, tomato, tobacco, peppers, etc.), and various other crops. See protocols described in Ammirato et al. (1984) Handbook of Plant Cell Culture -Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, include: U.S. Patent Nos. 5,571,706; 5,677;175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modifed trait can be any of those traits described above. Additionally, to confirm that the modified trait is due to changes in expression levels or activity of the polypeptide or polynucleotide described herein can be determined by analyzing mRNA expression using Northern blots, RT-PCR or microarrays, or protein expression using immunoblots or Western blots or gel shift assays.

### INTEGRATED SYSTEMS-SEQUENCE IDENTITY

Also described herein is an integrated system, computer or computer readable medium that comprises an instruction set for determining the identity of one or more sequences in a database, In addition, the instruction set can be used to generate or identify sequences that meet any specified criteria. Furthermore, the instruction set may be used to associate or link certain functional benefits, such improved environmental stress tolerance, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra*.

A variety of methods of determining sequence relationships can be used, including manual alignment and computer assisted sequence alignment and analysis. This later approach is a preferred approach in the present invention, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915).

In addition to calculating percent sequence identity, the BLAST algorithm also performs a statistical analysis of the similarity between two sequences (*see,* e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element which displays an alignment produced by the alignment instruction set.

The methods can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intra-net or an internet.

Thus, described herein are methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### EXAMPLES

The following examples are intended to illustrate but not limit the present invention.

### EXAMPLE I. FULL LENGTH GENE IDENTIFICATION AND CLONING

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, Arabidopsis *thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with ³²p dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60°C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the Marathon^{™} cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon^{™} Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### EXAMPLE II. CONSTRUCTION OF EXPRESSION VECTORS

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20 and the amplified DNA fragment were digested separately with SalI and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E. coli* strain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### EXAMPLE III. TRANSFORMATION OF AGROBACTERIUM WITH THE EXPRESSION VECTOR

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4°C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 - 100 ng DNA (generally resuspended in 10 mM Tris-HCl, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28°C in a shaking incubator. After recovery, cells were plated onto selective medium of LB broth containing 100 µg/ml spectinomycin (Sigma) and incubated for 24-48 hours at 28°C. Single colonies were then picked and inoculated in fresh medium. The presence of the plasmid construct was verified by PCR amplification and sequence analysis.

### EXAMPLE IV. TRANSFORMATION OF ARABIDOPSIS PLANTS WITH AGROBACTERIUM TUMEFACIENS WITH EXPRESSION VECTOR

After transformation *of Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of ∼ 10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### EXAMPLE V. IDENTIFICATION OF ARABIDOPSIS PRIMARY TRANSFORMANTS

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1M KOH), I X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### EXAMPLE VI. IDENTIFICATION OF ARABIDOPSIS PLANTS WITH TRANSCRIPTION FACTOR GENE KNOCKOUTS

The screening of insertion mutagenized *Arabidopsis* collections for null mutants in a known target gene was essentially as described in Krysan et al (1999) Plant Cell 11:2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### EXAMPLE VII. IDENTIFICATION OF ENVIRONMENTAL STRESS TOLERANCE PHENOTYPE IN OVEREXPRESSOR OR GENE KNOCKOUT PLANTS

Experiments were performed to identify those transformants or knockouts that exhibited an improved environmental stress tolerance. For such studies, the transformants were exposed to a variety of environmental stresses. Plants were exposed to chilling stress (6 hour exposure to 4-8°C), heat stress (6 hour exposure to 32-37°C), high salt stress (6 hour exposure to 200 mM NaCl), drought stress (168 hours after removing water from trays), osmotic stress (6 hour exposure to 3 M mannitol), or nutrient limitation (nitrogen, phosphate, and potassium) (Nitrogen: all components of MS medium remained constant except N was reduced to 20mg/L of NH 4 NO3, or Phosphate: All components of MS medium except KH 2 PO 4, which was replaced by K2SO4, Potassium: All components of MS medium except removal of KNO3 and KH2PO4, which were replaced by NaH4PO4).

Table 3 shows the phenotypes observed for particular overexpressor or knockout plants and provides the SEQ ID No., the internal reference code (GID), whether a knockout or overexpressor plant was analyzed and the observed phenotype.

**Table 3**

| SEQ ID No. | GID | Knockout (KO) or overexpressor (OX) | Phenotype observed |
|---|---|---|---|
| 1 | G22 | OE | Increased tolerance to high salt |
| 3 | G188 | KO | Better germination under osmotic stress |
| 5 | G225 | OE | Increased tolerance to nitrogen-limited medium |
| 7 | G226 | OE | Increased tolerance to nitrogen-limited medium |
| 9 | G256 | OE | Better germination and growth in cold |
| 11 | G419 | OE | Increased tolerance to potassium-free medium |
| 13 | G464 | OE | Better germination and growth in heat |
| 15 | G482 | OE | Increased tolerance to high salt |
| 17 | G502 | KO | Increased sensitivity to osmotic stress |
| 19 | G526 | OE | Increased sensitivity to osmotic stress |
| 21 | G545 | OE | Susceptible to high salt |
| 23 | G561 | OE | Increased tolerance to potassium-free medium |
| 25 | G664 | OE | Better germination and growth in cold |
| 27 | G682 | OE | Better germination and growth in heat |
| 29 | G911 | OE | Increased growth on potassium-free medium |
| 31 | G964 | OE | Better germination and growth in heat |
| 33 | G394 | OE | More sensitive to chilling |
| 35 | G489 | OE | Increased tolerance to osmotic stress |

For a particular overexpressor that shows a decreased tolerance to an environmental stress, it may be more useful to select a plant with a decreased expression of the particular transcription factor. For a particular knockout that shows a decreased tolerance to an environmental stress, it may be more useful to select a plant with an increased expression of the particular transcription factor.

### EXAMPLE VIII. IDENTIFICATION OF HOMOLOGOUS SEQUENCES

Homologous sequences from *Arabidopsis* and plant species other than *Arabidopsis* were identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs were employed using the BLOSUM-62 scoring matrix (Henikoff, S. and Henikoff, J. G. (1992) Proc. Natl. Acad Sci. USA 89:0915-10919).

Identified *Arabidopsis* homologous sequences are provided in Figure 2 and included in the Sequence Listing. The percent sequence identity among these sequences is as low as 47% sequence identity. Additionally, the entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*). These sequences were compared to sequences representing genes of SEQ IDs Nos. 1-54 on 9/26/2000 using the Washington University TBLASTX algorithm (version 2.0a19MP). For each gene of SEQ IDs Nos. 1-54, individual comparisons were ordered by probability score (P-value), where the score reflects the probability that a particular alignment occurred by chance. For example, a score of 3.6e-40 is 3.6 x 10⁻⁴⁰. For up to ten species, the gene with the lowest P-value (and therefore the most likely homolog) is listed in Figure 3.

In addition to P-values, comparisons were also scored by percentage identity. Percentage identity reflects the degree to which two segments of DNA or protein are identical over a particular length. The ranges of percent identity between the non-Arabidopsis genes shown in Figure 3 and the Arabidopsis genes in the sequence listing are: SEQ ID No. 1: 53%-67%; SEQ ID No. 3: 38%-76%; SEQ ID No. 5: 34%-67%; SEQ ID No. 7: 50%-69%; SEQ ID No. 9: 32%-91%; SEQ ID No. 11: 48%-66%; SEQ ID No. 13: 34%-60%; SEQ ID No. 15: 58%-81%; SEQ ID No. 17: 65%-94%; SEQ ID No. 19: 72%-83%; SEQ ID No. 21: 52%-64%; SEQ ID No. 23: 40%-89%; SEQ ID No. 25: 86%-97%; SEQ ID No. 27: 41%-75%; SEQ ID No. 29: 29%-72%; SEQ ID No. 31: 49%-70%; SEQ ID No, 33: 56%-86%; SEQ ID No. 35: 61%-84%; SEQ ID No. 37: 40%-58%; SEQ ID No. 39: 63%-87%; SEQ ID No. 41: 51%-88%; SEQ ID No. 43: 80%-90%; SEQ ID No. 45: 79%-90%; SEQ ID No. 47: 30%-58%; SEQ ID No. 49: 52%-62%; SEQ ID No. 51: 55%-73% and SEQ ID No. 53: 44%-80%.

The polynucleotides and polypeptides in the Sequence Listing and the identified homologous sequences may be stored in a computer system and have associated or linked with the sequences a function, such as that the polynucleotides and polypeptides are useful for modifying the environmental stress tolerance of a plant.

## Claims

1. A transgenic plant having greater tolerance to an environmental stress than a wild-type plant of the same species,
wherein the environmental stress is selected from the group consisting of drought, salt, heat and cold,
wherein the transgenic plant comprises an expression vector or cassette, and wherein the expression vector or cassette comprises a polynucleotide having a sequence encoding a polypeptide having a conserved domain with at least 80% sequence identity to the conserved domain of amino acid residues 25 to 116 of SEQ ID NQ:16,
and wherein overexpression of the polypeptide in the transgenic plant increases the tolerance of the transgenic plant to the environmental stress as compared to the tolerance of the wild-type plant to drought, salt, heat or cold.

2. The transgenic plant of claim 1, wherein the polynucleotide has a sequence comprising SEQ ID NO:15.

3. The transgenic plant of any one of claims 1 or 2, wherein the expression vector or cassette further comprises a constitutive, inducible or tissue-specific promoter operably linked to the polynucleotide having said sequence.

4. The transgenic plant of any one of claims 1 to 3, wherein the transgenic plant has greater tolerance to salt than the wlld-type plant.

5. The transgenic plant of any one of claims 1 to 4, wherein the plant is selected from the group consisting of soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits and vegetable brassicas.

6. The transgenic plant of any one of claims 1 to 5, wherein the transgenic plant is more tolerant than the wild type plant to a six-hour exposure to 200 mM NaCl.

7. A seed obtainable from the transgenic plant of any of claims 1 to 6, wherein the seed comprises the expression vector or cassette comprising the polynucleotide having said sequence.

8. Use of an expression vector or cassette to produce a transformed plant, wherein the expression vector or cassette encodes a polypeptide having a conserved domain with at least 80% sequence identity to the conserved domain of amino acid residues 25 to 116 of SEQ ID NO:16, and overexpression of the polypeptide in the transformed plant increases tolerance of the transformed plant to heat, drought, salt and/or cold as compared to the tolerance of the wild-type plant.

9. Use of a polynucleotide to produce a transformed plant, wherein the polynucleotide encodes a polypeptide having a conserved domain with at least 80% sequence identity to a conserved domain of amino acid residues 25 to 116 of SEQ ID NO:16, the polynucleotide is incorporated into an expression vector or cassette, the expression vector or cassette is introduced into a plant to produce the transformed plant, and the transformed plant has increased tolerance to heat, drought, salt and/or cold as compared to the tolerance of the wild type plant.

10. A method for producing a plant having increased tolerance to heat, drought, salt and/or cold, the method comprising the steps of:
(a) providing an expression vector or cassette comprising an isolated or recombinant polynucleotide encoding a polypeptide that comprises a conserved domain with at least 80% sequence identity to a conserved domain of amino acid residues 25 to 116 of SEQ ID NO:16; and
(b) introducing the expression vector or cassette into the plant to produce a transformed plant, wherein
overexpression of the polypeptide in the transformed plant increases tolerance of the transformed plant to heat, drought, salt and/or cold as compared to the tolerance of a wild-type plant to drought, salt, heat and/or cold.

11. The method of claim 10, wherein the transformed plant is more tolerant than the wild type plant to a six-hour exposure to 200 mM NaCl.

12. The method according to claim 10 or 11, wherein the expression vector or cassette comprises a constitutive, inducible or tissue-specific promoter.

## Patentansprüche

1. Transgene Pflanze mit höherer Toleranz gegenüber Umweltbelastungen als eine Wildtyppflanze derselben Spezies,
worin die Umweltbelastung aus der aus Trockenheit, Salz, Hitze und Kälte bestehenden Gruppe ausgewählt ist,
worin die transgene Pflanze einen Expressionsvektor oder eine Expressionskassette umfasst und worin der Expressionsvektor oder die Expressionskassette ein Polynucleotid mit einer Sequenz umfasst, die für ein Polypeptid kodiert, das eine konservierte Domäne mit zumindest 80 % Sequenzidentität mit der konservierten Domäne der Aminosäurereste 25 bis 116 der Seq.-ID Nr. 16 aufweist,
und worin eine Überexpression des Polypeptids in der transgenen Pflanze die Toleranz der transgenen Pflanze gegenüber Umweltbelastungen im Vergleich zur Toleranz der Wildtyppflanze gegenüber Trockenheit, Salz, Hitze oder Kälte steigert.

2. Transgene Pflanze nach Anspruch 1, worin das Polynucleotid eine Sequenz aufweist, die Seq.-ID Nr. 15 umfasst.

3. Transgene Pflanze nach einem der Ansprüche 1 bis 2, worin der Expressionsvektor oder die Expressionskassette weiteres einen konstitutiven, induzierbaren oder gewebespezifischen Promotor umfasst, der operabel an das Polynucleotid mit der genannten Sequenz gebunden ist.

4. Transgene Pflanze nach einem der Ansprüche 1 bis 3, worin die transgene Pflanze höhere Toleranz gegenüber Salz aufweist als die Wildtyppflanze.

5. Transgene Pflanze nach einem der Ansprüche 1 bis 4, worin die Pflanze aus der aus Sojabohne, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Cantaloupe-Melone, Karotte, Karfiol, Kaffee, Gurke, Melanzani, Weintrauben, Honigmelone, Gartensalat, Mango, Zuckermelone, Zwiebel, Papaya, Erbsen, Paprika, Ananas, Spinat, Kürbis, Süßmais, Tabak, Tomate, Wassermelone, Rosengewächs-Obstbäume und Gemüsekohlsorten bestehenden Gruppe ausgewählt ist.

6. Transgene Pflanze nach einem der Ansprüche 1 bis 5, worin die transgene Pflanze gegenüber einer sechsstündigen Aussetzung gegenüber 200 mM NaCl toleranter ist als die Wildtyppflanze.

7. Samen, der von einer transgenen Pflanze nach einem der Ansprüche 1 bis 6 erhältlich ist, worin der Samen den Expressionsvektor oder die Expressionskassette umfasst, der/die das Polynucleotid mit der genannten Sequenz umfasst.

8. Verwendung eines Expressionsvektors oder einer Expressionskassette, um eine transformierte Pflanze herzustellen, worin der Expressionsvektor oder die Expressionskassette für ein Polypeptid kodiert, das eine konservierte Domäne mit zumindest 80 % Sequenzidentität mit der konservierten Domäne der Aminosäurereste 25 bis 116 der Seq.-ID Nr. 16 aufweist, und eine Überexpression des Polypeptids in der transformierten Pflanze die Toleranz der transformierten Pflanze gegenüber Hitze, Trockenheit, Salz und/oder Kälte im Vergleich zur Toleranz der Wildtyppflanze steigert.

9. Verwendung eines Polynucleotids zur Herstellung einer transformierten Pflanze, worin das Polynucleotid für ein Polypeptid kodiert, das eine konservierte Domäne mit zumindest 80 % Sequenzidentität mit der konservierten Domäne der Aminosäurereste 25 bis 116 der Seq.-ID Nr. 16 aufweist, das Polynucleotid in einen Expressionsvektor oder eine Expressionskassette inkorporiert ist, der Expressionsvektor oder die Expressionskassette in eine Pflanze eingeführt wird, um die transformierte Pflanze herzustellen, und die transformierte Pflanze im Vergleich zur Toleranz der Wildtyppflanze gesteigerte Toleranz gegenüber Hitze, Trockenheit, Salz und/oder Kälte aufweist.

10. Verfahren zur Herstellung einer Pflanze mit gesteigerter Toleranz gegenüber Hitze, Trockenheit, Salz und/oder Kälte, wobei das Verfahren folgenden Schritte umfasst:
(a) das Bereitstellen eines Expressionsvektors oder einer Expressionskassette, der/die ein isoliertes oder rekombinantes Polynucleotid umfasst, das für ein Polypeptid kodiert, welches eine konservierte Domäne mit zumindest 80 % Sequenzidentität mit der konservierten Domäne der Aminosäurereste 25 bis 116 von Seq.-ID Nr. 16 aufweist; und
(b) das Einführen des Expressionsvektors oder der Expressionskassette in die Pflanze, um eine transformierte Pflanze herzustellen, worin
eine Überexpression des Polypeptids in der transformierten Pflanze die Toleranz der transformierten Pflanze gegenüber Trockenheit, Salz, Hitze und/oder Kälte im Vergleich zur Toleranz der Wildtyppflanze Trockenheit, Salz, Hitze und/oder Kälte steigert.

11. Verfahren nach Anspruch 10, worin die transformierte Pflanze gegenüber einer sechsstündigen Aussetzung gegenüber 200 mM NaCl toleranter ist als die Wildtyppflanze.

12. Verfahren nach Anspruch 10 oder 11, worin der Expressionsvektor oder die Expressionskassette einen konstitutiven, induzierbaren oder gewebespezifischen Promoter umfasst.

## Revendications

1. Plante transgénique ayant une plus grande tolérance vis-à-vis d'un stress environnemental qu'une plante du type sauvage de la même espèce,
où le stress environnemental est sélectionné dans le groupe consistant en sécheresse, sel, chaleur et froid,
où la plante transgénique comprend un vecteur d'expression ou cassette et où le vecteur d'expression ou cassette comprend un polynucléotide ayant une séquence codant pour un polypeptide ayant un domaine conservé avec au moins 80% d'identité de séquence avec le domaine conservé de résidus d'acides aminés 25 à 116 de SEQ ID NO:16;
et où la surexpression du polypeptide dans la plante transgénique augmente la tolérance de la plante transgénique au stress environnemental en comparaison avec la tolérance de la plante du type sauvage vis-à-vis de la sécheresse, du sel, de la chaleur ou du froid.

2. Plante transgénique de la revendication 1, où le polynucléotide a une séquence comprenant SEQ ID NO:15.

3. Plante transgénique de l'une quelconque des revendications 1 ou 2, où le vecteur d'expression ou cassette comprend de plus un promoteur constitutif inductible spécifique du tissu opérativement enchaîné au polynucléotide ayant ladite séquence.

4. Plante transgénique de l'une quelconque des revendications 1 à 3, où la plante transgénique a une plus grande tolérance vis-à-vis d'un sel que la plante du type sauvage.

5. Plante transgénique de l'une quelconque des revendications 1 à 4, où la plante est sélectionnée dans le groupe consistant en soja, blé, maïs, pomme de terre, coton, riz, colza, tournesol, luzerne, canne à sucre, gazon, banane, mûre, myrtille, fraise, framboise, cantaloup, carotte, choux-fleur, café, concombre, aubergine, raisin, honeydew, laitue, mangue, melon, oignon, papaye, pois, poivre, ananas, épinard, patisson, maïs doux, tabac, tomate, melon d'eau, fruits rosacés, brasicas végétaux.

6. Plante transgénique de l'une quelconque des revendications 1 à 5, où la plante transgénique est plus tolérante que la plante du type sauvage vis-à-vis d'une exposition de six heures à NaCl 200 nM.

7. Graine obtenue à partir de la plante transgénique de l'une quelconque des revendications 1 à 6, où la graine comprend le vecteur d'expression ou cassette comprenant le polynucléotide ayant ladite séquence.

8. Utilisation d'un vecteur d'expression ou d'une cassette pour produire une plante transformée où le vecteur d'expression ou cassette code pour un polypeptide ayant un domaine conservé avec au moins 80% d'identité de séquence avec le domaine conservé des résidus d'acides aminés 25 à 116 de SEQ ID NO:16 et la surexpression du polypeptide dans la plante transformée augmente la tolérance de la plante transformée à la chaleur, à la sécheresse, au sel et/ou au froid en comparaison avec la tolérance du type sauvage.

9. Utilisation d'un polynucléotide pour produire une plante transformée où le polynucléotide code pour un polypeptide ayant un domaine conservé avec au moins 80% d'identité de séquence avec un domaine conservé de résidus d'acides aminés 25 à 116 de SEQ ID NO:16, le polynucléotide est incorporé dans un vecteur d'expression ou cassette, le vecteur d'expression ou cassette est introduit dans une plante pour produire la plante transformée et la plante transformée a une tolérance accrue à la chaleur, à la sécheresse, au sel et/ou au froid en comparaison avec la tolérance de la plante du type sauvage.

10. Méthode de production d'une plante ayant une tolérance accrue à la chaleur, à la sécheresse, au sel et/ou au froid, la méthode comprenant les étapes de :
(a) prévoir un vecteur d'expression ou cassette comprenant un polynucléotide isolé ou recombinant codant pour un polypeptide qui comprend un domaine conservé avec au moins 80% d'identité de séquence avec un domaine conservé des résidus d'acides aminés 25 à 116 de SEQ ID NO:16 ; et
(b) introduire le vecteur d'expression ou cassette dans la plante pour produire une plante transformée, où
la surexpression du polypeptide dans la plante transformée augmente la tolérance de la plante transformée à la chaleur, la sécheresse, le sel et/ou le froid en comparaison avec la tolérance d'une plante du type sauvage à la sécheresse, le sel, la chaleur et/ou le froid.

11. Méthode de la revendication 10, où la plante transformée est plus tolérance que la plante du type sauvage à une exposition de six heures à NaCl 200 nM.

12. Méthode selon la revendication 10 ou 11, où le vecteur d'expression ou cassette comprend un promoteur constitutif inductible spécifique du tissu.


### SEQUENCE LISTING

<110> Pineda, Omaira
   Yu, Guo-Liang
   Creelman, Robert
   Riechmann, Jose Luis
   Heard, Jacqueline
   Ratcliffe, Oliver
   Reuber, Lynne
   Keddie, James
<120> Environmental Stress Tolerance Genes
<130> MBI-0016
<150> 60/166,228
   <151> 1999-11-17
<150> 60/197,899
   <151> 2000-04-17
<150> Plant Trait Modification III
   <151> 2000-08-22
<160> 54
<170> PatentIn version 3.0
<210> 1
   <211> 913
   <212> DNA
   <213> Arabidopsis thaliana
<220>
   <221> CDS
   <222> (81)..(761)
   <223> G22
<400> 1
   agaaaacatc tctcactctc taaaatacac actctcatca aaaaccttct cttcggttca      60
   gaagcattca agaatccatt atg agc tca tct gat tcc gtt aat aac ggc gtt     113
                         Met Ser Ser Ser Asp Ser Val Asn Asn Gly Val
                         1               5                   10
   aac tca cgg atg tac ttc cgt aac ccg agt ttc agc aac gtt atc tta       161
   Asn Ser Arg Met Tyr Phe Arg Asn Pro Ser Phe Ser Asn Val Ile Leu
               15                  20                  25
   aac gat aac tgg agc gac ttg ccg tta agt gtc gac gat tct caa gac       209
   Asn Asp Asn Trp Ser Asp Leu Pro Leu Ser Val Asp Asp Ser Gln Asp
           30                  35                  40
   atg gct att tac aac act ctc cgt gat gcc gtt agc tcc ggc tgg aca       257
   Met Ala Ile Tyr Asn Thr Leu Arg Asp Ala Val Ser Ser Gly Trp Thr
       45                  50                  55
   ccc tcc gtt cct ccc gtt acc tct ccg gcg gag gaa aat aag cct ccg       305
   Pro Ser Val Pro Pro Val Thr Ser Pro Ala Glu Glu Asn Lys Pro Pro
   60                  65                  70                  75
   gcg acg aag gcg agt ggc tca cac gcg ccg agg cag aag ggg atg cag       353
   Ala Thr Lys Ala Ser Gly Ser His Ala Pro Arg Gln Lys Gly Met Gln
                   80                  85                  90
   tac aga gga gtg agg agg agg ccg tgg ggg aaa ttc gcg gcg gag att       401
   Tyr Arg Gly Val Arg Arg Arg Pro Trp Gly Lys Phe Ala Ala Glu Ile
               95                  100                 105
   agg gat ccg aag aag aac gga gct agg gtt tgg ctc ggg act tac gag       449
   Arg Asp Pro Lys Lys Asn Gly Ala Arg Val Trp Leu Gly Thr Tyr Glu
           110                 115                 120
   acg ccg gag gac gcg gcg gtg gcg tac gac cga gcg gcg ttt cag ctc       497
   Thr Pro Glu Asp Ala Ala Val Ala Tyr Asp Arg Ala Ala Phe Gln Leu
       125                 130                 135
   aga gga tcg aaa gct aag ctg aat ttt ccg cat ttg att ggt tct tgt      545
   Arg Gly Ser Lys Ala Lys Leu Asn Phe Pro His Leu Ile Gly Ser Cys
   140                 145                 150                 155
   aag tat gag ccg gtt agg att agg cct cgc cgt cgc tcg ccg gaa ccg      593
   Lys Tyr Glu Pro Val Arg Ile Arg Pro Arg Arg Arg Ser Pro Glu Pro
                   160                 165                 170
   tca gtc tcc gat cag tta acg tcg gag cag aag agg gaa agc cac gtg      641
   Ser Val Ser Asp Gln Leu Thr Ser Glu Gln Lys Arg Glu Ser His Val
               175                 180                 185
   gat gac ggc gag tct agt ttg gtt gta ccg gag ttg gat ttc acg gtg      689
   Asp Asp Gly Glu Ser Ser Leu Val Val Pro Glu Leu Asp Phe Thr Val
           190                 195                 200
   gat cag ttt tac ttc gat ggt agt tta tta atg gac caa tca gaa tgt      737
   Asp Gln Phe Tyr Phe Asp Gly Ser Leu Leu Met Asp Gln Ser Glu Cys
       205                 210                 215
   tct tat tct gat aat cgg ata taa ttagttttaa gattaagcaa aatttgtcca     791
   Ser Tyr Ser Asp Asn Arg Ile
   220                 225
   acgagttttg ctgtatgaaa tatctatcga tgactcaaca ggttttgatc atgatcatat    851
   gtaatgtgat ggaaattaaa tattgacgtt tgtttttttg ttgtaaaaaa aaaaaaaaaa     911
   aa                                                                    913
<400> 2
<210> 3
   <211> 1195
   <212> DNA
   <213> Arabidopsis thaliana
<220>
   <221> CDS
   <222> (50)..(1096)
   <223> G188
<400> 3
   ctctcaccaa cataatcaaa gaagctttcc tcacgaattc aagatcgcc atg tcc tcc      58
                                                         Met Ser Ser
                                                         1
   gag gat tgg gat ctc ttc gcc gtc gtc aga agc tgc agc tct tct gtt       106
   Glu Asp Trp Asp Leu Phe Ala Val Val Arg Ser Cys Ser Ser Ser Val
       5                   10                  15
   tcc acc acc aat tct tgt gct ggt cat gaa gac gac ata gga aac tgt       154
   Ser Thr Thr Asn Ser Cys Ala Gly His Glu Asp Asp Ile Gly Asn Cys
   20                  25                  30                  35
   aaa caa caa caa gat cct cct cct cct cct ctg ttt caa gct tct tct       202
   Lys Gln Gln Gln Asp Pro Pro Pro Pro Pro Leu Phe Gln Ala Ser Ser
                   40                  45                  50
   tct tgc aac gag tta caa gat tct tgc aaa cca ttt tta ccc gtt act       250
   Ser Cys Asn Glu Leu Gln Asp Ser Cys Lys Pro Phe Leu Pro Val Thr
               55                  60                  65
   act act act act act act tgg tct cct cct cct cta ctt cct cct cct       298
   Thr Thr Thr Thr Thr Thr Trp Ser Pro Pro Pro Leu Leu Pro Pro Pro
           70                  75                  80
   aaa gcc tca tca cca tct ccc aat atc tta cta aaa caa gaa caa gta       346
   Lys Ala Ser Ser Pro Ser Pro Asn Ile Leu Leu Lys Gln Glu Gln Val
       85                  90                  95
   ctt ctc gaa tca caa gat caa aaa cct cct ctt agt gtt agg gtt ttc       394
   Leu Leu Glu Ser Gln Asp Gln Lys Pro Pro Leu Ser Val Arg Val Phe
   100                 105                 110                 115
   cca cca tcc act tct tct tct gtc ttt gtt ttt aga ggt caa cgc gac       442
   Pro Pro Ser Thr Ser Ser Ser Val Phe Val Phe Arg Gly Gln Arg Asp
                   120                 125                 130
   cag ctt ctt caa caa caa tcc caa cct ccc ctt cga tct aga aaa aga       490
   Gln Leu Leu Gln Gln Gln Ser Gln Pro Pro Leu Arg Ser Arg Lys Arg
               135                 140                 145
   aag aat cag caa aaa aga acc ata tgt cat gta acg caa gag aat ctt      538
   Lys Asn Gln Gln Lys Arg Thr Ile Cys His Val Thr Gln Glu Asn Leu
           150                 155                 160
   tct tct gat ttg tgg gct tgg cgt aaa tac ggt caa aaa ccc atc aaa      586
   Ser Ser Asp Leu Trp Ala Trp Arg Lys Tyr Gly Gln Lys Pro Ile Lys
       165                 170                 175
   ggc tct cct tat cca agg aat tat tac aga tgt agt agc tca aaa gga      634
   Gly Ser Pro Tyr Pro Arg Asn Tyr Tyr Arg Cys Ser Ser Ser Lys Gly
   180                 185                 190                 195
   tgt tta gca cga aaa caa gtt gaa aga agt aat tta gat cct aat atc      682
   Cys Leu Ala Arg Lys Gln Val Glu Arg Ser Asn Leu Asp Pro Asn Ile
                   200                 205                 210
   ttc atc gtt act tac acc gga gaa cac act cat cca cgt cct act cac      730
   Phe Ile Val Thr Tyr Thr Gly Glu His Thr His Pro Arg Pro Thr His
               215                 220                 225
   cgg aac tct ctc gcc gga agt act cgt aac aaa tct cag ccc gtt aac      778
   Arg Asn Ser Leu Ala Gly Ser Thr Arg Asn Lys Ser Gln Pro Val Asn
           230                 235                 240
   ccg gtt cct aaa ccg gac aca tct cct tta tcg gat aca gta aaa gaa      826
   Pro Val Pro Lys Pro Asp Thr Ser Pro Leu Ser Asp Thr Val Lys Glu
       245                 250                 255
   gag att cat ctt tct ccg acg aca ccg ttg aaa gga aac gat gac gtt      874
   Glu Ile His Leu Ser Pro Thr Thr Pro Leu Lys Gly Asn Asp Asp Val
   260                 265                 270                 275
   caa gaa acg aat gga gat gaa gat atg gtt ggt caa gaa gtc aac atg      922
   Gln Glu Thr Asn Gly Asp Glu Asp Met Val Gly Gln Glu Val Asn Met
                   280                 285                 290
   gaa gag gaa gag gag gaa gaa gaa gtg gaa gaa gat gat gaa gaa gaa      970
   Glu Glu Glu Glu Glu Glu Glu Glu Val Glu Glu Asp Asp Glu Glu Glu
               295                 300                 305
   gaa gat gat gat gac gtg gat gat ctt ttg ata cca aat tta gcg gtg     1018
   Glu Asp Asp Asp Asp Val Asp Asp Leu Leu Ile Pro Asn Leu Ala Val
           310                 315                 320
   aga gat cga gat gat ttg ttc ttc gct gga agt ttt cca tct tgg tcc     1066
   Arg Asp Arg Asp Asp Leu Phe Phe Ala Gly Ser Phe Pro Ser Trp Ser
       325                 330                 335
   gcc gga tcc gcc ggt gac ggt ggt gga tga tgaaaacgaa taaaatctca       1116
   Ala Gly Ser Ala Gly Asp Gly Gly Gly
   340                 345
   atttacaatt tacaaaaaga aaaaagtcag tttttaatta ttatttttgt ttgttaaaac   1176
   ttgacattta ttgtgttat                                                1195
<210> 4
   <211> 348
   <212> PRT
   <213> Arabidopsis thaliana
<400> 4
   Met Ser Ser Glu Asp Trp Asp Leu Phe Ala Val Val Arg Ser Cys Ser
   1               5                   10                  15
   Ser Ser Val Ser Thr Thr Asn Ser Cys Ala Gly His Glu Asp Asp Ile
               20                  25                  30
   Gly Asn Cys Lys Gln Gln Gln Asp Pro Pro Pro Pro Pro Leu Phe Gln
       35                  40                  45
   Ala Ser Ser Ser Cys Asn Glu Leu Gln Asp Ser Cys Lys Pro Phe Leu
       50                  55                  60
   Pro Val Thr Thr Thr Thr Thr Thr Thr Trp Ser Pro Pro Pro Leu Leu
   65                  70                  75                  80
   Pro Pro Pro Lys Ala Ser Ser Pro Ser Pro Asn Ile Leu Leu Lys Gln
                   85                  90                  95
   Glu Gln Val Leu Leu Glu Ser Gln Asp Gln Lys Pro Pro Leu Ser Val
               100                 105                 110
   Arg Val Phe Pro Pro Ser Thr Ser Ser Ser Val Phe Val Phe Arg Gly
           115                 120                 125
   Gln Arg Asp Gln Leu Leu Gln Gln Gln Ser Gln Pro Pro Leu Arg Ser
       130                 135                 140
   Arg Lys Arg Lys Asn Gln Gln Lys Arg Thr Ile Cys His Val Thr Gln
   145                 150                 155                 160
   Glu Asn Leu Ser Ser Asp Leu Trp Ala Trp Arg Lys Tyr Gly Gln Lys
                   165                 170                 175
   Pro Ile Lys Gly Ser Pro Tyr Pro Arg Asn Tyr Tyr Arg Cys Ser Ser
               180                 185                 190
   Ser Lys Gly Cys Leu Ala Arg Lys Gln Val Glu Arg Ser Asn Leu Asp
           195                 200                 205
   Pro Asn Ile Phe Ile Val Thr Tyr Thr Gly Glu His Thr His Pro Arg
       210                 215                 220
   Pro Thr His Arg Asn Ser Leu Ala Gly Ser Thr Arg Asn Lys Ser Gln
   225                 230                 235                 240
   Pro Val Asn Pro Val Pro Lys Pro Asp Thr Ser Pro Leu Ser Asp Thr
                   245                 250                 255
   Val Lys Glu Glu Ile His Leu Ser Pro Thr Thr Pro Leu Lys Gly Asn
               260                 265                 270
   Asp Asp Val Gln Glu Thr Asn Gly Asp Glu Asp Met Val Gly Gln Glu
           275                 280                 285
   Val Asn Met Glu Glu Glu Glu Glu Glu Glu Glu Val Glu Glu Asp Asp
       290                 295                 300
   Glu Glu Glu Glu Asp Asp Asp Asp Val Asp Asp Leu Leu Ile Pro Asn
   305                 310                 315                 320
   Leu Ala Val Arg Asp Arg Asp Asp Leu Phe Phe Ala Gly Ser Phe Pro
                   325                 330                 335
   Ser Trp Ser Ala Gly Ser Ala Gly Asp Gly Gly Gly
               340                 345
<210> 5
   <211> 584
   <212> DNA
   <213> Arabidopsis thaliana
<220>
   <221> CDS
   <222> (157)..(441)
   <223> G225
<400> 5
   ctctctctct cactcttttc ttttccgaga acccaacaaa aaaaaagcta ctattaatcc      60
   ttcccctcgt gaggaaatca tttcttcttg tttctcgaga tttattctct ttctctctct     120
   ctttctctgt gtgtttcgtg tcttcagatt agttcg atg ttt cgt tca gac aag       174
                                           Met Phe Arg Ser Asp Lys
                                           1               5
   gcg gaa aaa atg gat aaa cga cga cgg aga cag agc aaa gcc aag gct       222
   Ala Glu Lys Met Asp Lys Arg Arg Arg Arg Gln Ser Lys Ala Lys Ala
               10                  15                  20
   tct tgt tcc gaa gag gtg agt agt atc gaa tgg gaa gct gtg aag atg       270
   Ser Cys Ser Glu Glu Val Ser Ser Ile Glu Trp Glu Ala Val Lys Met
           25                  30                  35
   tca gaa gaa gaa gaa gat ctc att tct cgg atg tat aaa ctc gtt ggc       318
   Ser Glu Glu Glu Glu Asp Leu Ile Ser Arg Met Tyr Lys Leu Val Gly
       40                  45                  50
   gac agg tgg gag ttg atc gcc gga agg atc ccg gga cgg acg ccg gag       366
   Asp Arg Trp Glu Leu Ile Ala Gly Arg Ile Pro Gly Arg Thr Pro Glu
   55                  60                  65                  70
   gag ata gag aga tat tgg ctt atg aaa cac ggc gtc gtt ttt gcc aac       414
   Glu Ile Glu Arg Tyr Trp Leu Met Lys His Gly Val Val Phe Ala Asn
                   75                  80                  85
   aga cga aga gac ttt ttt agg aaa tga ttttttttgt ttggattaaa             461
   Arg Arg Arg Asp Phe Phe Arg Lys
               90
   agaaaatttt cctctcctta attcacaaga caagaaaaaa aggaaatgta cctgtccttg     521
   aattactatt ttggaatgta taattatcta tatatataag aagaaaaaat tgcttaggaa     581
   ttt                                                                   584
<210> 6
   <211> 94
   <212> PRT
   <213> Arabidopsis thaliana
<400> 6
   Met Phe Arg Ser Asp Lys Ala Glu Lys Met Asp Lys Arg Arg Arg Arg
   1               5                   10                  15
   Gln Ser Lys Ala Lys Ala Ser Cys Ser Glu Glu Val Ser Ser Ile Glu
               20                  25                  30
   Trp Glu Ala Val Lys Met Ser Glu Glu Glu Glu Asp Leu Ile Ser Arg
           35                  40                  45
   Met Tyr Lys Leu Val Gly Asp Arg Trp Glu Leu Ile Ala Gly Arg Ile
       50                  55                  60
   Pro Gly Arg Thr Pro Glu Glu Ile Glu Arg Tyr Trp Leu Met Lys His
   65                  70                  75                  80
   Gly Val Val Phe Ala Asn Arg Arg Arg Asp Phe Phe Arg Lys
                   85                  90
<210> 7
   <211> 407
   <212> DNA
   <213> Arabidopsis thaliana
<220>
   <221> CDS
   <222> (10)..(348)
   <223> G226
<400> 7
   ccagtagtt atg gat aat acc aac cgt ctt cgt ctt cgt cgc ggt ccc agt      51
             Met Asp Asn Thr Asn Arg Leu Arg Leu Arg Arg Gly Pro Ser
             1               5                   10
   ctt agg caa act aag ttc act cga tcc cga tat gac tct gaa gaa gtg        99
   Leu Arg Gln Thr Lys Phe Thr Arg Ser Arg Tyr Asp Ser Glu Glu Val
   15                  20                  25                  30
   agt agc atc gaa tgg gag ttt atc agt atg acc gaa caa gaa gaa gat       147
   Ser Ser Ile Glu Trp Glu Phe Ile Ser Met Thr Glu Gln Glu Glu Asp
                   35                  40                  45
   ctc atc tct cga atg tac aga ctt gtc ggt aat agg tgg gat tta ata       195
   Leu Ile Ser Arg Met Tyr Arg Leu Val Gly Asn Arg Trp Asp Leu Ile
               50                  55                  60
   gca gga aga gtc gta gga aga aag gca aat gag att gag aga tac tgg       243
   Ala Gly Arg Val Val Gly Arg Lys Ala Asn Glu Ile Glu Arg Tyr Trp
           65                  70                  75
   att atg aga aac tct gac tat ttt tct cac aaa cga cga cgt ctt aat       291
   Ile Met Arg Asn Ser Asp Tyr Phe Ser His Lys Arg Arg Arg Leu Asn
       80                  85                  90
   aat tct ccc ttt ttt tct act tct cct ctt aat ctc caa gaa aat cta       339
   Asn Ser Pro Phe Phe Ser Thr Ser Pro Leu Asn Leu Gln Glu Asn Leu
   95                  100                 105                 110
   aaa ttg taa agaaatcaaa ataaaagctt tcaatcataa aagtagaaca               388
   Lys Leu
   aatcttgaat gtcttctca                                                   407
<210> 8
   <211> 112
   <212> PRT
   <213> Arabidopsis thaliana
<400> 8
   Met Asp Asn Thr Asn Arg Leu Arg Leu Arg Arg Gly Pro Ser Leu Arg
   1               5                   10                  15
   Gln Thr Lys Phe Thr Arg Ser Arg Tyr Asp Ser Glu Glu Val Ser Ser
               20                  25                  30
   Ile Glu Trp Glu Phe Ile Ser Met Thr Glu Gln Glu Glu Asp Leu Ile
   35 40 45
   Ser Arg Met Tyr Arg Leu Val Gly Asn Arg Trp Asp Leu Ile Ala Gly
   50 55 60
   Arg Val Val Gly Arg Lys Ala Asn Glu Ile Glu Arg Tyr Trp Ile Met
   65 70 75 80
   Arg Asn Ser Asp Tyr Phe Ser His Lys Arg Arg Arg Leu Asn Asn Ser
   85 90 95
   Pro Phe Phe Ser Thr Ser Pro Leu Asn Leu Gln Glu Asn Leu Lys Leu
   100 105 110
<210> 9
   <211> 1547
   <212> DNA
   <213> Arabidopsis
<220>
   <221> CDS
   <222> (312)..(1310)
   <223> G256
<400> 9
   tcgtgagcgt tgtgtttctc ctcaacattc aaagtcttta gtgaaacctc tcttgtaaga    60
   agccaaaaaa ataaagagaa agattcaaag aaggaaagaa attgaggatg actatttcaa   120
   gtccaaagag agattttgag tagaccctct tcacaaaaat ccaatcttag agtcttacta   180
   gttactatct agcttacata cacagagaca ctataccaaa aatccaatct tattagagta   240
   cttactatat agcttacaca tacacacaca cgaagtacta tttcaacgat caagagcgtg   300
   tgcgtgagga t atg ggt aga cca cct tgt tgc gag aag att gag gtg aag    350
                Met Gly Arg Pro Pro Cys Cys Glu Lys Ile Glu Val Lys
                1               5                   10
   aaa gga cca tgg act ccc gaa gaa gac ata atc ttg gtc tct tat atc     398
   Lys Gly Pro Trp Thr Pro Glu Glu Asp Ile Ile Leu Val Ser Tyr Ile
       15                  20                  25
   caa caa cac ggc cct gga aat tgg aga tct gtc cct gca aac acc ggt     446
   Gln Gln His Gly Pro Gly Asn Trp Arg Ser Val Pro Ala Asn Thr Gly
   30                  35                  40                  45
   ttg cta agg tgt agc aag agt tgc aga ctt aga tgg act aat tac ctt     494
   Leu Leu Arg Cys Ser Lys Ser Cys Arg Leu Arg Trp Thr Asn Tyr Leu
                   50                  55                  60
   cgt ccc ggg atc aaa cga gga aat ttc act caa ccg gaa gag aag atg     542
   Arg Pro Gly Ile Lys Arg Gly Asn Phe Thr Gln Pro Glu Glu Lys Met
               65                  70                  75
   atc atc cac ctt caa gct ctt ttg gga aat aga tgg gca gct ata gca     590
   Ile Ile His Leu Gln Ala Leu Leu Gly Asn Arg Trp Ala Ala Ile Ala
       80                  85                  90
   tca tat cta cct cag agg acc gac aat gat atc aag aac tac tgg aac     638
   Ser Tyr Leu Pro Gln Arg Thr Asp Asn Asp Ile Lys Asn Tyr Trp Asn
       95                  100                 105
   act cat ctt aaa aag aaa cta gtg atg atg aag ttt caa aat ggt atc     686
   Thr His Leu Lys Lys Lys Leu Val Met Met Lys Phe Gln Asn Gly Ile
   110                 115                 120                 125
   atc aac gaa aac aaa acc aat ctg gca aca gat att tcg tct tgt aat     734
   Ile Asn Glu Asn Lys Thr Asn Leu Ala Thr Asp Ile Ser Ser Cys Asn
                   130                 135                 140
   aat aac aac aat gga tgt aat cac aac aaa agg acc acc aac aaa ggc      782
   Asn Asn Asn Asn Gly Cys Asn His Asn Lys Arg Thr Thr Asn Lys Gly
               145                 150                 155
   caa tgg gag aaa aaa ctt caa aca gac atc aac atg gcc aaa caa gcc      830
   Gln Trp Glu Lys Lys Leu Gln Thr Asp Ile Asn Met Ala Lys Gln Ala
           160                 165                 170
   tta ttc caa gcc ttg tca ctt gac caa cca tct tca ttg atc cct ccc      878
   Leu Phe Gln Ala Leu Ser Leu Asp Gln Pro Ser Ser Leu Ile Pro Pro
       175                 180                 185
   gat cct gac tca cca aaa cct cat cat cat tct acc acc act tat gcc      926
   Asp Pro Asp Ser Pro Lys Pro His His His Ser Thr Thr Thr Tyr Ala
   190                 195                 200                 205
   tca agc aca gat aac atc tct aaa tta ctc cag aac tgg aca agc tca      974
   Ser Ser Thr Asp Asn Ile Ser Lys Leu Leu Gln Asn Trp Thr Ser Ser
                   210                 215                 220
   tca tcg tca aag cct aac act tca tca gtc tcc aac aac cgg agc tca     1022
   Ser Ser Ser Lys Pro Asn Thr Ser Ser Val Ser Asn Asn Arg Ser Ser
               225                 230                 235
   agc ccc ggt gaa gga gga ctt ttt gat cat cac tct ttg ttc tca tcg     1070
   Ser Pro Gly Glu Gly Gly Leu Phe Asp His His Ser Leu Phe Ser Ser
           240                 245                 250
   aat tca gaa tct gga tca gtt gat gag aag ctg aat ttg atg tcc gag     1118
   Asn Ser Glu Ser Gly Ser Val Asp Glu Lys Leu Asn Leu Met Ser Glu
       255                 260                 265
   aca agc atg ttc aaa ggt gag agc aag cca gac ata gac atg gaa gct     1166
   Thr Ser Met Phe Lys Gly Glu Ser Lys Pro Asp Ile Asp Met Glu Ala
   270                 275                 280                 285
   aca cct act act act act act act act gat gat caa ggc tcg ttg tca     1214
   Thr Pro Thr Thr Thr Thr Thr Thr Thr Asp Asp Gln Gly Ser Leu Ser
                   290                 295                 300
   ttg atc gag aaa tgg ttg ttt gat gat caa ggc ttg gtt cag tgt gat     1262
   Leu Ile Glu Lys Trp Leu Phe Asp Asp Gln Gly Leu Val Gln Cys Asp
               305                 310                 315
   gat agt caa gaa gat ctc atc gac gtg tct tta gag gag tta aaa taa     1310
   Asp Ser Gln Glu Asp Leu Ile Asp Val Ser Leu Glu Glu Leu Lys
           320                 325                 330
   tgataacaac agtcaagatt tgttctataa gaaaataaaa cgtatagaac aacgataaag   1370
   ctagctaggt ttattaattt ttctttcttt tgtcttttct ctatgatctt tagttacatt   1430
   ttattttact gtgtggcttg cttgtggtca agtcgatgaa gatcaaactg tgatatacta   1490
   tttatatgta aagtactata aagttaagag tagttgaata aaaaaaaaaa aaaaaaa      1547
<210> 10
   <211> 332
   <212> PRT
   <213> Arabidopsis
<400> 10
   Met Gly Arg Pro Pro Cys Cys Glu Lys Ile Glu Val Lys Lys Gly Pro
   1               5                   10                  15
   Trp Thr Pro Glu Glu Asp Ile Ile Leu Val Ser Tyr Ile Gln Gln His
               20                  25                  30
   Gly Pro Gly Asn Trp Arg Ser Val Pro Ala Asn Thr Gly Leu Leu Arg
           35                  40                  45
   Cys Ser Lys Ser Cys Arg Leu Arg Trp Thr Asn Tyr Leu Arg Pro Gly
       50                  55                  60
   Ile Lys Arg Gly Asn Phe Thr Gln Pro Glu Glu Lys Met Ile Ile His
   65                  70                  75                  80
   Leu Gln Ala Leu Leu Gly Asn Arg Trp Ala Ala Ile Ala Ser Tyr Leu
                   85                  90                  95
   Pro Gln Arg Thr Asp Asn Asp Ile Lys Asn Tyr Trp Asn Thr His Leu
               100                 105                 110
   Lys Lys Lys Leu Val Met Met Lys Phe Gln Asn Gly Ile Ile Asn Glu
           115                 120                 125
   Asn Lys Thr Asn Leu Ala Thr Asp Ile Ser Ser Cys Asn Asn Asn Asn
       130                 135                 140
   Asn Gly Cys Asn His Asn Lys Arg Thr Thr Asn Lys Gly Gln Trp Glu
   145                 150                 155                 160
   Lys Lys Leu Gln Thr Asp Ile Asn Met Ala Lys Gln Ala Leu Phe Gln
                   165                 170                 175
   Ala Leu Ser Leu Asp Gln Pro Ser Ser Leu Ile Pro Pro Asp Pro Asp
               180                 185                 190
   Ser Pro Lys Pro His His His Ser Thr Thr Thr Tyr Ala Ser Ser Thr
           195                 200                 205
   Asp Asn Ile Ser Lys Leu Leu Gln Asn Trp Thr Ser Ser Ser Ser Ser
       210                 215                 220
   Lys Pro Asn Thr Ser Ser Val Ser Asn Asn Arg Ser Ser Ser Pro Gly
   225                 230                 235                 240
   Glu Gly Gly Leu Phe Asp His His Ser Leu Phe Ser Ser Asn Ser Glu
                   245                 250                 255
   Ser Gly Ser Val Asp Glu Lys Leu Asn Leu Met Ser Glu Thr Ser Met
               260                 265                 270
   Phe Lys Gly Glu Ser Lys Pro Asp Ile Asp Met Glu Ala Thr Pro Thr
           275                 280                 285
   Thr Thr Thr Thr Thr Thr Asp Asp Gln Gly Ser Leu Ser Leu Ile Glu
       290                 295                 300
   Lys Trp Leu Phe Asp Asp Gln Gly Leu Val Gln Cys Asp Asp Ser Gln
   305                 310                 315                 320
   Glu Asp Leu Ile Asp Val Ser Leu Glu Glu Leu Lys
                   325                 330
<210> 11
   <211> 2405
   <212> DNA
   <213> Arabidopsis thaliana
<220>
   <221> CDS
   <222> (381)..(2213)
   <223> G419
<400> 11
   aagccacaca atctcttttc ttctctctct ctctgttata tctcttctgt ttaattcttt      60
   tattcttctt cgtctatctt ctcctataat ctcttctctc tccctcttca cctaaagaat     120
   aagaagaaaa ataattcaca tctttatgca aactactttc ttgtagggtt ttaggagcta     180
   tctctattgt cttggttctg atacaaagtt ttgtaatttt catggtatga gaagatttgc     240
   ctttctattt tgtttattgg ttctttttaa ctttttcttg gagatgggtt cttgtagatc     300
   ttaatgaaac ttctgttttt gtcccaaaaa gagttttctt ttttcttctc ttctttttgg     360
   gttttcaatt cttgagagac atg gca aga gat cag ttc tat ggt cac aat aac     413
                         Met Ala Arg Asp Gln Phe Tyr Gly His Asn Asn
                         1               5                   10
   cat cat cat caa gag caa caa cat caa atg att aat cag atc caa ggg      461
   His His His Gln Glu Gln Gln His Gln Met Ile Asn Gln Ile Gln Gly
               15                  20                  25
   ttt gat gag aca aac caa aac cca acc gat cat cat cat tac aat cat      509
   Phe Asp Glu Thr Asn Gln Asn Pro Thr Asp His His His Tyr Asn His
           30                  35                  40
   cag atc ttt ggc tca aac tcc aac atg ggt atg atg ata gac ttc tct      557
   Gln Ile Phe Gly Ser Asn Ser Asn Met Gly Met Met Ile Asp Phe Ser
       45                  50                  55
   aag caa caa cag att agg atg aca agt ggt tcg gat cat cat cat cat      605
   Lys Gln Gln Gln Ile Arg Met Thr Ser Gly Ser Asp His His His His
   60                  65                  70                  75
   cat cat cag aca agt ggt ggt act gat cag aat cag ctt ctg gaa gat      653
   His His Gln Thr Ser Gly Gly Thr Asp Gln Asn Gln Leu Leu Glu Asp
                   80                  85                  90
   tct tca tct gcc atg aga cta tgc aat gtt aat aat gat ttc cca agt      701
   Ser Ser Ser Ala Met Arg Leu Cys Asn Val Asn Asn Asp Phe Pro Ser
               95                  100                 105
   gaa gta aat gat gag aga cca cca caa aga cca agc caa ggt ctt tcc      749
   Glu Val Asn Asp Glu Arg Pro Pro Gln Arg Pro Ser Gln Gly Leu Ser
           110                 115                 120
   ctt tct ctc tcc tct tca aat cct aca agc atc agt ctc caa tct ttc      797
   Leu Ser Leu Ser Ser Ser Asn Pro Thr Ser Ile Ser Leu Gln Ser Phe
       125                 130                 135
   gaa ctc aga ccc caa caa caa caa caa ggg tat tcc ggt aat aaa tca      845
   Glu Leu Arg Pro Gln Gln Gln Gln Gln Gly Tyr Ser Gly Asn Lys Ser
   140                 145                 150                 155
   aca caa cat cag aat ctc caa cac acg cag atg atg atg atg atg atg      893
   Thr Gln His Gln Asn Leu Gln His Thr Gln Met Met Met Met Met Met
                   160                 165                 170
   aat agt cac cac caa aac aac aac aat aac aat cat cag cat cat aat      941
   Asn Ser His His Gln Asn Asn Asn Asn Asn Asn His Gln His His Asn
               175                 180                 185
   cat cat cag ttt cag att ggg agt tcc aag tat ttg agt cca gct caa      989
   His His Gln Phe Gln Ile Gly Ser Ser Lys Tyr Leu Ser Pro Ala Gln
           190                 195                 200
   gag cta ctg agt gag ttt tgc agt ctt gga gta aag gaa agc gat gaa      1037
   Glu Leu Leu Ser Glu Phe Cys Ser Leu Gly Val Lys Glu Ser Asp Glu
       205                 210                 215
   gaa gtg atg atg atg aag cat aag aag aag caa aag ggt aaa caa caa      1085
   Glu Val Met Met Met Lys His Lys Lys Lys Gln Lys Gly Lys Gln Gln
   220                 225                 230                 235
   gaa gag tgg gac aca agt cac cac agc aac aat gat caa cat gac caa      1133
   Glu Glu Trp Asp Thr Ser His His Ser Asn Asn Asp Gln His Asp Gln
                   240                 245                 250
   tct gcg act act tct tca aag aaa cat gtt cca cca ctt cac tct ctt      1181
   Ser Ala Thr Thr Ser Ser Lys Lys His Val Pro Pro Leu His Ser Leu
               255                 260                 265
   gag ttc atg gaa ctt cag aaa aga aaa gcc aag ttg ctc tcc atg ctc      1229
   Glu Phe Met Glu Leu Gln Lys Arg Lys Ala Lys Leu Leu Ser Met Leu
           270                 275                 280
   gaa gag ctt aaa aga aga tat gga cat tac cga gag caa atg aga gtt      1277
   Glu Glu Leu Lys Arg Arg Tyr Gly His Tyr Arg Glu Gln Met Arg Val
       285                 290                 295
   gcg gcg gca gcc ttt gaa gcg gcg gtt gga cta gga ggg gca gag ata      1325
   Ala Ala Ala Ala Phe Glu Ala Ala Val Gly Leu Gly Gly Ala Glu Ile
   300                 305                 310                 315
   tac act gcg tta gcg tca agg gca atg tca aga cac ttt cgg tgt tta      1373
   Tyr Thr Ala Leu Ala Ser Arg Ala Met Ser Arg His Phe Arg Cys Leu
                   320                 325                 330
   aaa gac gga ctt gtg gga cag att caa gca aca agt caa gct ttg gga      1421
   Lys Asp Gly Leu Val Gly Gln Ile Gln Ala Thr Ser Gln Ala Leu Gly
               335                 340                 345
   gag aga gaa gag gat aat cgt gcg gtt tct att gca gca cgt gga gaa      1469
   Glu Arg Glu Glu Asp Asn Arg Ala Val Ser Ile Ala Ala Arg Gly Glu
           350                 355                 360
   act cca cgg ttg aga ttg ctc gat caa gct ttg cgg caa cag aaa tcg      1517
   Thr Pro Arg Leu Arg Leu Leu Asp Gln Ala Leu Arg Gln Gln Lys Ser
       365                 370                 375
   tat cgc caa atg act ctt gtt gac gct cat cct tgg cgt cca caa cgc      1565
   Tyr Arg Gln Met Thr Leu Val Asp Ala His Pro Trp Arg Pro Gln Arg
   380                 385                 390                 395
   ggc ttg cct gaa cgc gca gtc aca acg ttg aga gct tgg ctc ttt gaa      1613
   Gly Leu Pro Glu Arg Ala Val Thr Thr Leu Arg Ala Trp Leu Phe Glu
                   400                 405                 410
   cac ttt ctt cac cca tat ccg agc gat gtt gat aag cat ata ttg gcc      1661
   His Phe Leu His Pro Tyr Pro Ser Asp Val Asp Lys His Ile Leu Ala
               415                 420                 425
   cga caa act ggt tta tca aga agt cag gta tca aat tgg ttt att aat      1709
   Arg Gln Thr Gly Leu Ser Arg Ser Gln Val Ser Asn Trp Phe Ile Asn
           430                 435                 440
   gca aga gtt agg cta tgg aaa cca atg att gaa gaa atg tac tgt gaa      1757
   Ala Arg Val Arg Leu Trp Lys Pro Met Ile Glu Glu Met Tyr Cys Glu
       445                 450                 455
   gaa aca aga agt gaa caa atg gag att aca aac ccg atg atg atc gat      1805
   Glu Thr Arg Ser Glu Gln Met Glu Ile Thr Asn Pro Met Met Ile Asp
   460                 465                 470                 475
   act aaa ccg gac ccg gac cag ttg atc cgt gtc gaa ccg gaa tct tta      1853
   Thr Lys Pro Asp Pro Asp Gln Leu Ile Arg Val Glu Pro Glu Ser Leu
                   480                 485                 490
   tcc tca ata gtg aca aac cct aca tcc aaa tcc ggt cac aac tca acc      1901
   Ser Ser Ile Val Thr Asn Pro Thr Ser Lys Ser Gly His Asn Ser Thr
                   495                     500                     505
   cat gga acg atg tcg tta ggg tca acg ttt gac ttt tcc ttg tac ggt      1949
   His Gly Thr Met Ser Leu Gly Ser Thr Phe Asp Phe Ser Leu Tyr Gly
           510                 515                 520
   aac caa gct gtg aca tac gct ggt gaa gga ggg cca cgt ggt gac gtt      1997
   Asn Gln Ala Val Thr Tyr Ala Gly Glu Gly Gly Pro Arg Gly Asp Val
       525                 530                 535
   tcc ttg acg ctt ggg tta caa cgt aac gat ggt aac ggt ggt gtg agt      2045
   Ser Leu Thr Leu Gly Leu Gln Arg Asn Asp Gly Asn Gly Gly Val Ser
   540                 545                 550                 555
   tta gcg ttg tct cca gtg acg gct caa ggt ggc caa ctt ttc tac ggt      2093
   Leu Ala Leu Ser Pro Val Thr Ala Gln Gly Gly Gln Leu Phe Tyr Gly
                   560                 565                 570
   aga gac cac att gaa gaa gga ccg gtt caa tat tca gcg tcg atg tta      2141
   Arg Asp His Ile Glu Glu Gly Pro Val Gln Tyr Ser Ala Ser Met Leu
               575                 580                 585
   gat gat gat caa gtt cag aat ttg cct tat agg aat ttg atg gga gct      2189
   Asp Asp Asp Gln Val Gln Asn Leu Pro Tyr Arg Asn Leu Met Gly Ala
           590                 595                 600
   caa tta ctt cat gat att gtt tga gattaaaaga ttaggaccaa agttatcgat     2243
   Gln Leu Leu His Asp Ile Val
       605                 610
   acatattttc caaaaccgat tcggttatgt aacggtttag ttagataaaa accaaattag    2303
   atatttatat ataccgttgt ctgattggat tggaggattg gtggacaagg agatattatt    2363
   aatgtatgag ttagttggtt cgtcaaaaaa aaaaaaaaaa aa                       2405
<210> 12
   <211> 610
   <212> PRT
   <213> Arabidopsis thaliana
<400> 12
   Met Ala Arg Asp Gln Phe Tyr Gly His Asn Asn His His His Gln Glu
   1               5                   10                  15
   Gln Gln His Gln Met Ile Asn Gln Ile Gln Gly Phe Asp Glu Thr Asn
               20                  25                  30
   Gln Asn Pro Thr Asp His His His Tyr Asn His Gln Ile Phe Gly Ser
           35                  40                  45
   Asn Ser Asn Met Gly Met Met Ile Asp Phe Ser Lys Gln Gln Gln Ile
       50                  55                  60
   Arg Met Thr Ser Gly Ser Asp His His His His His His Gln Thr Ser
   65                  70                  75                  80
   Gly Gly Thr Asp Gln Asn Gln Leu Leu Glu Asp Ser Ser Ser Ala Met
                   85                  90                  95
   Arg Leu Cys Asn Val Asn Asn Asp Phe Pro Ser Glu Val Asn Asp Glu
               100                 105                 110
   Arg Pro Pro Gln Arg Pro Ser Gln Gly Leu Ser Leu Ser Leu Ser Ser
           115                 120                 125
   Ser Asn Pro Thr Ser Ile Ser Leu Gln Ser Phe Glu Leu Arg Pro Gln
   130 135 140
   Gln Gln Gln Gln Gly Tyr Ser Gly Asn Lys Ser Thr Gln His Gln Asn
   145 150 155 160
   Leu Gln His Thr Gln Met Met Met Met Met Met Asn Ser His His Gln
   165 170 175
   Asn Asn Asn Asn Asn Asn His Gln His His Asn His His Gln Phe Gln
   180 185 190
   Ile Gly Ser Ser Lys Tyr Leu Ser Pro Ala Gln Glu Leu Leu Ser Glu
   195 200 205
   Phe Cys Ser Leu Gly Val Lys Glu Ser Asp Glu Glu Val Met Met Met
   210 215 220
   Lys His Lys Lys Lys Gln Lys Gly Lys Gln Gln Glu Glu Trp Asp Thr
   225 230 235 240
   Ser His His Ser Asn Asn Asp Gln His Asp Gln Ser Ala Thr Thr Ser
   245 250 255
   Ser Lys Lys His Val Pro Pro Leu His Ser Leu Glu Phe Met Glu Leu
   260 265 270
   Gln Lys Arg Lys Ala Lys Leu Leu Ser Met Leu Glu Glu Leu Lys Arg
   275 280 285
   Arg Tyr Gly His Tyr Arg Glu Gln Met Arg Val Ala Ala Ala Ala Phe
   290 295 300
   Glu Ala Ala Val Gly Leu Gly Gly Ala Glu Ile Tyr Thr Ala Leu Ala
   305 310 315 320
   Ser Arg Ala Met Ser Arg His Phe Arg Cys Leu Lys Asp Gly Leu Val
   325 330 335
   Gly Gln Ile Gln Ala Thr Ser Gln Ala Leu Gly Glu Arg Glu Glu Asp
   340 345 350
   Asn Arg Ala Val Ser Ile Ala Ala Arg Gly Glu Thr Pro Arg Leu Arg
   355 360 365
   Leu Leu Asp Gln Ala Leu Arg Gln Gln Lys Ser Tyr Arg Gln Met Thr
   370 375 380
   Leu Val Asp Ala His Pro Trp Arg Pro Gln Arg Gly Leu Pro Glu Arg
   385 390 395 400
   Ala Val Thr Thr Leu Arg Ala Trp Leu Phe Glu His Phe Leu His Pro
   405 410 415
   Tyr Pro Ser Asp Val Asp Lys His Ile Leu Ala Arg Gln Thr Gly Leu
   420 425 430
   Ser Arg Ser Gln Val Ser Asn Trp Phe Ile Asn Ala Arg Val Arg Leu
           435                 440                 445
   Trp Lys Pro Met Ile Glu Glu Met Tyr Cys Glu Glu Thr Arg Ser Glu
       450                 455                 460
   Gln Met Glu Ile Thr Asn Pro Met Met Ile Asp Thr Lys Pro Asp Pro
   465                 470                 475                 480
   Asp Gln Leu Ile Arg Val Glu Pro Glu Ser Leu Ser Ser Ile Val Thr
                   485                 490                 495
   Asn Pro Thr Ser Lys Ser Gly His Asn Ser Thr His Gly Thr Met Ser
               500                 505                 510
   Leu Gly Ser Thr Phe Asp Phe Ser Leu Tyr Gly Asn Gln Ala Val Thr
           515                 520                 525
   Tyr Ala Gly Glu Gly Gly Pro Arg Gly Asp Val Ser Leu Thr Leu Gly
       530                 535                 540
   Leu Gln Arg Asn Asp Gly Asn Gly Gly Val Ser Leu Ala Leu Ser Pro
   545                 550                 555                 560
   Val Thr Ala Gln Gly Gly Gln Leu Phe Tyr Gly Arg Asp His Ile Glu
                   565                 570                 575
   Glu Gly Pro Val Gln Tyr Ser Ala Ser Met Leu Asp Asp Asp Gln Val
               580                 585                 590
   Gln Asn Leu Pro Tyr Arg Asn Leu Met Gly Ala Gln Leu Leu His Asp
           595                 600                 605
   Ile Val
       610
<210> 13
   <211> 989
   <212> DNA
   <213> Arabidopsis thaliana
<220>
   <221> CDS
   <222> (41)..(664)
   <223> G464
<400> 13
   ctctgctggt atcattggag tctagggttt tgttattgac atg cgt ggt gtg tca      55
                                               Met Arg Gly Val Ser
                                               1               5
   gaa ttg gag gtg ggg aag agt aat ctt ccg gcg gag agt gag ctg gaa     103
   Glu Leu Glu Val Gly Lys Ser Asn Leu Pro Ala Glu Ser Glu Leu Glu
                   10                  15                  20
   ttg gga tta ggg ctc agc ctc ggt ggt ggc gcg tgg aaa gag cgt ggg     151
   Leu Gly Leu Gly Leu Ser Leu Gly Gly Gly Ala Trp Lys Glu Arg Gly
               25                  30                  35
   agg att ctt act gct aag gat ttt cct tcc gtt ggg tct aaa cgc tct     199
   Arg Ile Leu Thr Ala Lys Asp Phe Pro Ser Val Gly Ser Lys Arg Ser
           40                  45                  50
   gct gaa tct tcc tct cac caa gga gct tct cct cct cgt tca agt caa     247
   Ala Glu Ser Ser Ser His Gln Gly Ala Ser Pro Pro Arg Ser Ser Gln
       55                  60                  65
   gtg gta gga tgg cca cca att ggg tta cac agg atg aac agt ttg gtt     295
   Val Val Gly Trp Pro Pro Ile Gly Leu His Arg Met Asn Ser Leu Val
   70                  75                  80                  85
   aat aac caa gct atg aag gca gca aga gcg gaa gaa gga gac ggg gag     343
   Asn Asn Gln Ala Met Lys Ala Ala Arg Ala Glu Glu Gly Asp Gly Glu
                   90                  95                  100
   aag aaa gtt gtg aag aat ggt gag ctc aaa gat gtg tca atg aag gtg     391
   Lys Lys Val Val Lys Asn Gly Glu Leu Lys Asp Val Ser Met Lys Val
               105                 110                 115
   aat ccg aaa gtt cag ggc tta ggg ttt gtt aag gtg aat atg gat gga     439
   Asn Pro Lys Val Gln Gly Leu Gly Phe Val Lys Val Asn Met Asp Gly
           120                 125                 130
   gtt ggt ata ggc aga aaa gtg gat atg aga gct cat tcg tct tac gaa     487
   Val Gly Ile Gly Arg Lys Val Asp Met Arg Ala His Ser Ser Tyr Glu
       135                 140                 145
   aac ttg gct cag acg ctt gag gaa atg ttc ttt gga atg aca ggt act     535
   Asn Leu Ala Gln Thr Leu Glu Glu Met Phe Phe Gly Met Thr Gly Thr
   150                 155                 160                 165
   act tgt cga gaa acg gtt aaa cct tta agg ctt tta gat gga tca tca     583
   Thr Cys Arg Glu Thr Val Lys Pro Leu Arg Leu Leu Asp Gly Ser Ser
                   170                 175                 180
   gac ttt gta ctc act tat gaa gat aag ggg att gga tgc ttg ttg gag     631
   Asp Phe Val Leu Thr Tyr Glu Asp Lys Gly Ile Gly Cys Leu Leu Glu
               185                 190                 195
   atg ttc cat gga gaa tgt tta tca act cgg tga aaaggcttcg gatcatggga   684
   Met Phe His Gly Glu Cys Leu Ser Thr Arg
           200                 205
   acctcagaag ctagtggact agctccaaga cgtcaagagc agaaggatag acaaagaaac   744
   aaccctgttt agcttccctt ccaaagctgg cattgtttat gtattgtttg aggtttgcaa   804
   tttactcgat actttttgaa gaaagtattt tggagaatat ggataaaagc atgcagaagc   864
   ttagatatga tttgaatccg gttttcggat atggttttgc ttaggtcatt caattcgtag   924
   ttttccagtt tgtttcttct ttggctgtgt accaattatc tatgttctgt gagagaaagc   984
   tcttg                                                                989
<210> 14
   <211> 207
   <212> PRT
   <213> Arabidopsis thaliana
<400> 14
   Met Arg Gly Val Ser Glu Leu Glu Val Gly Lys Ser Asn Leu Pro Ala
   1               5                   10                  15
   Glu Ser Glu Leu Glu Leu Gly Leu Gly Leu Ser Leu Gly Gly Gly Ala
               20                  25                  30
   Trp Lys Glu Arg Gly Arg Ile Leu Thr Ala Lys Asp Phe Pro Ser Val
           35                  40                  45
   Gly Ser Lys Arg Ser Ala Glu Ser Ser Ser His Gln Gly Ala Ser Pro
       50                  55                  60
   Pro Arg Ser Ser Gln Val Val Gly Trp Pro Pro Ile Gly Leu His Arg
   65                  70                  75                  80
   Met Asn Ser Leu Val Asn Asn Gln Ala Met Lys Ala Ala Arg Ala Glu
                   85                  90                  95
   Glu Gly Asp Gly Glu Lys Lys Val Val Lys Asn Gly Glu Leu Lys Asp
               100                 105                 110
   Val Ser Met Lys Val Asn Pro Lys Val Gln Gly Leu Gly Phe Val Lys
           115                 120                 125
   Val Asn Met Asp Gly Val Gly Ile Gly Arg Lys Val Asp Met Arg Ala
       130                 135                 140
   His Ser Ser Tyr Glu Asn Leu Ala Gln Thr Leu Glu Glu Met Phe Phe
   145                 150                 155                 160
   Gly Met Thr Gly Thr Thr Cys Arg Glu Thr Val Lys Pro Leu Arg Leu
                   165                 170                 175
   Leu Asp Gly Ser Ser Asp Phe Val Leu Thr Tyr Glu Asp Lys Gly Ile
               180                 185                 190
   Gly Cys Leu Leu Glu Met Phe His Gly Glu Cys Leu Ser Thr Arg
           195                 200                 205
<210> 15
   <211> 1065
   <212> DNA
   <213> Arabidopsis thaliana
<220>
   <221> CDS
   <222> (188)..(760)
   <223> G482
<400> 15
   tcgacccacg cgtccggaca cttaacaatt cacaccttct ctttttactc ttcctaaaac      60
   cctaaatttc ctcgcttcag tcttcccact caagtcaacc accaattgaa ttcgatttcg     120
   aatcattgat ggaaatgatt tgaaaaaaga gtaaagttta tttttttatt ccttgtaatt     180
   ttcagaa atg ggg gat tcc gac agg gat tcc ggt gga ggg caa aac ggg       229
           Met Gly Asp Ser Asp Arg Asp Ser Gly Gly Gly Gln Asn Gly
           1               5                   10
   aac aac cag aac gga cag tcc tcc ttg tct cca aga gag caa gac agg       277
   Asn Asn Gln Asn Gly Gln Ser Ser Leu Ser Pro Arg Glu Gln Asp Arg
   15                  20                  25                  30
   ttc ttg ccg atc gct aac gtc agc cgg atc atg aag aag gcc ttg ccc       325
   Phe Leu Pro Ile Ala Asn Val Ser Arg Ile Met Lys Lys Ala Leu Pro
                   35                  40                  45
   gcc aac gcc aag atc tct aaa gat gcc aaa gag acg atg cag gag tgt       373
   Ala Asn Ala Lys Ile Ser Lys Asp Ala Lys Glu Thr Met Gln Glu Cys
               50                  55                  60
   gtc tcc gag ttc atc agc ttc gtc acc gga gaa gca tct gat aag tgt       421
   Val Ser Glu Phe Ile Ser Phe Val Thr Gly Glu Ala Ser Asp Lys Cys
           65                  70                  75
   cag aag gag aag agg aag acg atc aac gga gac gat ttg ctc tgg gct       469
   Gln Lys Glu Lys Arg Lys Thr Ile Asn Gly Asp Asp Leu Leu Trp Ala
       80                  85                  90
   atg act act cta ggt ttt gag gat tat gtt gag cca ttg aaa gtt tac       517
   Met Thr Thr Leu Gly Phe Glu Asp Tyr Val Glu Pro Leu Lys Val Tyr
   95                  100                 105                 110
   ttg cag agg ttt agg gag atc gaa ggg gag agg act gga cta ggg agg       565
   Leu Gln Arg Phe Arg Glu Ile Glu Gly Glu Arg Thr Gly Leu Gly Arg
                   115                 120                 125
   cca cag act ggt ggt gag gtc gga gag cat cag aga gat gct gtc gga       613
   Pro Gln Thr Gly Gly Glu Val Gly Glu His Gln Arg Asp Ala Val Gly
               130                 135                 140
   gat ggc ggt ggg ttc tac ggt ggt ggt ggt ggg atg cag tat cac caa       661
   Asp Gly Gly Gly Phe Tyr Gly Gly Gly Gly Gly Met Gln Tyr His Gln
           145                 150                 155
   cat cat cag ttt ctt cac cag cag aac cat atg tat gga gcc aca ggt       709
   His His Gln Phe Leu His Gln Gln Asn His Met Tyr Gly Ala Thr Gly
       160                 165                 170
   ggc ggt agc gac agt gga ggt gga gct gcc tcc ggt agg aca agg act       757
   Gly Gly Ser Asp Ser Gly Gly Gly Ala Ala Ser Gly Arg Thr Arg Thr
   175                 180                 185                 190
   taa caaagattgg tgaagtggat ctctctctgt atatagatac ataaatacat            810
   gtatacacat gcctattttt acgacccata taaggtatct atcatgtgat agaacgaaca     870
   ttggtgttgg tgatgtaaaa tcagatgtgc attaagggtt tagattttga ggctgtgtaa     930
   aagaagatca agtgtgcttt gttggacaat aggattcact aacgaatctg cttcattgga     990
   tcttgtatgt aactaaagcc attgtattga atgcaaatgt tttcatttgg gatgctttaa    1050
   aaaaaaaaaa aaaaa                                                      1065
<210> 16
   <211> 190
   <212> PRT
   <213> Arabidopsis thaliana
<400> 16
   Met Gly Asp Ser Asp Arg Asp Ser Gly Gly Gly Gln Asn Gly Asn Asn
   1               5                   10                  15
   Gln Asn Gly Gln Ser Ser Leu Ser Pro Arg Glu Gln Asp Arg Phe Leu
               20                  25                  30
   Pro Ile Ala Asn Val Ser Arg Ile Met Lys Lys Ala Leu Pro Ala Asn
           35                  40                  45
   Ala Lys Ile Ser Lys Asp Ala Lys Glu Thr Met Gln Glu Cys Val Ser
       50                  55                  60
   Glu Phe Ile Ser Phe Val Thr Gly Glu Ala Ser Asp Lys Cys Gln Lys
   65                  70                  75                  80
   Glu Lys Arg Lys Thr Ile Asn Gly Asp Asp Leu Leu Trp Ala Met Thr
                   85                  90                  95
   Thr Leu Gly Phe Glu Asp Tyr Val Glu Pro Leu Lys Val Tyr Leu Gln
               100                 105                 110
   Arg Phe Arg Glu Ile Glu Gly Glu Arg Thr Gly Leu Gly Arg Pro Gln
           115                 120                 125
   Thr Gly Gly Glu Val Gly Glu His Gln Arg Asp Ala Val Gly Asp Gly
       130                 135                 140
   Gly Gly Phe Tyr Gly Gly Gly Gly Gly Met Gln Tyr His Gln His His
   145                 150                 155                 160
   Gln Phe Leu His Gln Gln Asn His Met Tyr Gly Ala Thr Gly Gly Gly
                   165                 170                 175
   Ser Asp Ser Gly Gly Gly Ala Ala Ser Gly Arg Thr Arg Thr
               180                 185                 190
<210> 17
   <211> 1409
   <212> DNA
   <213> Arabidopsis thaliana
<220>
   <221> CDS
   <222> (224)..(1093)
   <223> G502
<400> 17
   ttgatgccgc tcaatcccac tatccttcgc aaggaccctt cctctatata aggaagttca      60
   tttcatttgg agaggacacg ctgacaagct gactctagca gatctgggac cgtcgaccca     120
   cgcgtccgaa ttgattagga taggatcagg atcatcctca acaacctcct cctaattcct     180
   cctccattca tagtaacaat aatattaaga aagagggtaa act atg tca gaa tta       235
                                                   Met Ser Glu Leu
                                                   1
   tta cag ttg cct cca ggt ttc cga ttt cac cct acc gat gaa gag ctt       283
   Leu Gln Leu Pro Pro Gly Phe Arg Phe His Pro Thr Asp Glu Glu Leu
   5                   10                  15                  20
   gtc atg cac tat ctc tgc cgc aaa tgt gcc tct cag tcc atc gcc gtt       331
   Val Met His Tyr Leu Cys Arg Lys Cys Ala Ser Gln Ser Ile Ala Val
                   25                  30                  35
   ccg atc atc gct gag atc gat ctc tac aaa tac gat cca tgg gag ctt       379
   Pro Ile Ile Ala Glu Ile Asp Leu Tyr Lys Tyr Asp Pro Trp Glu Leu
               40                  45                  50
   cct ggt tta gcc ttg tat ggt gag aag gaa tgg tac ttc ttc tct ccc       427
   Pro Gly Leu Ala Leu Tyr Gly Glu Lys Glu Trp Tyr Phe Phe Ser Pro
           55                  60                  65
   agg gac aga aaa tat ccc aac ggt tcg cgt cct aac cgg tcc gct ggt       475
   Arg Asp Arg Lys Tyr Pro Asn Gly Ser Arg Pro Asn Arg Ser Ala Gly
       70                  75                  80
   tct ggt tac tgg aaa gct acc gga gct gat aaa ccg atc gga cta cct       523
   Ser Gly Tyr Trp Lys Ala Thr Gly Ala Asp Lys Pro Ile Gly Leu Pro
   85                  90                  95                  100
   aaa ccg gtc gga att aag aaa gct ctt gtt ttc tac gcc ggc aaa gct       571
   Lys Pro Val Gly Ile Lys Lys Ala Leu Val Phe Tyr Ala Gly Lys Ala
                   105                 110                 115
   cca aag gga gag aaa acc aat tgg atc atg cac gag tac cgt ctc gcc       619
   Pro Lys Gly Glu Lys Thr Asn Trp Ile Met His Glu Tyr Arg Leu Ala
               120                 125                 130
   gac gtt gac cgg tcc gtt cgc aag aag aag aat agt ctc agg ctg gat     667
   Asp Val Asp Arg Ser Val Arg Lys Lys Lys Asn Ser Leu Arg Leu Asp
           135                 140                 145
   gat tgg gtt ctc tgc cgg att tac aac aaa aaa gga gct acc gag agg     715
   Asp Trp Val Leu Cys Arg Ile Tyr Asn Lys Lys Gly Ala Thr Glu Arg
       150                 155                 160
   cgg gga cca ccg cct ccg gtt gtt tac ggc gac gaa atc atg gag gag     763
   Arg Gly Pro Pro Pro Pro Val Val Tyr Gly Asp Glu Ile Met Glu Glu
   165                 170                 175                 180
   aag ccg aag gtg acg gag atg gtt atg cct ccg ccg ccg caa cag aca     811
   Lys Pro Lys Val Thr Glu Met Val Met Pro Pro Pro Pro Gln Gln Thr
                   185                 190                 195
   agt gag ttc gcg tat ttc gac acg tcg gat tcg gtg ccg aag ctg cat     859
   Ser Glu Phe Ala Tyr Phe Asp Thr Ser Asp Ser Val Pro Lys Leu His
               200                 205                 210
   act acg gat tcg agt tgc tcg gag cag gtg gtg tcg ccg gag ttc acg     907
   Thr Thr Asp Ser Ser Cys Ser Glu Gln Val Val Ser Pro Glu Phe Thr
           215                 220                 225
   agc gag gtt cag agc gag ccc aag tgg aaa gat tgg tcg gcc gta agt     955
   Ser Glu Val Gln Ser Glu Pro Lys Trp Lys Asp Trp Ser Ala Val Ser
       230                 235                 240
   aat gac aat aac aat acc ctt gat ttt ggg ttt aat tac att gat gcc    1003
   Asn Asp Asn Asn Asn Thr Leu Asp Phe Gly Phe Asn Tyr Ile Asp Ala
   245                 250                 255                 260
   acc gtg gat aac gcg ttt gga gga gga ggg agt agt aat cag atg ttt    1051
   Thr Val Asp Asn Ala Phe Gly Gly Gly Gly Ser Ser Asn Gln Met Phe
                   265                 270                 275
   ccg cta cag gat atg ttc atg tac atg cag aag cct tac tag            1093
   Pro Leu Gln Asp Met Phe Met Tyr Met Gln Lys Pro Tyr
               280                 285
   aagggaattc ctttcctgcc gccgaaacgc aacgcaaaac gaccctcgtt tttgcgttta  1153
   tggcaacacg agaccgtttt atatggtcaa tgagtgtgcc gattcggcca ttagatttct  1213
   gttcagtctt cgtttattct atagaccgtc cgatttcaga tcatccctaa tcggacggtg  1273
   gtcgttggat gtatcagtag tgtattactg tgttaggtag aagaaaatcc acttgttctt  1333
   aaattggcat aaaagtcaga agctaatatt tatatgtgcc gcaatcaatt taatattttc  1393
   tgtctaaaaa aaaaaa                                                  1409
<210> 18
   <211> 289
   <212> PRT
   <213> Arabidopsis thaliana
<400> 18
   Met Ser Glu Leu Leu Gln Leu Pro Pro Gly Phe Arg Phe His Pro Thr
   1               5                   10                  15
   Asp Glu Glu Leu Val Met His Tyr Leu Cys Arg Lys Cys Ala Ser Gln
               20                  25                  30
   Ser Ile Ala Val Pro Ile Ile Ala Glu Ile Asp Leu Tyr Lys Tyr Asp
           35                  40                  45
   Pro Trp Glu Leu Pro Gly Leu Ala Leu Tyr Gly Glu Lys Glu Trp Tyr
       50                  55                  60
   Phe Phe Ser Pro Arg Asp Arg Lys Tyr Pro Asn Gly Ser Arg Pro Asn
   65                  70                  75                  80
   Arg Ser Ala Gly Ser Gly Tyr Trp Lys Ala Thr Gly Ala Asp Lys Pro
                   85                  90                  95
   Ile Gly Leu Pro Lys Pro Val Gly Ile Lys Lys Ala Leu Val Phe Tyr
               100                 105                 110
   Ala Gly Lys Ala Pro Lys Gly Glu Lys Thr Asn Trp Ile Met His Glu
           115                 120                 125
   Tyr Arg Leu Ala Asp Val Asp Arg Ser Val Arg Lys Lys Lys Asn Ser
       130                 135                 140
   Leu Arg Leu Asp Asp Trp Val Leu Cys Arg Ile Tyr Asn Lys Lys Gly
   145                 150                 155                 160
   Ala Thr Glu Arg Arg Gly Pro Pro Pro Pro Val Val Tyr Gly Asp Glu
                   165                 170                 175
   Ile Met Glu Glu Lys Pro Lys Val Thr Glu Met Val Met Pro Pro Pro
               180                 185                 190
   Pro Gln Gln Thr Ser Glu Phe Ala Tyr Phe Asp Thr Ser Asp Ser Val
           195                 200                 205
   Pro Lys Leu His Thr Thr Asp Ser Ser Cys Ser Glu Gln Val Val Ser
       210                 215                 220
   Pro Glu Phe Thr Ser Glu Val Gln Ser Glu Pro Lys Trp Lys Asp Trp
   225                 230                 235                 240
   Ser Ala Val Ser Asn Asp Asn Asn Asn Thr Leu Asp Phe Gly Phe Asn
                   245                 250                 255
   Tyr Ile Asp Ala Thr Val Asp Asn Ala Phe Gly Gly Gly Gly Ser Ser
               260                 265                 270
   Asn Gln Met Phe Pro Leu Gln Asp Met Phe Met Tyr Met Gln Lys Pro
           275                 280                 285
   Tyr
<210> 19
   <211> 1481
   <212> DNA
   <213> Arabidopsis thaliana
<220>
   <221> CDS
   <222> (181)..(1188)
   <223> G526
<400> 19
   cgacccacgc gtccgagatt ctctcccagc tagctttctc aattcatttt tctttcttca     60
   tcttcttctt gtgtgatctc tctttccaaa taagcttatc attcttacaa aaatatttct    120
   gggtttctga tattgttctt gttctcttga atctttatta cttgaaaaac atataaagtg    180
   atg gcg gtt gtg gtt gaa gaa ggt gtg gtg ttg aat cat gga ggt gaa      228
   Met Ala Val Val Val Glu Glu Gly Val Val Leu Asn His Gly Gly Glu
   1               5                   10                  15
   gag ctt gtg gat ttg cca cct ggt ttc agg ttt cat cca aca gac gaa      276
   Glu Leu Val Asp Leu Pro Pro Gly Phe Arg Phe His Pro Thr Asp Glu
               20                  25                  30
   gag atc ata aca tgt tac ctt aag gag aag gtt tta aac agc cga ttc      324
   Glu Ile Ile Thr Cys Tyr Leu Lys Glu Lys Val Leu Asn Ser Arg Phe
           35                  40                  45
   acg gct gtg gcc atg gga gaa gct gat ctc aac aag tgt gag cct tgg      372
   Thr Ala Val Ala Met Gly Glu Ala Asp Leu Asn Lys Cys Glu Pro Trp
       50                  55                  60
   gat ttg cca aag agg gca aag atg ggg gag aaa gag ttc tac ttc ttc      420
   Asp Leu Pro Lys Arg Ala Lys Met Gly Glu Lys Glu Phe Tyr Phe Phe
   65                  70                  75                  80
   tgt caa agg gac agg aag tat ccg act ggg atg agg acg aac cgt gcg      468
   Cys Gln Arg Asp Arg Lys Tyr Pro Thr Gly Met Arg Thr Asn Arg Ala
                   85                  90                  95
   acg gag tca gga tac tgg aaa gcc acc ggg aag gat aag gag atc ttc      516
   Thr Glu Ser Gly Tyr Trp Lys Ala Thr Gly Lys Asp Lys Glu Ile Phe
               100                 105                 110
   aaa ggc aaa ggt tgt ctc gtt ggg atg aag aaa aca ctt gtg ttt tat      564
   Lys Gly Lys Gly Cys Leu Val Gly Met Lys Lys Thr Leu Val Phe Tyr
           115                 120                 125
   aga gga aga gct cca aaa ggt gaa aag act aat tgg gtc atg cat gaa      612
   Arg Gly Arg Ala Pro Lys Gly Glu Lys Thr Asn Trp Val Met His Glu
       130                 135                 140
   tat cgt ctt gaa ggc aaa tat tcg tat tac aat ctc cca aaa tct gca      660
   Tyr Arg Leu Glu Gly Lys Tyr Ser Tyr Tyr Asn Leu Pro Lys Ser Ala
   145                 150                 155                 160
   agg gac gaa tgg gtc gtg tgt agg gtt ttt cac aag aac aat cct tct      708
   Arg Asp Glu Trp Val Val Cys Arg Val Phe His Lys Asn Asn Pro Ser
                   165                 170                 175
   acc aca acc caa cca atg acg aga ata ccc gtt gaa gat ttc aca agg      756
   Thr Thr Thr Gln Pro Met Thr Arg Ile Pro Val Glu Asp Phe Thr Arg
               180                 185                 190
   atg gat tct cta gag aac att gat cat ctc cta gac ttc tca tct ctt      804
   Met Asp Ser Leu Glu Asn Ile Asp His Leu Leu Asp Phe Ser Ser Leu
           195                 200                 205
   cct cct ctc ata gac ccg agt ttc atg agt caa acc gaa caa cca aac      852
   Pro Pro Leu Ile Asp Pro Ser Phe Met Ser Gln Thr Glu Gln Pro Asn
       210                 215                 220
   ttc aaa ccc atc aac cct cca act tac gat atc tca tca cca atc caa      900
   Phe Lys Pro Ile Asn Pro Pro Thr Tyr Asp Ile Ser Ser Pro Ile Gln
   225                 230                 235                 240
   ccc cat cat ttc aat tct tac caa tca atc ttt aac cac cag gtt ttt      948
   Pro His His Phe Asn Ser Tyr Gln Ser Ile Phe Asn His Gln Val Phe
                   245                 250                 255
   ggt tct gct tcg ggc tct acg tac aac aac aac aac gag atg atc aag      996
   Gly Ser Ala Ser Gly Ser Thr Tyr Asn Asn Asn Asn Glu Met Ile Lys
               260                 265                 270
   atg gag caa tca ctt gtt agt gta tct caa gaa aca tgc cta agc tca      1044
   Met Glu Gln Ser Leu Val Ser Val Ser Gln Glu Thr Cys Leu Ser Ser
           275                 280                 285
   gat gtg aac gcg aac atg act aca acc acg gag gta tct tcg ggt cct      1092
   Asp Val Asn Ala Asn Met Thr Thr Thr Thr Glu Val Ser Ser Gly Pro
       290                 295                 300
   gta atg aaa caa gaa atg ggg atg atg gga atg gtg aat ggt agc aag      1140
   Val Met Lys Gln Glu Met Gly Met Met Gly Met Val Asn Gly Ser Lys
   305                 310                 315                 320
   tcg tat gaa gat cta tgt gac ttg agg ggg gac ttg tgg gac ttc taa      1188
   Ser Tyr Glu Asp Leu Cys Asp Leu Arg Gly Asp Leu Trp Asp Phe
                   325                 330                 335
   ttaatcattt gactgtggtg aaagagtata tttgttggga tttaaatcat gttagttaat    1248
   acatatacat ataggattta ctagaggctt aatcctagtt aactattttc acttcattga    1308
   tattatttaa ttagttgatt gtttaattag tttatacttt atagtgtggt taaaaaagaa    1368
   aagaaaggat tgtgataatt tgggatttta gtgcataagt tatatctcaa tgtaaactgt    1428
   atttgtatcc aaaaaaaaaa aaaaaaaaaa aaaaaaaaaa aaaaaaaaaa aaa           1481
<210> 20
   <211> 335
   <212> PRT
   <213> Arabidopsis thaliana
<400> 20
   Met Ala Val Val Val Glu Glu Gly Val Val Leu Asn His Gly Gly Glu
   1               5                   10                  15
   Glu Leu Val Asp Leu Pro Pro Gly Phe Arg Phe His Pro Thr Asp Glu
               20                  25                  30
   Glu Ile Ile Thr Cys Tyr Leu Lys Glu Lys Val Leu Asn Ser Arg Phe
           35                  40                  45
   Thr Ala Val Ala Met Gly Glu Ala Asp Leu Asn Lys Cys Glu Pro Trp
       50                  55                  60
   Asp Leu Pro Lys Arg Ala Lys Met Gly Glu Lys Glu Phe Tyr Phe Phe
   65                  70                  75                  80
   Cys Gln Arg Asp Arg Lys Tyr Pro Thr Gly Met Arg Thr Asn Arg Ala
                   85                  90                  95
   Thr Glu Ser Gly Tyr Trp Lys Ala Thr Gly Lys Asp Lys Glu Ile Phe
               100                 105                 110
   Lys Gly Lys Gly Cys Leu Val Gly Met Lys Lys Thr Leu Val Phe Tyr
           115                 120                 125
   Arg Gly Arg Ala Pro Lys Gly Glu Lys Thr Asn Trp Val Met His Glu
       130                 135                 140
   Tyr Arg Leu Glu Gly Lys Tyr Ser Tyr Tyr Asn Leu Pro Lys Ser Ala
   145                 150                 155                 160
   Arg Asp Glu Trp Val Val Cys Arg Val Phe His Lys Asn Asn Pro Ser
   165 170 175
   Thr Thr Thr Gln Pro Met Thr Arg Ile Pro Val Glu Asp Phe Thr Arg
   180 185 190
   Met Asp Ser Leu Glu Asn Ile Asp His Leu Leu Asp Phe Ser Ser Leu
   195 200 205
   Pro Pro Leu Ile Asp Pro Ser Phe Met Ser Gln Thr Glu Gln Pro Asn
   210 215 220
   Phe Lys Pro Ile Asn Pro Pro Thr Tyr Asp Ile Ser Ser Pro Ile Gln
   225 230 235 240
   Pro His His Phe Asn Ser Tyr Gln Ser Ile Phe Asn His Gln Val Phe
   245 250 255
   Gly Ser Ala Ser Gly Ser Thr Tyr Asn Asn Asn Asn Glu Met Ile Lys
   260 265 270
   Met Glu Gln Ser Leu Val Ser Val Ser Gln Glu Thr Cys Leu Ser Ser
   275 280 285
   Asp Val Asn Ala Asn Met Thr Thr Thr Thr Glu Val Ser Ser Gly Pro
   290 295 300
   Val Met Lys Gln Glu Met Gly Met Met Gly Met Val Asn Gly Ser Lys
   305 310 315 320
   Ser Tyr Glu Asp Leu Cys Asp Leu Arg Gly Asp Leu Trp Asp Phe
   325 330 335
<210> 21
   <211> 890
   <212> DNA
   <213> Arabidopsis thaliana
<220>
   <221> CDS
   <222> (55)..(738)
   <223> G545
<400> 21
   gcaaccttca aactaaaact cgagagacaa gaaatcctca gaatctttaa ctta atg      57
                                                               Met
                                                               1
   gcg ctc gag gct ctt aca tca cca aga tta gct tct ccg att cct cct     105
   Ala Leu Glu Ala Leu Thr Ser Pro Arg Leu Ala Ser Pro Ile Pro Pro
               5                   10                  15
   ttg ttc gaa gat tct tca gtc ttc cat gga gtc gag cac tgg aca aag     153
   Leu Phe Glu Asp Ser Ser Val Phe His Gly Val Glu His Trp Thr Lys
           20                  25                  30
   ggt aag cga tct aag aga tca aga tcc gat ttc cac cac caa aac ctc     201
   Gly Lys Arg Ser Lys Arg Ser Arg Ser Asp Phe His His Gln Asn Leu
       35                  40                  45
   act gag gaa gag tat cta gct ttt tgc ctc atg ctt ctc gct cgc gac     249
   Thr Glu Glu Glu Tyr Leu Ala Phe Cys Leu Met Leu Leu Ala Arg Asp
   50                  55                  60                  65
   aac cgt cag cct cct cct cct ccg gcg gtg gag aag ttg agc tac aag     297
   Asn Arg Gln Pro Pro Pro Pro Pro Ala Val Glu Lys Leu Ser Tyr Lys
                   70                  75                  80
   tgt agc gtc tgc gac aag acg ttc tct tct tac caa gct ctc ggt ggt     345
   Cys Ser Val Cys Asp Lys Thr Phe Ser Ser Tyr Gln Ala Leu Gly Gly
               85                  90                  95
   cac aag gca agc cac cgt aag aac tta tca cag act ctc tcc ggc gga     393
   His Lys Ala Ser His Arg Lys Asn Leu Ser Gln Thr Leu Ser Gly Gly
           100                 105                 110
   gga gat gat cat tca acc tcg tcg gcg aca acc aca tcc gcc gtg act     441
   Gly Asp Asp His Ser Thr Ser Ser Ala Thr Thr Thr Ser Ala Val Thr
       115                 120                 125
   act gga agt ggg aaa tca cac gtt tgc acc atc tgt aac aag tct ttt     489
   Thr Gly Ser Gly Lys Ser His Val Cys Thr Ile Cys Asn Lys Ser Phe
   130                 135                 140                 145
   cct tcc ggt caa gct ctc ggc gga cac aag cgg tgc cac tac gaa gga     537
   Pro Ser Gly Gln Ala Leu Gly Gly His Lys Arg Cys His Tyr Glu Gly
                   150                 155                 160
   aac aac aac atc aac act agt agc gtg tcc aac tcc gaa ggt gcg ggg     585
   Asn Asn Asn Ile Asn Thr Ser Ser Val Ser Asn Ser Glu Gly Ala Gly
               165                 170                 175
   tcc act agc cac gtt agc agt agc cac cgt ggg ttt gac ctc aac atc     633
   Ser Thr Ser His Val Ser Ser Ser His Arg Gly Phe Asp Leu Asn Ile
           180                 185                 190
   cct ccg atc cct gaa ttc tcg atg gtc aac gga gac gac gaa gtc atg     681
   Pro Pro Ile Pro Glu Phe Ser Met Val Asn Gly Asp Asp Glu Val Met
       195                 200                 205
   agc cct atg ccg gcg aag aag cct cgg ttt gac ttt ccg gtc aaa ctt     729
   Ser Pro Met Pro Ala Lys Lys Pro Arg Phe Asp Phe Pro Val Lys Leu
   210                 215                 220                 225
   caa ctt taa ggaaatttac ttagacgata agatttcgtt tgtatactgt             778
   Gln Leu
   tgagagttgt gtaggaattt gttgactgta cataccaaat tggactttga ctgattccaa   838
   ttcttcttgt tctttcattt taaaaattat taaaccgatt ctttaccaca aa           890
<210> 22
   <211> 227
   <212> PRT
   <213> Arabidopsis thaliana
<400> 22
   Met Ala Leu Glu Ala Leu Thr Ser Pro Arg Leu Ala Ser Pro Ile Pro
   1 5 10 15
   Pro Leu Phe Glu Asp Ser Ser Val Phe His Gly Val Glu His Trp Thr
   20 25 30
   Lys Gly Lys Arg Ser Lys Arg Ser Arg Ser Asp Phe His His Gln Asn
   35 40 45
   Leu Thr Glu Glu Glu Tyr Leu Ala Phe Cys Leu Met Leu Leu Ala Arg
   50 55 60
   Asp Asn Arg Gln Pro Pro Pro Pro Pro Ala Val Glu Lys Leu Ser Tyr
   65 70 75 80
   Lys Cys Ser Val Cys Asp Lys Thr Phe Ser Ser Tyr Gln Ala Leu Gly
   85 90 95
   Gly His Lys Ala Ser His Arg Lys Asn Leu Ser Gln Thr Leu Ser Gly
   100 105 110
   Gly Gly Asp Asp His Ser Thr Ser Ser Ala Thr Thr Thr Ser Ala Val
   115 120 125
   Thr Thr Gly Ser Gly Lys Ser His Val Cys Thr Ile Cys Asn Lys Ser
   130 135 140
   Phe Pro Ser Gly Gln Ala Leu Gly Gly His Lys Arg Cys His Tyr Glu
   145 150 155 160
   Gly Asn Asn Asn Ile Asn Thr Ser Ser Val Ser Asn Ser Glu Gly Ala
   165 170 175
   Gly Ser Thr Ser His Val Ser Ser Ser His Arg Gly Phe Asp Leu Asn
   180 185 190
   Ile Pro Pro Ile Pro Glu Phe Ser Met Val Asn Gly Asp Asp Glu Val
   195 200 205
   Met Ser Pro Met Pro Ala Lys Lys Pro Arg Phe Asp Phe Pro Val Lys
   210 215 220
   Leu Gln Leu
   225
<210> 23
   <211> 1413
   <212> DNA
   <213> Arabidopsis thaliana
<220>
   <221> CDS
   <222> (86)..(1168)
   <223> G561
<400> 23
   aatttgtttt tttttctttt gtgggttcaa ttcgaattgt tttccctgag actcaagtta      60
   ctgtgtcatt actctgcatt gagca atg ggt agc aac gaa gaa gga aac ccc      112
                               Met Gly Ser Asn Glu Glu Gly Asn Pro
                               1               5
   act aac aac tct gat aag cca tcg caa gct gct gct cct gag cag agt      160
   Thr Asn Asn Ser Asp Lys Pro Ser Gln Ala Ala Ala Pro Glu Gln Ser
   10                  15                  20                  25
   aat gtt cat gtg tat cat cat gac tgg gct gct atg cag gca tat tat      208
   Asn Val His Val Tyr His His Asp Trp Ala Ala Met Gln Ala Tyr Tyr
                   30                  35                  40
   ggg cct aga gtt ggt ata cct caa tat tac aac tca aat ttg gcg cct      256
   Gly Pro Arg Val Gly Ile Pro Gln Tyr Tyr Asn Ser Asn Leu Ala Pro
               45                  50                  55
   ggt cat gct cca ccg cct tat atg tgg gcg tct cca tcg cca atg atg      304
   Gly His Ala Pro Pro Pro Tyr Met Trp Ala Ser Pro Ser Pro Met Met
           60                  65                  70
   gct cct tat gga gca cca tat cca cca ttt tgc cct cct ggt gga gtt     352
   Ala Pro Tyr Gly Ala Pro Tyr Pro Pro Phe Cys Pro Pro Gly Gly Val
       75                  80                  85
   tat gct cat cct ggt gtt caa atg ggc tca caa cca caa ggt cct gtt     400
   Tyr Ala His Pro Gly Val Gln Met Gly Ser Gln Pro Gln Gly Pro Val
   90                  95                  100                 105
   tct caa tca gca tct gga gtt aca acc cct ttg acc att gat gca cca     448
   Ser Gln Ser Ala Ser Gly Val Thr Thr Pro Leu Thr Ile Asp Ala Pro
                   110                 115                 120
   gct aat tca gct gga aac tca gat cat ggg ttc atg aaa aag ctg aaa     496
   Ala Asn Ser Ala Gly Asn Ser Asp His Gly Phe Met Lys Lys Leu Lys
               125                 130                 135
   gag ttc gat gga ctt gca atg tca ata agc aat aac aaa gtt ggg agt     544
   Glu Phe Asp Gly Leu Ala Met Ser Ile Ser Asn Asn Lys Val Gly Ser
           140                 145                 150
   gct gaa cat agc agc agt gaa cat agg agt tct cag agc tcc gag aat     592
   Ala Glu His Ser Ser Ser Glu His Arg Ser Ser Gln Ser Ser Glu Asn
       155                 160                 165
   gat ggc tct agc aat ggt agt gat ggt aat aca act ggg gga gaa caa     640
   Asp Gly Ser Ser Asn Gly Ser Asp Gly Asn Thr Thr Gly Gly Glu Gln
   170                 175                 180                 185
   tct agg agg aaa aga agg caa caa aga tca cca agc act ggt gaa aga     688
   Ser Arg Arg Lys Arg Arg Gln Gln Arg Ser Pro Ser Thr Gly Glu Arg
                   190                 195                 200
   ccc tca tct caa aac agt ctg cct ctt aga ggt gaa aat gag aaa ccc     736
   Pro Ser Ser Gln Asn Ser Leu Pro Leu Arg Gly Glu Asn Glu Lys Pro
               205                 210                 215
   gat gtg act atg ggg act cct gtt atg ccc aca gca atg agt ttc caa     784
   Asp Val Thr Met Gly Thr Pro Val Met Pro Thr Ala Met Ser Phe Gln
           220                 225                 230
   aac tct gct ggc atg aac ggt gtg cca cag cca tgg aat gaa aaa gag     832
   Asn Ser Ala Gly Met Asn Gly Val Pro Gln Pro Trp Asn Glu Lys Glu
       235                 240                 245
   gtt aaa cga gag aag aga aaa cag tca aac cga gaa tct gct agg agg     880
   Val Lys Arg Glu Lys Arg Lys Gln Ser Asn Arg Glu Ser Ala Arg Arg
   250                 255                 260                 265
   tca aga ctg agg aag cag gct gaa aca gaa caa cta tct gtc aaa gtt     928
   Ser Arg Leu Arg Lys Gln Ala Glu Thr Glu Gln Leu Ser Val Lys Val
                   270                 275                 280
   gac gca tta gta gct gag aac atg tct ctg agg tct aaa cta ggc cag     976
   Asp Ala Leu Val Ala Glu Asn Met Ser Leu Arg Ser Lys Leu Gly Gln
               285                 290                 295
   cta aac aat gag tct gag aaa cta cgg ctg gag aac gaa gct ata ttg    1024
   Leu Asn Asn Glu Ser Glu Lys Leu Arg Leu Glu Asn Glu Ala Ile Leu
           300                 305                 310
   gat caa ctg aaa gcg caa gca aca ggg aaa aca gag aac ctg atc tct    1072
   Asp Gln Leu Lys Ala Gln Ala Thr Gly Lys Thr Glu Asn Leu Ile Ser
       315                 320                 325
   cga gtt gat aag aac aac tct gta tca ggt agc aaa act gtg cag cat    1120
   Arg Val Asp Lys Asn Asn Ser Val Ser Gly Ser Lys Thr Val Gln His
   330                 335                 340                 345
   caa ctg tta aat gca agt ccg ata acc gat cct gtc gcg gct agc tga    1168
   Gln Leu Leu Asn Ala Ser Pro Ile Thr Asp Pro Val Ala Ala Ser
                   350                 355                 360
   ccgtggccgc aacaatgaga acccgatatt tcttcctttg ggttgtgatt gtaacttaaa   1228
   aggagacttt ttgtttttat tcttagattt gtagctctct gcatagtgag cataaattga   1288
   tgtaatatgg tttaagagat tcggtgttct ctggtgtgtg ctgcaaccac ataattggtg 1348
   atagataggt ttagttatat aagcaaatgt attagagata aggggagaca tatttgatgg 1408
   tcttt 1413
<210> 24
   <211> 360
   <212> PRT
   <213> Arabidopsis thaliana
<400> 24
   Met Gly Ser Asn Glu Glu Gly Asn Pro Thr Asn Asn Ser Asp Lys Pro
   1               5                   10                  15
   Ser Gln Ala Ala Ala Pro Glu Gln Ser Asn Val His Val Tyr His His
               20                  25                  30
   Asp Trp Ala Ala Met Gln Ala Tyr Tyr Gly Pro Arg Val Gly Ile Pro
           35                  40                  45
   Gln Tyr Tyr Asn Ser Asn Leu Ala Pro Gly His Ala Pro Pro Pro Tyr
       50                  55                  60
   Met Trp Ala Ser Pro Ser Pro Met Met Ala Pro Tyr Gly Ala Pro Tyr
   65                  70                  75                  80
   Pro Pro Phe Cys Pro Pro Gly Gly Val Tyr Ala His Pro Gly Val Gln
                   85                  90                  95
   Met Gly Ser Gln Pro Gln Gly Pro Val Ser Gln Ser Ala Ser Gly Val
               100                 105                 110
   Thr Thr Pro Leu Thr Ile Asp Ala Pro Ala Asn Ser Ala Gly Asn Ser
           115                 120                 125
   Asp His Gly Phe Met Lys Lys Leu Lys Glu Phe Asp Gly Leu Ala Met
       130                 135                 140
   Ser Ile Ser Asn Asn Lys Val Gly Ser Ala Glu His Ser Ser Ser Glu
   145                 150                 155                 160
   His Arg Ser Ser Gln Ser Ser Glu Asn Asp Gly Ser Ser Asn Gly Ser
                   165                 170                 175
   Asp Gly Asn Thr Thr Gly Gly Glu Gln Ser Arg Arg Lys Arg Arg Gln
               180                 185                 190
   Gln Arg Ser Pro Ser Thr Gly Glu Arg Pro Ser Ser Gln Asn Ser Leu
           195                 200                 205
   Pro Leu Arg Gly Glu Asn Glu Lys Pro Asp Val Thr Met Gly Thr Pro
       210                 215                 220
   Val Met Pro Thr Ala Met Ser Phe Gln Asn Ser Ala Gly Met Asn Gly
   225                 230                 235                 240
   Val Pro Gln Pro Trp Asn Glu Lys Glu Val Lys Arg Glu Lys Arg Lys
                   245                 250                 255
   Gln Ser Asn Arg Glu Ser Ala Arg Arg Ser Arg Leu Arg Lys Gln Ala
               260                 265                 270
   Glu Thr Glu Gln Leu Ser Val Lys Val Asp Ala Leu Val Ala Glu Asn
           275                 280                 285
   Met Ser Leu Arg Ser Lys Leu Gly Gln Leu Asn Asn Glu Ser Glu Lys
       290                 295                 300
   Leu Arg Leu Glu Asn Glu Ala Ile Leu Asp Gln Leu Lys Ala Gln Ala
   305                 310                 315                 320
   Thr Gly Lys Thr Glu Asn Leu Ile Ser Arg Val Asp Lys Asn Asn Ser
                   325                 330                 335
   Val Ser Gly Ser Lys Thr Val Gln His Gln Leu Leu Asn Ala Ser Pro
               340                 345                 350
   Ile Thr Asp Pro Val Ala Ala Ser
           355                 360
<210> 25
   <211> 1087
   <212> DNA
   <213> Arabidopsis thaliana
<220>
   <221> CDS
   <222> (104)..(952)
   <223> G664
<400> 25
   caatccctca atataaaata acaagtagaa ttgatctgcc tatatataag attttgagac      60
   gaaataagat ctaaaccaca agaaagaaag taaacataaa agt atg gga agg tca       115
                                                   Met Gly Arg Ser
                                                   1
   ccg tgc tgt gag aaa gct cac aca aac aaa gga gca tgg acg aaa gaa       163
   Pro Cys Cys Glu Lys Ala His Thr Asn Lys Gly Ala Trp Thr Lys Glu
   5                   10                  15                  20
   gag gac gag agg ctc gtc gcc tac att aaa gct cat gga gaa ggc tgc       211
   Glu Asp Glu Arg Leu Val Ala Tyr Ile Lys Ala His Gly Glu Gly Cys
                   25                  30                  35
   tgg aga tct ctc ccc aaa gcc gcc gga ctt ctt cgc tgt ggc aag agc       259
   Trp Arg Ser Leu Pro Lys Ala Ala Gly Leu Leu Arg Cys Gly Lys Ser
               40                  45                  50
   tgc cgt ctc cgg tgg atc aac tat ctc cgg cct gac ctt aag cgt gga       307
   Cys Arg Leu Arg Trp Ile Asn Tyr Leu Arg Pro Asp Leu Lys Arg Gly
           55                  60                  65
   aac ttc acc gag gaa gaa gac gaa ctc atc atc aag ctc cat agc ctt       355
   Asn Phe Thr Glu Glu Glu Asp Glu Leu Ile Ile Lys Leu His Ser Leu
       70                  75                  80
   ctt ggc aac aaa tgg tcg ctt att gcc ggg aga tta ccg gga aga aca       403
   Leu Gly Asn Lys Trp Ser Leu Ile Ala Gly Arg Leu Pro Gly Arg Thr
   85                  90                  95                  100
   gat aac gag ata aag aac tat tgg aac acg cat ata cga aga aag ctt       451
   Asp Asn Glu Ile Lys Asn Tyr Trp Asn Thr His Ile Arg Arg Lys Leu
                   105                 110                 115
   ata aac aga ggg att gat cca acg agt cat aga cca atc caa gaa tca      499
   Ile Asn Arg Gly Ile Asp Pro Thr Ser His Arg Pro Ile Gln Glu Ser
               120                 125                 130
   tca gct tct caa gat tct aaa cct aca caa cta gaa cca gtt acg agt      547
   Ser Ala Ser Gln Asp Ser Lys Pro Thr Gln Leu Glu Pro Val Thr Ser
           135                 140                 145
   aat acc att aat atc tca ttc act tct gct cca aag gtc gaa acg ttc      595
   Asn Thr Ile Asn Ile Ser Phe Thr Ser Ala Pro Lys Val Glu Thr Phe
       150                 155                 160
   cat gaa agt ata agc ttt ccg gga aaa tca gag aaa atc tca atg ctt      643
   His Glu Ser Ile Ser Phe Pro Gly Lys Ser Glu Lys Ile Ser Met Leu
   165                 170                 175                 180
   acg ttc aaa gaa gaa aaa gat gag tgc cca gtt caa gaa aag ttc cca      691
   Thr Phe Lys Glu Glu Lys Asp Glu Cys Pro Val Gln Glu Lys Phe Pro
                   185                 190                 195
   gat ttg aat ctt gag ctc aga atc agt ctt cct gat gat gtt gat cgt      739
   Asp Leu Asn Leu Glu Leu Arg Ile Ser Leu Pro Asp Asp Val Asp Arg
               200                 205                 210
   ctt caa ggg cat gga aag tca aca acg cca cgt tgt ttc aag tgc agc      787
   Leu Gln Gly His Gly Lys Ser Thr Thr Pro Arg Cys Phe Lys Cys Ser
           215                 220                 225
   tta ggg atg ata aac ggc atg gag tgc aga tgc gga aga atg aga tgc      835
   Leu Gly Met Ile Asn Gly Met Glu Cys Arg Cys Gly Arg Met Arg Cys
       230                 235                 240
   gat gta gtc gga ggt agc agc aag ggg agt gac atg agc aat gga ttt      883
   Asp Val Val Gly Gly Ser Ser Lys Gly Ser Asp Met Ser Asn Gly Phe
   245                 250                 255                 260
   gat ttt tta ggg ttg gca aag aaa gag acc act tct ctt ttg ggc ttt      931
   Asp Phe Leu Gly Leu Ala Lys Lys Glu Thr Thr Ser Leu Leu Gly Phe
                   265                 270                 275
   cga agc ttg gag atg aaa taa tattgtcaaa ttttaggcgt aactgtacaa         982
   Arg Ser Leu Glu Met Lys
               280
   aacttttgcc tagataattt gaaagtatat cttcaacttg tatgagaaat ttaactggtg    1042
   aattataata tatagaattt gttttttaaa aaaaaaaaaa aaaaa                      1087
<210> 26
   <211> 282
   <212> PRT
   <213> Arabidopsis thaliana
<400> 26
   Met Gly Arg Ser Pro Cys Cys Glu Lys Ala His Thr Asn Lys Gly Ala
   1               5                   10                  15
   Trp Thr Lys Glu Glu Asp Glu Arg Leu Val Ala Tyr Ile Lys Ala His
               20                  25                  30
   Gly Glu Gly Cys Trp Arg Ser Leu Pro Lys Ala Ala Gly Leu Leu Arg
           35                  40                  45
   Cys Gly Lys Ser Cys Arg Leu Arg Trp Ile Asn Tyr Leu Arg Pro Asp
       50                  55                  60
   Leu Lys Arg Gly Asn Phe Thr Glu Glu Glu Asp Glu Leu Ile Ile Lys
   65                  70                  75                  80
   Leu His Ser Leu Leu Gly Asn Lys Trp Ser Leu Ile Ala Gly Arg Leu
                   85                  90                  95
   Pro Gly Arg Thr Asp Asn Glu Ile Lys Asn Tyr Trp Asn Thr His Ile
               100                 105                 110
   Arg Arg Lys Leu Ile Asn Arg Gly Ile Asp Pro Thr Ser His Arg Pro
           115                 120                 125
   Ile Gln Glu Ser Ser Ala Ser Gln Asp Ser Lys Pro Thr Gln Leu Glu
       130                 135                 140
   Pro Val Thr Ser Asn Thr Ile Asn Ile Ser Phe Thr Ser Ala Pro Lys
   145                 150                 155                 160
   Val Glu Thr Phe His Glu Ser Ile Ser Phe Pro Gly Lys Ser Glu Lys
                   165                 170                 175
   Ile Ser Met Leu Thr Phe Lys Glu Glu Lys Asp Glu Cys Pro Val Gln
               180                 185                 190
   Glu Lys Phe Pro Asp Leu Asn Leu Glu Leu Arg Ile Ser Leu Pro Asp
           195                 200                 205
   Asp Val Asp Arg Leu Gln Gly His Gly Lys Ser Thr Thr Pro Arg Cys
       210                 215                 220
   Phe Lys Cys Ser Leu Gly Met Ile Asn Gly Met Glu Cys Arg Cys Gly
   225                 230                 235                 240
   Arg Met Arg Cys Asp Val Val Gly Gly Ser Ser Lys Gly Ser Asp Met
                   245                 250                 255
   Ser Asn Gly Phe Asp Phe Leu Gly Leu Ala Lys Lys Glu Thr Thr Ser
               260                 265                 270
   Leu Leu Gly Phe Arg Ser Leu Glu Met Lys
           275                 280
<210> 27
   <211> 228
   <212> DNA
   <213> Arabidopsis thaliana
<220>
   <221> CDS
   <222> (1)..(228)
   <223> G682
<400> 27
   atg gat aac cat cgc agg act aag caa ccc aag acc aac tcc atc gtt        48
   Met Asp Asn His Arg Arg Thr Lys Gln Pro Lys Thr Asn Ser Ile Val
   1               5                   10                  15
   act tct tct tct gaa gaa gtg agt agt ctt gag tgg gaa gtt gtg aac        96
   Thr Ser Ser Ser Glu Glu Val Ser Ser Leu Glu Trp Glu Val Val Asn
               20                  25                  30
   atg agt caa gaa gaa gaa gat ttg gtc tct cga atg cat aag ctt gtc       144
   Met Ser Gln Glu Glu Glu Asp Leu Val Ser Arg Met His Lys Leu Val
           35                  40                  45
   ggt gac agg tgg gaa ctg ata gct ggg agg atc cca gga aga acc gct       192
   Gly Asp Arg Trp Glu Leu Ile Ala Gly Arg Ile Pro Gly Arg Thr Ala
       50                  55                  60
   gga gaa att gag agg ttt tgg gtc atg aaa aat tga                       228
   Gly Glu Ile Glu Arg Phe Trp Val Met Lys Asn
   65                  70                  75
<210> 28
   <211> 75
   <212> prt
   <213> Arabidopsis thaliana
<400> 28
   Met Asp Asn His Arg Arg Thr Lys Gln Pro Lys Thr Asn Ser Ile Val
   1               5                   10                  15
   Thr Ser Ser Ser Glu Glu Val Ser Ser Leu Glu Trp Glu Val Val Asn
               20                  25                  30
   Met Ser Gln Glu Glu Glu Asp Leu Val Ser Arg Met His Lys Leu Val
           35                  40                  45
   Gly Asp Arg Trp Glu Leu Ile Ala Gly Arg Ile Pro Gly Arg Thr Ala
       50                  55                  60
   Gly Glu Ile Glu Arg Phe Trp Val Met Lys Asn
   65                  70                  75
<210> 29
   <211> 480
   <212> DNA
   <213> Arabidopsis thaliana
<220>
   <221> CDS
   <222> (1)..(480)
   <223> G911
<400> 29
   atg ggt ctt cct gaa gat ttc atc acc gag ctt cag att cca ggt tac        48
   Met Gly Leu Pro Glu Asp Phe Ile Thr Glu Leu Gln Ile Pro Gly Tyr
   1               5                   10                  15
   ata tta aag ata ctt tac gtc atc ggt ttc ttt aga gac atg gtc gat        96
   Ile Leu Lys Ile Leu Tyr Val Ile Gly Phe Phe Arg Asp Met Val Asp
               20                  25                  30
   gct ctt tgt cct tac att ggt cta cct agt ttt cta gac cac aac gag       144
   Ala Leu Cys Pro Tyr Ile Gly Leu Pro Ser Phe Leu Asp His Asn Glu
           35                  40                  45
   acc tct gga ccc gat ccg acc cga cac gct ctc tct acg tca gcg agt       192
   Thr Ser Gly Pro Asp Pro Thr Arg His Ala Leu Ser Thr Ser Ala Ser
       50                  55                  60
   ctt gct aac gag ttg atc ccg gtg gtt cgg ttc tcg gat ctt ccg acc       240
   Leu Ala Asn Glu Leu Ile Pro Val Val Arg Phe Ser Asp Leu Pro Thr
   65                  70                  75                  80
   gat ccg gaa gat tgt tgt acg gtt tgt ttg tca gat ttt gag tcc gac       288
   Asp Pro Glu Asp Cys Cys Thr Val Cys Leu Ser Asp Phe Glu Ser Asp
                   85                  90                  95
   gat aag gtt agg cag cta ccc aag tgt gga cac gtg ttt cat cat cat       336
   Asp Lys Val Arg Gln Leu Pro Lys Cys Gly His Val Phe His His His
               100                 105                 110
   tgt tta gac cgt tgg atc gtt gac tac aac aag atg aaa tgt ccg gtt       384
   Cys Leu Asp Arg Trp Ile Val Asp Tyr Asn Lys Met Lys Cys Pro Val
           115                 120                 125
   tgt cgg cac cgg ttc tta ccg aaa gaa aag tac acg caa tgt gat tgg       432
   Cys Arg His Arg Phe Leu Pro Lys Glu Lys Tyr Thr Gln Cys Asp Trp
       130                 135                 140
   ggt tct ggt tca gat tgg ttt agt gat gaa gtg gaa agt acc aac taa       480
   Gly Ser Gly Ser Asp Trp Phe Ser Asp Glu Val Glu Ser Thr Asn
   145                 150                 155
<210> 30
   <211> 159
   <212> prt
   <213> Arabidopsis thaliana
<400> 30
   Met Gly Leu Pro Glu Asp Phe Ile Thr Glu Leu Gln Ile Pro Gly Tyr
   1               5                   10                  15
   Ile Leu Lys Ile Leu Tyr Val Ile Gly Phe Phe Arg Asp Met Val Asp
               20                  25                  30
   Ala Leu Cys Pro Tyr Ile Gly Leu Pro Ser Phe Leu Asp His Asn Glu
           35                  40                  45
   Thr Ser Gly Pro Asp Pro Thr Arg His Ala Leu Ser Thr Ser Ala Ser
       50                  55                  60
   Leu Ala Asn Glu Leu Ile Pro Val Val Arg Phe Ser Asp Leu Pro Thr
   65                  70                  75                  80
   Asp Pro Glu Asp Cys Cys Thr Val Cys Leu Ser Asp Phe Glu Ser Asp
                   85                  90                  95
   Asp Lys Val Arg Gln Leu Pro Lys Cys Gly His Val Phe His His His
               100                 105                 110
   Cys Leu Asp Arg Trp Ile Val Asp Tyr Asn Lys Met Lys Cys Pro Val
           115                 120                 125
   Cys Arg His Arg Phe Leu Pro Lys Glu Lys Tyr Thr Gln Cys Asp Trp
       130                 135                 140
   Gly Ser Gly Ser Asp Trp Phe Ser Asp Glu Val Glu Ser Thr Asn
   145                 150                 155
<210> 31
   <211> 1221
   <212> DNA
   <213> Arabidopsis thaliana
<220>
   <221> CDS
   <222> (162)..(1013)
   <223> G964
<400> 31
   atttctcttc cacaaagagt cctaacttcg agttgaaaca aacaccattt ctcatctcta     60
   tctcagaaag aacaaaccat ttcgtgttct ttctttctct attctcataa ggaaatataa    120
   ttcctgaaac tgttgagttc ttgtgaaagg aaataaaaaa c atg atg atg ggc aaa    176
                                                 Met Met Met Gly Lys
                                                 1               5
   gaa gat cta ggt ttg agc cta agc tta ggg ttt tca caa aat cac aat      224
   Glu Asp Leu Gly Leu Ser Leu Ser Leu Gly Phe Ser Gln Asn His Asn
               10                  15                  20
   cct ctt cag atg aat ctg aat cct aac tct tca tta tca aac aat ctc      272
   Pro Leu Gln Met Asn Leu Asn Pro Asn Ser Ser Leu Ser Asn Asn Leu
           25                  30                  35
   cag aga ctc cca tgg aac caa aca ttc gat cct aca tca gat ctt cgc      320
   Gln Arg Leu Pro Trp Asn Gln Thr Phe Asp Pro Thr Ser Asp Leu Arg
       40                  45                  50
   aag ata gac gtg aac agt ttt cca tca acg gtt aac tgc gag gaa gac      368
   Lys Ile Asp Val Asn Ser Phe Pro Ser Thr Val Asn Cys Glu Glu Asp
       55                  60                  65
   aca gga gtt tcg tca cca aac agt acg atc tca agc acc att agc ggg      416
   Thr Gly Val Ser Ser Pro Asn Ser Thr Ile Ser Ser Thr Ile Ser Gly
   70                  75                  80                  85
   aag aga agt gag aga gaa gga atc tcc gga acc ggc gtt ggc tcc ggc      464
   Lys Arg Ser Glu Arg Glu Gly Ile Ser Gly Thr Gly Val Gly Ser Gly
                   90                  95                  100
   gac gat cac gac gag atc act ccg gat cga ggg tac tca cgt gga acc      512
   Asp Asp His Asp Glu Ile Thr Pro Asp Arg Gly Tyr Ser Arg Gly Thr
               105                 110                 115
   tca gat gaa gaa gaa gac ggg ggc gaa acg tcg agg aag aag ctc agg      560
   Ser Asp Glu Glu Glu Asp Gly Gly Glu Thr Ser Arg Lys Lys Leu Arg
           120                 125                 130
   tta tca aaa gat cag tct gct ttt ctc gaa gag act ttc aaa gaa cac      608
   Leu Ser Lys Asp Gln Ser Ala Phe Leu Glu Glu Thr Phe Lys Glu His
       135                 140                 145
   aac act ctc aat ccc aaa cag aag cta gct ttg gct aag aag ctg aac      656
   Asn Thr Leu Asn Pro Lys Gln Lys Leu Ala Leu Ala Lys Lys Leu Asn
   150                 155                 160                 165
   ttg acg gca aga caa gtg gaa gtg tgg ttc caa aac aga aga gct aga      704
   Leu Thr Ala Arg Gln Val Glu Val Trp Phe Gln Asn Arg Arg Ala Arg
                   170                 175                 180
   acc aag tta aag caa acg gag gta gat tgc gaa tac ttg aaa cgg tgc      752
   Thr Lys Leu Lys Gln Thr Glu Val Asp Cys Glu Tyr Leu Lys Arg Cys
               185                 190                 195
   gta gag aag cta acg gaa gag aac cgg aga ctt cag aaa gag gct atg      800
   Val Glu Lys Leu Thr Glu Glu Asn Arg Arg Leu Gln Lys Glu Ala Met
           200                 205                 210
   gag ctt cga act ctc aag ctg tct cca caa ttc tac ggt cag atg act      848
   Glu Leu Arg Thr Leu Lys Leu Ser Pro Gln Phe Tyr Gly Gln Met Thr
       215                 220                 225
   cca cca act aca ctc atc atg tgt cct tcg tgc gag cgt gta gct ggt      896
   Pro Pro Thr Thr Leu Ile Met Cys Pro Ser Cys Glu Arg Val Ala Gly
   230                 235                 240                 245
   cca tca tca tcg aac cat cac cac aat cac agg ccg gtt tcg att aac      944
   Pro Ser Ser Ser Asn His His His Asn His Arg Pro Val Ser Ile Asn
                   250                 255                 260
   ccg tgg att gct tgt gct ggt cag gtg gct cat ggg ctg aat ttt gaa      992
   Pro Trp Ile Ala Cys Ala Gly Gln Val Ala His Gly Leu Asn Phe Glu
               265                 270                 275
   gcc ttg cgt cca cga tcg taa tttttagtgg tgggggaagg gtgttttggg        1043
   Ala Leu Arg Pro Arg Ser
           280
   ttttttcatt atcgttatat agtctatctg tgtggggtca ttgtaatttt ggatgattgg   1103
   ccttctcatg aactagtcat atgtatgatg caaccttaaa aatatttcaa gtagcaaaac   1163
   ttaattacaa acttgctata ttaaccaaaa attatgaaaa aaaaaaaaaa aaaaaaaa     1221
<210> 32
   <211> 283
   <212> PRT
   <213> Arabidopsis thaliana
<400> 32
   Met Met Met Gly Lys Glu Asp Leu Gly Leu Ser Leu Ser Leu Gly Phe
   1               5                   10                  15
   Ser Gln Asn His Asn Pro Leu Gln Met Asn Leu Asn Pro Asn Ser Ser
               20                  25                  30
   Leu Ser Asn Asn Leu Gln Arg Leu Pro Trp Asn Gln Thr Phe Asp Pro
           35                  40                  45
   Thr Ser Asp Leu Arg Lys Ile Asp Val Asn Ser Phe Pro Ser Thr Val
       50                  55                  60
   Asn Cys Glu Glu Asp Thr Gly Val Ser Ser Pro Asn Ser Thr Ile Ser
   65                  70                  75                  80
   Ser Thr Ile Ser Gly Lys Arg Ser Glu Arg Glu Gly Ile Ser Gly Thr
                   85                  90                  95
   Gly Val Gly Ser Gly Asp Asp His Asp Glu Ile Thr Pro Asp Arg Gly
               100                 105                 110
   Tyr Ser Arg Gly Thr Ser Asp Glu Glu Glu Asp Gly Gly Glu Thr Ser
           115                 120                 125
   Arg Lys Lys Leu Arg Leu Ser Lys Asp Gln Ser Ala Phe Leu Glu Glu
       130                 135                 140
   Thr Phe Lys Glu His Asn Thr Leu Asn Pro Lys Gln Lys Leu Ala Leu
   145                 150                 155                 160
   Ala Lys Lys Leu Asn Leu Thr Ala Arg Gln Val Glu Val Trp Phe Gln
                   165                 170                 175
   Asn Arg Arg Ala Arg Thr Lys Leu Lys Gln Thr Glu Val Asp Cys Glu
               180                 185                 190
   Tyr Leu Lys Arg Cys Val Glu Lys Leu Thr Glu Glu Asn Arg Arg Leu
           195                 200                 205
   Gln Lys Glu Ala Met Glu Leu Arg Thr Leu Lys Leu Ser Pro Gln Phe
       210                 215                 220
   Tyr Gly Gln Met Thr Pro Pro Thr Thr Leu Ile Met Cys Pro Ser Cys
   225                 230                 235                 240
   Glu Arg Val Ala Gly Pro Ser Ser Ser Asn His His His Asn His Arg
                   245                 250                 255
   Pro Val Ser Ile Asn Pro Trp Ile Ala Cys Ala Gly Gln Val Ala His
               260                 265                 270
   Gly Leu Asn Phe Glu Ala Leu Arg Pro Arg Ser
           275                 280
<210> 33
   <211> 1249
   <212> DNA
   <213> Arabidopsis thaliana
<220>
   <221> CDS
   <222> (82)..(918)
   <223> G394
<400> 33
   gaaattctta acaaacaatt ttcttcataa tattaattct caagatctta aagattatat      60
   taatacgaag agaaaattca a atg ggt ctt gat gat tca tgc aac aca ggt       111
                           Met Gly Leu Asp Asp Ser Cys Asn Thr Gly
                           1               5                   10
   ctt gtt ctt ggt tta ggc ctc tca cca acg cct aat aat tac aat cat       159
   Leu Val Leu Gly Leu Gly Leu Ser Pro Thr Pro Asn Asn Tyr Asn His
                   15                  20                  25
   gcc atc aag aaa tct tcc tcc act gtg gac cat cgt ttc atc agg ctc       207
   Ala Ile Lys Lys Ser Ser Ser Thr Val Asp His Arg Phe Ile Arg Leu
               30                  35                  40
   gat ccg tcg ttg act cta agc cta tcc ggt gag agc tac aag atc aag       255
   Asp Pro Ser Leu Thr Leu Ser Leu Ser Gly Glu Ser Tyr Lys Ile Lys
           45                  50                  55
   act ggt gcc ggc gcc ggc gac caa att tgc cgg cag acc tcg tcc cac       303
   Thr Gly Ala Gly Ala Gly Asp Gln Ile Cys Arg Gln Thr Ser Ser His
       60                  65                  70
   agc ggc atc tca tct ttc tcg agc gga agg gta aag aga gaa aga gaa       351
   Ser Gly Ile Ser Ser Phe Ser Ser Gly Arg Val Lys Arg Glu Arg Glu
   75                  80                  85                  90
   atc tcc ggc ggc gat gga gaa gaa gag gcg gag gag acg acg gag aga       399
   Ile Ser Gly Gly Asp Gly Glu Glu Glu Ala Glu Glu Thr Thr Glu Arg
                   95                  100                 105
   gtg gtg tgt tcg aga gtg agt gat gat cat gac gat gaa gaa ggt gtt       447
   Val Val Cys Ser Arg Val Ser Asp Asp His Asp Asp Glu Glu Gly Val
               110                 115                 120
   agt gct cgt aaa aag ctt aga ctc act aaa caa caa tct gct ctt ctc       495
   Ser Ala Arg Lys Lys Leu Arg Leu Thr Lys Gln Gln Ser Ala Leu Leu
           125                 130                 135
   gaa gat aac ttc aaa ctt cat agc acc ctt aat ccc aag caa aaa caa       543
   Glu Asp Asn Phe Lys Leu His Ser Thr Leu Asn Pro Lys Gln Lys Gln
       140                 145                 150
   gct ctt gcg aga cag ctg aat cta agg cct aga caa gtt gaa gtg tgg       591
   Ala Leu Ala Arg Gln Leu Asn Leu Arg Pro Arg Gln Val Glu Val Trp
   155                 160                 165                 170
   ttc caa aac agg aga gct aga aca aaa cta aag caa aca gaa gtg gat      639
   Phe Gln Asn Arg Arg Ala Arg Thr Lys Leu Lys Gln Thr Glu Val Asp
                   175                 180                 185
   tgt gag ttt ttg aag aaa tgt tgc gag act tta acg gat gag aat aga      687
   Cys Glu Phe Leu Lys Lys Cys Cys Glu Thr Leu Thr Asp Glu Asn Arg
               190                 195                 200
   agg ctt caa aaa gag ctt caa gac ctt aag gct tta aaa ttg tct caa      735
   Arg Leu Gln Lys Glu Leu Gln Asp Leu Lys Ala Leu Lys Leu Ser Gln
           205                 210                 215
   ccg ttt tac atg cac atg ccg gcg gcg act ttg act atg tgc cct tct      783
   Pro Phe Tyr Met His Met Pro Ala Ala Thr Leu Thr Met Cys Pro Ser
       220                 225                 230
   tgt gag aga ctc ggc ggt ggt ggt gtc gga gga gat acg acg gcg gtt      831
   Cys Glu Arg Leu Gly Gly Gly Gly Val Gly Gly Asp Thr Thr Ala Val
   235                 240                 245                 250
   gat gaa gaa acg gcg aaa gga gct ttc tcc atc gtc aca aag cct cgt      879
   Asp Glu Glu Thr Ala Lys Gly Ala Phe Ser Ile Val Thr Lys Pro Arg
                   255                 260                 265
   ttc tat aac cct ttc act aat cct tct gca gca tgt tag ttacttatta      928
   Phe Tyr Asn Pro Phe Thr Asn Pro Ser Ala Ala Cys
               270                 275
   gttatttaat tctttttgtt ggtttttttt ttgtttctta aatcaaatta ggaattagtt    988
   agaagataaa tcccagggaa aaaatattac gttgaaattg gggggaaatg gggtatagtc   1048
   tttatagata agactcttca acgattccac tttatttttc ggtgggattg ttggttgatg   1108
   aagaaaaaaa aatagtttgt aattacaggt ttaaatatgt agagaaaaaa tgacgaatat   1168
   gtattatctt gttttttttt ccttcgaata tgtattacgg taatataaat ttgcttgtaa   1228
   aaataataaa tatattattt g                                             1249
<210> 34
   <211> 278
   <212> PRT
   <213> Arabidopsis thaliana
<400> 34
   Met Gly Leu Asp Asp Ser Cys Asn Thr Gly Leu Val Leu Gly Leu Gly
   1               5                   10                  15
   Leu Ser Pro Thr Pro Asn Asn Tyr Asn His Ala Ile Lys Lys Ser Ser
               20                  25                  30
   Ser Thr Val Asp His Arg Phe Ile Arg Leu Asp Pro Ser Leu Thr Leu
           35                  40                  45
   Ser Leu Ser Gly Glu Ser Tyr Lys Ile Lys Thr Gly Ala Gly Ala Gly
       50                  55                  60
   Asp Gln Ile Cys Arg Gln Thr Ser Ser His Ser Gly Ile Ser Ser Phe
   65                  70                  75                  80
   Ser Ser Gly Arg Val Lys Arg Glu Arg Glu Ile Ser Gly Gly Asp Gly
                   85                  90                  95
   Glu Glu Glu Ala Glu Glu Thr Thr Glu Arg Val Val Cys Ser Arg Val
               100                 105                 110
   Ser Asp Asp His Asp Asp Glu Glu Gly Val Ser Ala Arg Lys Lys Leu
           115                 120                 125
   Arg Leu Thr Lys Gln Gln Ser Ala Leu Leu Glu Asp Asn Phe Lys Leu
       130                 135                 140
   His Ser Thr Leu Asn Pro Lys Gln Lys Gln Ala Leu Ala Arg Gln Leu
   145                 150                 155                 160
   Asn Leu Arg Pro Arg Gln Val Glu Val Trp Phe Gln Asn Arg Arg Ala
                   165                 170                 175
   Arg Thr Lys Leu Lys Gln Thr Glu Val Asp Cys Glu Phe Leu Lys Lys
               180                 185                 190
   Cys Cys Glu Thr Leu Thr Asp Glu Asn Arg Arg Leu Gln Lys Glu Leu
           195                 200                 205
   Gln Asp Leu Lys Ala Leu Lys Leu Ser Gln Pro Phe Tyr Met His Met
       210                 215                 220
   Pro Ala Ala Thr Leu Thr Met Cys Pro Ser Cys Glu Arg Leu Gly Gly
   225                 230                 235                 240
   Gly Gly Val Gly Gly Asp Thr Thr Ala Val Asp Glu Glu Thr Ala Lys
                   245                 250                 255
   Gly Ala Phe Ser Ile Val Thr Lys Pro Arg Phe Tyr Asn Pro Phe Thr
               260                 265                 270
   Asn Pro Ser Ala Ala Cys
           275
<210> 35
   <211> 1147
   <212> DNA
   <213> Arabidopsis thaliana
<220>
   <221> CDS
   <222> (33)..(695)
   <223> G489
<400> 35
   tggatcaaca agaccatgga cagtctggag ct atg aac tat ggc aca aac cca        53
                                       Met Asn Tyr Gly Thr Asn Pro
                                       1               5
   tac caa acc aac ccg atg agc acc act gct gct act gta gca gga ggt       101
   Tyr Gln Thr Asn Pro Met Ser Thr Thr Ala Ala Thr Val Ala Gly Gly
           10                  15                  20
   gcg gca caa cca ggc cag ctg gcg ttc cac cag atc cat cag cag cag       149
   Ala Ala Gln Pro Gly Gln Leu Ala Phe His Gln Ile His Gln Gln Gln
       25                  30                  35
   cag cag caa cag ctg gca cag cag ctt caa gca ttt tgg gag aac caa       197
   Gln Gln Gln Gln Leu Ala Gln Gln Leu Gln Ala Phe Trp Glu Asn Gln
   40                  45                  50                  55
   ttc aaa gag att gag aag act acc gat ttc aag aac cac agc ctt ccc       245
   Phe Lys Glu Ile Glu Lys Thr Thr Asp Phe Lys Asn His Ser Leu Pro
                   60                  65                  70
   ctt gcg aga atc aag aaa atc atg aaa gcg gat gaa gat gtc cgt atg       293
   Leu Ala Arg Ile Lys Lys Ile Met Lys Ala Asp Glu Asp Val Arg Met
               75                  80                  85
   atc tcg gct gag gcg ccg gtc gtg ttt gca agg gcc tgt gag atg ttc       341
   Ile Ser Ala Glu Ala Pro Val Val Phe Ala Arg Ala Cys Glu Met Phe
           90                  95                  100
   atc ctg gag ctg aca ctc agg tcg tgg aac cac act gag gag aat aag       389
   Ile Leu Glu Leu Thr Leu Arg Ser Trp Asn His Thr Glu Glu Asn Lys
       105                 110                 115
   agg cgg acg ttg cag aag aac gat att gct gct gct gtg act aga acc       437
   Arg Arg Thr Leu Gln Lys Asn Asp Ile Ala Ala Ala Val Thr Arg Thr
   120                 125                 130                 135
   gat att ttt gat ttc ctt gtg gat att gtt ccc cgg gag gat ctc cga       485
   Asp Ile Phe Asp Phe Leu Val Asp Ile Val Pro Arg Glu Asp Leu Arg
                   140                 145                 150
   gat gaa gtc ttg gga agt att ccg agg ggc act gtc ccg gaa gct gct       533
   Asp Glu Val Leu Gly Ser Ile Pro Arg Gly Thr Val Pro Glu Ala Ala
               155                 160                 165
   gct gct ggt tac ccg tat gga tac ttg cct gca gga act gct cca ata       581
   Ala Ala Gly Tyr Pro Tyr Gly Tyr Leu Pro Ala Gly Thr Ala Pro Ile
           170                 175                 180
   gga aat ccg gga atg gtt atg ggt aat ccc ggt ggt gcg tat cca cct       629
   Gly Asn Pro Gly Met Val Met Gly Asn Pro Gly Gly Ala Tyr Pro Pro
       185                 190                 195
   aat cct tat atg ggt caa cca atg tgg caa caa cag gca cct gac caa       677
   Asn Pro Tyr Met Gly Gln Pro Met Trp Gln Gln Gln Ala Pro Asp Gln
   200                 205                 210                 215
   cct gac cag gaa aat tag caagaaactg tgagtcttcc agcttcgcgg              725
   Pro Asp Gln Glu Asn
                   220
   ccgctctaga caggcctcgt accggatcct ctagctagag ctttcgttcg tatcatcggt     785
   ttcgacaacg ttcgtcaagt tcaatgcatc agtttcattg cgcacacacc agaatcctac     845
   tgagtttgag tattatggca ttgggaaaac tgtttttctt gtccatttgt tgtgcttgta     905
   atttactgtg ttttttattc ggttttcgct atcgaactgt gaaatggaaa tggatggaga     965
   agagttaatg aatgatatgg ccttttgttc attctcaaat taatattatt tggtttttct    1025
   cttatttgtg gggatgaatt tgaaattata agagatatgc aaacattttg tttgagtaaa    1085
   atgtgcaaat cgtggcctct aatgacccga agttaatatg aggagtaaaa cacttgtagg    1145
   tg                                                                   1147
<210> 36
   <211> 220
   <212> PRT
   <213> Arabidopsis thaliana
<400> 36
   Met Asn Tyr Gly Thr Asn Pro Tyr Gln Thr Asn Pro Met Ser Thr Thr
   1               5                   10                  15
   Ala Ala Thr Val Ala Gly Gly Ala Ala Gln Pro Gly Gln Leu Ala Phe
               20                  25                  30
   His Gln Ile His Gln Gln Gln Gln Gln Gln Gln Leu Ala Gln Gln Leu
           35                  40                  45
   Gln Ala Phe Trp Glu Asn Gln Phe Lys Glu Ile Glu Lys Thr Thr Asp
       50                  55                  60
   Phe Lys Asn His Ser Leu Pro Leu Ala Arg Ile Lys Lys Ile Met Lys
   65                  70                  75                  80
   Ala Asp Glu Asp Val Arg Met Ile Ser Ala Glu Ala Pro Val Val Phe
                   85                  90                  95
   Ala Arg Ala Cys Glu Met Phe Ile Leu Glu Leu Thr Leu Arg Ser Trp
               100                 105                 110
   Asn His Thr Glu Glu Asn Lys Arg Arg Thr Leu Gln Lys Asn Asp Ile
           115                 120                 125
   Ala Ala Ala Val Thr Arg Thr Asp Ile Phe Asp Phe Leu Val Asp Ile
       130                 135                 140
   Val Pro Arg Glu Asp Leu Arg Asp Glu Val Leu Gly Ser Ile Pro Arg
   145                 150                 155                 160
   Gly Thr Val Pro Glu Ala Ala Ala Ala Gly Tyr Pro Tyr Gly Tyr Leu
                   165                 170                 175
   Pro Ala Gly Thr Ala Pro Ile Gly Asn Pro Gly Met Val Met Gly Asn
               180                 185                 190
   Pro Gly Gly Ala Tyr Pro Pro Asn Pro Tyr Met Gly Gln Pro Met Trp
           195                 200                 205
   Gln Gln Gln Ala Pro Asp Gln Pro Asp Gln Glu Asn
       210                 215                 220
<210> 37
   <211> 1262
   <212> DNA
   <213> Arabidopsis thaliana
<220>
   <221> CDS
   <222> (217)..(957)
   <223> G463
<400> 37
   ctcgagctac gtcaggggtc tctttctgtt tgtttgtttt cttgtttcct tctctctctc      60
   tctttctttc tttgtcttcc tttcccaggt tgtttttttt tgctctctct gccttcttga     120
   ctttcaaaag actctttctt tcttttggat tgattttgga ttctagggct ctctttcttt     180
   tagtgggttt ttgttgttgt tgttgtggtc tctctg atg att act gaa ctt gag       234
                                           Met Ile Thr Glu Leu Glu
                                           1               5
   atg ggg aaa ggt gag agt gag ctt gag ctt ggt cta ggg ctg agt ctt       282
   Met Gly Lys Gly Glu Ser Glu Leu Glu Leu Gly Leu Gly Leu Ser Leu
           10                  15                  20
   ggc ggt gga acg gcg gcc aag att ggt aaa tca ggt ggt ggt ggc gcg      330
   Gly Gly Gly Thr Ala Ala Lys Ile Gly Lys Ser Gly Gly Gly Gly Ala
           25                  30                  35
   tgg gga gag cgt gga agg ctt ttg acg gct aag gat ttt cct tct gtt      378
   Trp Gly Glu Arg Gly Arg Leu Leu Thr Ala Lys Asp Phe Pro Ser Val
       40                  45                  50
   ggt tct aaa cgt gct gct gat tct gct tct cat gct ggt tca tct cct      426
   Gly Ser Lys Arg Ala Ala Asp Ser Ala Ser His Ala Gly Ser Ser Pro
   55                  60                  65                  70
   cct cgt tca agt caa gtt gtt gga tgg cct cct ata ggg tca cac agg      474
   Pro Arg Ser Ser Gln Val Val Gly Trp Pro Pro Ile Gly Ser His Arg
                   75                  80                  85
   atg aac agt ttg gtt aat aac caa gct aca aag tca gca aga gaa gaa      522
   Met Asn Ser Leu Val Asn Asn Gln Ala Thr Lys Ser Ala Arg Glu Glu
               90                  95                  100
   gaa gaa gct ggt aag aag aaa gtg aaa gat gat gaa cct aaa gat gtg      570
   Glu Glu Ala Gly Lys Lys Lys Val Lys Asp Asp Glu Pro Lys Asp Val
           105                 110                 115
   aca aag aaa gtg aat ggg aaa gta caa gtt gga ttt att aag gtg aac      618
   Thr Lys Lys Val Asn Gly Lys Val Gln Val Gly Phe Ile Lys Val Asn
       120                 125                 130
   atg gat gga gtt gct ata gga aga aaa gtg gat ttg aat gct cat tct      666
   Met Asp Gly Val Ala Ile Gly Arg Lys Val Asp Leu Asn Ala His Ser
   135                 140                 145                 150
   tct tac gag aat ttg gcg caa aca ttg gaa gat atg ttc ttt cgc act      714
   Ser Tyr Glu Asn Leu Ala Gln Thr Leu Glu Asp Met Phe Phe Arg Thr
                   155                 160                 165
   aat ccg ggt act gtc ggg tta acc agt cag ttc act aaa ccg ttg agg      762
   Asn Pro Gly Thr Val Gly Leu Thr Ser Gln Phe Thr Lys Pro Leu Arg
               170                 175                 180
   ctt tta gat gga tcg tct gag ttt gta ctt act tat gaa gat aag gaa      810
   Leu Leu Asp Gly Ser Ser Glu Phe Val Leu Thr Tyr Glu Asp Lys Glu
           185                 190                 195
   gga gat tgg atg ctt gtt ggt gat gtt cca tgg aga atg ttc atc aac      858
   Gly Asp Trp Met Leu Val Gly Asp Val Pro Trp Arg Met Phe Ile Asn
       200                 205                 210
   tcg gtg aaa agg cta cgt gtg atg aaa acc tct gaa gct aat gga ctc      906
   Ser Val Lys Arg Leu Arg Val Met Lys Thr Ser Glu Ala Asn Gly Leu
   215                 220                 225                 230
   gct gca cga aat caa gaa cca aac gag aga cag cga aag cag ccg gtt      954
   Ala Ala Arg Asn Gln Glu Pro Asn Glu Arg Gln Arg Lys Gln Pro Val
                   235                 240                 245
   tag atctcttttc gacgttacgg tgttacaggt tttatatttt ggggttttgc          1007
   aagtctgaga tacttctgaa gcaagcataa gctagattga tcttatatcc agtttgtgta   1067
   ttttcttggt tcttataatg gtttttactg gttttcttta gttttttttt ttgctgtctt   1127
   ttaattttcg gttgcgattt cactatatac tatggatgga agagaatgct ctttatatct   1187
   tttactacac tgtaaatatt tgaagcttat ctaatatcgt ttttaagggt taaaaaaccc   1247
   tgacgtagcc tcgag                                                    1262
<210> 38
   <211> 246
   <212> PRT
   <213> Arabidopsis thaliana
<400> 38
   Met Ile Thr Glu Leu Glu Met Gly Lys Gly Glu Ser Glu Leu Glu Leu
   1               5                   10                  15
   Gly Leu Gly Leu Ser Leu Gly Gly Gly Thr Ala Ala Lys Ile Gly Lys
               20                  25                  30
   Ser Gly Gly Gly Gly Ala Trp Gly Glu Arg Gly Arg Leu Leu Thr Ala
           35                  40                  45
   Lys Asp Phe Pro Ser Val Gly Ser Lys Arg Ala Ala Asp Ser Ala Ser
       50                  55                  60
   His Ala Gly Ser Ser Pro Pro Arg Ser Ser Gln Val Val Gly Trp Pro
   65                  70                  75                  80
   Pro Ile Gly Ser His Arg Met Asn Ser Leu Val Asn Asn Gln Ala Thr
                   85                  90                  95
   Lys Ser Ala Arg Glu Glu Glu Glu Ala Gly Lys Lys Lys Val Lys Asp
               100                 105                 110
   Asp Glu Pro Lys Asp Val Thr Lys Lys Val Asn Gly Lys Val Gln Val
           115                 120                 125
   Gly Phe Ile Lys Val Asn Met Asp Gly Val Ala Ile Gly Arg Lys Val
       130                 135                 140
   Asp Leu Asn Ala His Ser Ser Tyr Glu Asn Leu Ala Gln Thr Leu Glu
   145                 150                 155                 160
   Asp Met Phe Phe Arg Thr Asn Pro Gly Thr Val Gly Leu Thr Ser Gln
                   165                 170                 175
   Phe Thr Lys Pro Leu Arg Leu Leu Asp Gly Ser Ser Glu Phe Val Leu
               180                 185                 190
   Thr Tyr Glu Asp Lys Glu Gly Asp Trp Met Leu Val Gly Asp Val Pro
           195                 200                 205
   Trp Arg Met Phe Ile Asn Ser Val Lys Arg Leu Arg Val Met Lys Thr
       210                 215                 220
   Ser Glu Ala Asn Gly Leu Ala Ala Arg Asn Gln Glu Pro Asn Glu Arg
   225                 230                 235                 240
   Gln Arg Lys Gln Pro Val
                   245
<210> 39
   <211> 905
   <212> DNA
   <213> Arabidopsis thaliana
<220>
   <221> CDS
   <222> (76)..(837)
   <223> G767
<400> 39
   cgaagtttca actttcaaac atatctttac agttctttct tgctaaacaa caataaaggg    60
   gaaaataggt taatt atg atg aaa tct ggg gct gat ttg caa ttt cca cca   111
                    Met Met Lys Ser Gly Ala Asp Leu Gln Phe Pro Pro
                    1               5                   10
   gga ttt aga ttt cat cct acg gat gag gag cta gtc ctc atg tat ctc   159
   Gly Phe Arg Phe His Pro Thr Asp Glu Glu Leu Val Leu Met Tyr Leu
           15                  20                  25
   tgt cgt aaa tgc gcg tcg cag ccg atc cct gct ccg att atc acc gaa   207
   Cys Arg Lys Cys Ala Ser Gln Pro Ile Pro Ala Pro Ile Ile Thr Glu
       30                  35                  40
   ctc gat ttg tac cga tat gat cct tgg gac ctt ccc gac atg gct ttg   255
   Leu Asp Leu Tyr Arg Tyr Asp Pro Trp Asp Leu Pro Asp Met Ala Leu
   45                  50                  55                  60
   tac ggt gaa aag gag tgg tat ttt ttc tca cca aga gat cga aag tat   303
   Tyr Gly Glu Lys Glu Trp Tyr Phe Phe Ser Pro Arg Asp Arg Lys Tyr
                   65                  70                  75
   cca aac ggt tca aga ccc aac cgt gca gct ggt act gga tat tgg aaa   351
   Pro Asn Gly Ser Arg Pro Asn Arg Ala Ala Gly Thr Gly Tyr Trp Lys
               80                  85                  90
   gct acc gga gct gat aaa cca ata ggt cgt cct aaa ccg gtt ggt att   399
   Ala Thr Gly Ala Asp Lys Pro Ile Gly Arg Pro Lys Pro Val Gly Ile
           95                  100                 105
   aag aag gct cta gtg ttt tac tcg gga aaa cct cca aat gga gag aaa   447
   Lys Lys Ala Leu Val Phe Tyr Ser Gly Lys Pro Pro Asn Gly Glu Lys
       110                 115                 120
   acc aat tgg att atg cac gaa tac cgg ctc gct gac gtt gac cgg tcg   495
   Thr Asn Trp Ile Met His Glu Tyr Arg Leu Ala Asp Val Asp Arg Ser
   125                 130                 135                 140
   gtt cgt aag aaa aac agt cta aga ttg gac gat tgg gta ttg tgt cgt   543
   Val Arg Lys Lys Asn Ser Leu Arg Leu Asp Asp Trp Val Leu Cys Arg
                   145                 150                 155
   ata tat aac aag aaa ggt gtc atc gag aag cga cga agc gat atc gag   591
   Ile Tyr Asn Lys Lys Gly Val Ile Glu Lys Arg Arg Ser Asp Ile Glu
               160                 165                 170
   gac ggg tta aag cct gtg act gac acg tgt cca ccg gaa tct gtg gcg   639
   Asp Gly Leu Lys Pro Val Thr Asp Thr Cys Pro Pro Glu Ser Val Ala
           175                 180                 185
   aga ttg atc tcc ggc tcg gag caa gcg gtg tca ccg gaa ttc acg tgt   687
   Arg Leu Ile Ser Gly Ser Glu Gln Ala Val Ser Pro Glu Phe Thr Cys
       190                 195                 200
   agc aac ggt cgg ttg agt aat gcc ctt gat ttt ccg ttt aat tac gta   735
   Ser Asn Gly Arg Leu Ser Asn Ala Leu Asp Phe Pro Phe Asn Tyr Val
   205                 210                 215                 220
   gat gcc atc gcc gat aac gag att gtg tca cgg cta ttg ggc ggg aat   783
   Asp Ala Ile Ala Asp Asn Glu Ile Val Ser Arg Leu Leu Gly Gly Asn
                   225                 230                 235
   cag atg tgg tcg acg acg ctt gat cca ctt gtg gtt agg cag gga act   831
   Gln Met Trp Ser Thr Thr Leu Asp Pro Leu Val Val Arg Gln Gly Thr
               240                 245                 250
   ttc tga gttgtcacgt gcgattagag ttagtggaaa gtggaaacta tcactgtctg    887
   Phe
   ttttcgcacg tgtcgggc                                                905
<210> 40
   <211> 253
   <212> PRT
   <213> Arabidopsis thaliana
<400> 40
   Met Met Lys Ser Gly Ala Asp Leu Gln Phe Pro Pro Gly Phe Arg Phe
   1               5                   10                  15
   His Pro Thr Asp Glu Glu Leu Val Leu Met Tyr Leu Cys Arg Lys Cys
               20                  25                  30
   Ala Ser Gln Pro Ile Pro Ala Pro Ile Ile Thr Glu Leu Asp Leu Tyr
           35                  40                  45
   Arg Tyr Asp Pro Trp Asp Leu Pro Asp Met Ala Leu Tyr Gly Glu Lys
       50                  55                  60
   Glu Trp Tyr Phe Phe Ser Pro Arg Asp Arg Lys Tyr Pro Asn Gly Ser
   65                  70                  75                  80
   Arg Pro Asn Arg Ala Ala Gly Thr Gly Tyr Trp Lys Ala Thr Gly Ala
                   85                  90                  95
   Asp Lys Pro Ile Gly Arg Pro Lys Pro Val Gly Ile Lys Lys Ala Leu
               100                 105                 110
   Val Phe Tyr Ser Gly Lys Pro Pro Asn Gly Glu Lys Thr Asn Trp Ile
           115                 120                 125
   Met His Glu Tyr Arg Leu Ala Asp Val Asp Arg Ser Val Arg Lys Lys
       130                 135                 140
   Asn Ser Leu Arg Leu Asp Asp Trp Val Leu Cys Arg Ile Tyr Asn Lys
   145                 150                 155                 160
   Lys Gly Val Ile Glu Lys Arg Arg Ser Asp Ile Glu Asp Gly Leu Lys
                   165                 170                 175
   Pro Val Thr Asp Thr Cys Pro Pro Glu Ser Val Ala Arg Leu Ile Ser
               180                 185                 190
   Gly Ser Glu Gln Ala Val Ser Pro Glu Phe Thr Cys Ser Asn Gly Arg
           195                 200                 205
   Leu Ser Asn Ala Leu Asp Phe Pro Phe Asn Tyr Val Asp Ala Ile Ala
       210                 215                 220
   Asp Asn Glu Ile Val Ser Arg Leu Leu Gly Gly Asn Gln Met Trp Ser
   225                 230                 235                 240
   Thr Thr Leu Asp Pro Leu Val Val Arg Gln Gly Thr Phe
                   245                 250
<210> 41
   <211> 1479
   <212> DNA
   <213> Arabidopsis thaliana
<220>
   <221> CDS
   <222> (192)..(962)
   <223> G765
<400> 41
   tttaactgca tagttcttcc tcaaattgct catacctttg cttctctttc tttctttatt      60
   caacctctta tttcttcttc agcaccaacg tgtatctgca gtaaacacat atccatcgat     120
   cgatctcctc aaaaagttat tgttttcttg aaggattttt cttgttcttg atcaagcata     180
   catatatata g atg gtg gaa gaa ggc ggc gta gtt gtg aat caa gga gga     230
               Met Val Glu Glu Gly Gly Val Val Val Asn Gln Gly Gly
               1               5                   10
   gat cag gag gtg gtg gat ttg cct ccg ggg ttt cgg ttt cac cct act      278
   Asp Gln Glu Val Val Asp Leu Pro Pro Gly Phe Arg Phe His Pro Thr
       15                  20                  25
   gat gaa gag ata ata act cac tac ctc aaa gag aag gtc ttc aac atc      326
   Asp Glu Glu Ile Ile Thr His Tyr Leu Lys Glu Lys Val Phe Asn Ile
   30                  35                  40                  45
   cga ttt acc gcg gca gcg att ggt caa gcc gac ctc aac aag aac gag      374
   Arg Phe Thr Ala Ala Ala Ile Gly Gln Ala Asp Leu Asn Lys Asn Glu
                   50                  55                  60
   cca tgg gat cta cca aag att gca aag atg ggg gag aag gag ttt tac      422
   Pro Trp Asp Leu Pro Lys Ile Ala Lys Met Gly Glu Lys Glu Phe Tyr
               65                  70                  75
   ttt ttc tgc cag agg gat cgg aag tat ccg acc ggg atg agg acg aac      470
   Phe Phe Cys Gln Arg Asp Arg Lys Tyr Pro Thr Gly Met Arg Thr Asn
           80                  85                  90
   cgt gcg acc gtg tct ggt tat tgg aag gcg acc ggg aag gac aag gag      518
   Arg Ala Thr Val Ser Gly Tyr Trp Lys Ala Thr Gly Lys Asp Lys Glu
       95                  100                 105
   atc ttt aga ggc aaa ggt tgt ctt gtt ggg atg aag aaa aca ctt gtt      566
   Ile Phe Arg Gly Lys Gly Cys Leu Val Gly Met Lys Lys Thr Leu Val
   110                 115                 120                 125
   ttc tat aca gga aga gct cct aaa ggt gaa aag acc aat tgg gtt atg      614
   Phe Tyr Thr Gly Arg Ala Pro Lys Gly Glu Lys Thr Asn Trp Val Met
                   130                 135                 140
   cat gaa tat cgt ctt gat gga aaa tat tct tat cat aac ctc ccc aaa      662
   His Glu Tyr Arg Leu Asp Gly Lys Tyr Ser Tyr His Asn Leu Pro Lys
               145                 150                 155
   acc gca agg gat gaa tgg gtg gtg tgt agg gtt ttt cac aag aac gct      710
   Thr Ala Arg Asp Glu Trp Val Val Cys Arg Val Phe His Lys Asn Ala
           160                 165                 170
   cct agt act aca atc act act aca aaa caa ctc tca agg att gat tct      758
   Pro Ser Thr Thr Ile Thr Thr Thr Lys Gln Leu Ser Arg Ile Asp Ser
       175                 180                 185
   ctt gat aac att gat cat ctc tta gac ttc tca tct ctc cct cct ctc      806
   Leu Asp Asn Ile Asp His Leu Leu Asp Phe Ser Ser Leu Pro Pro Leu
   190                 195                 200                 205
   ata gat ccg ggt ttc ttg ggt caa ccc gcc caa gct tct ccg gtg ccc      854
   Ile Asp Pro Gly Phe Leu Gly Gln Pro Ala Gln Ala Ser Pro Val Pro
                   210                 215                 220
   gtc aac aac acg atc tca aac ctg tct cca cca tcc tac aac cgc acc      902
   Val Asn Asn Thr Ile Ser Asn Leu Ser Pro Pro Ser Tyr Asn Arg Thr
               225                 230                 235
   agt cga caa cac tta cct tcc tac cca agc tct caa ttt ccc tta cca      950
   Ser Arg Gln His Leu Pro Ser Tyr Pro Ser Ser Gln Phe Pro Leu Pro
        240                245                 250
   ctc ggt ccc taa ttccggatct gatttcggct acggggcagg ttcaggcaat         1002
   Leu Gly Pro
       255
   aataacaaag gtatgatcaa gttggagcat tctcttgtga gtgtgtctca agaaaccggt   1062
   ttgagttccg atgtgaacac aaccgcaacg ccagagatat cttcttatcc aatgatgatg   1122
   aatccggcaa tgatggatgg tagcaagtca gcgtgtgatg gtcttgatga cttgatcttc   1182
   tgggaagatt tatatactag ctaaatttgg gaaaaggtta tttgttaatt gtgattgaag   1242
   agtggcatat tgattactcg tctagtgttt ttaatcgtgt aattagttcg tatataatat   1302
   acatgtacat aagatcatta ggtttattag gcattggact ttagttcggt gattgcttac   1362
   ctagttttta gcttgagaaa aaaggctgtc attggggtta tgtttctttg tgattaactt   1422
   gtacatatat acatttaaat taaacgtatg gtttaaatcg tttaaaaaaa aaaaaaa      1479
<210> 42
   <211> 256
   <212> PRT
   <213> Arabidopsis thaliana
<400> 42
   Met Val Glu Glu Gly Gly Val Val Val Asn Gln Gly Gly Asp Gln Glu
   1 5 10 15
   Val Val Asp Leu Pro Pro Gly Phe Arg Phe His Pro Thr Asp Glu Glu
   20 25 30
   Ile Ile Thr His Tyr Leu Lys Glu Lys Val Phe Asn Ile Arg Phe Thr
   35 40 45
   Ala Ala Ala Ile Gly Gln Ala Asp Leu Asn Lys Asn Glu Pro Trp Asp
   50 55 60
   Leu Pro Lys Ile Ala Lys Met Gly Glu Lys Glu Phe Tyr Phe Phe Cys
   65 70 75 80
   Gln Arg Asp Arg Lys Tyr Pro Thr Gly Met Arg Thr Asn Arg Ala Thr
   85 90 95
   Val Ser Gly Tyr Trp Lys Ala Thr Gly Lys Asp Lys Glu Ile Phe Arg
   100 105 110
   Gly Lys Gly Cys Leu Val Gly Met Lys Lys Thr Leu Val Phe Tyr Thr
   115 120 125
   Gly Arg Ala Pro Lys Gly Glu Lys Thr Asn Trp Val Met His Glu Tyr
   130 135 140
   Arg Leu Asp Gly Lys Tyr Ser Tyr His Asn Leu Pro Lys Thr Ala Arg
   145 150 155 160
   Asp Glu Trp Val Val Cys Arg Val Phe His Lys Asn Ala Pro Ser Thr
   165 170 175
   Thr Ile Thr Thr Thr Lys Gln Leu Ser Arg Ile Asp Ser Leu Asp Asn
   180 185 190
   Ile Asp His Leu Leu Asp Phe Ser Ser Leu Pro Pro Leu Ile Asp Pro
   195 200 205
   Gly Phe Leu Gly Gln Pro Ala Gln Ala Ser Pro Val Pro Val Asn Asn
   210 215 220
   Thr Ile Ser Asn Leu Ser Pro Pro Ser Tyr Asn Arg Thr Ser Arg Gln
   225 230 235 240
   His Leu Pro Ser Tyr Pro Ser Ser Gln Phe Pro Leu Pro Leu Gly Pro
   245 250 255
<210> 43
   <211> 825
   <212> DNA
   <213> Arabidopsis thaliana
<220>
   <221> CDS
   <222> (1)..(825)
   <223> G197
<400> 43
   atg gga agg tct cct tgc tgt gag aaa gac cac aca aac aaa gga gct      48
   Met Gly Arg Ser Pro Cys Cys Glu Lys Asp His Thr Asn Lys Gly Ala
   1               5                   10                  15
   tgg act aag gaa gaa gac gat aag ctc atc tct tac atc aaa gct cac      96
   Trp Thr Lys Glu Glu Asp Asp Lys Leu Ile Ser Tyr Ile Lys Ala His
               20                  25                  30
   ggt gaa ggt tgt tgg cgt tct ctt cct aga tcc gcc ggt ctt caa cgt     144
   Gly Glu Gly Cys Trp Arg Ser Leu Pro Arg Ser Ala Gly Leu Gln Arg
           35                  40                  45
   tgc gga aaa agc tgt cgt ctc cga tgg att aac tat ctc cga cct gat     192
   Cys Gly Lys Ser Cys Arg Leu Arg Trp Ile Asn Tyr Leu Arg Pro Asp
       50                  55                  60
   ctc aag agg ggt aac ttc acc ctc gaa gaa gat gat ctc atc atc aaa     240
   Leu Lys Arg Gly Asn Phe Thr Leu Glu Glu Asp Asp Leu Ile Ile Lys
   65                  70                  75                  80
   cta cat agc ctt ctc ggt aac aag tgg tct ctt att gcg acg aga tta     288
   Leu His Ser Leu Leu Gly Asn Lys Trp Ser Leu Ile Ala Thr Arg Leu
                   85                  90                  95
   cca gga aga aca gat aac gag att aag aat tac tgg aac aca cat gtt     336
   Pro Gly Arg Thr Asp Asn Glu Ile Lys Asn Tyr Trp Asn Thr His Val
               100                 105                 110
   aag agg aag cta tta aga aaa ggg att gat ccg gcg act cat cga cct     384
   Lys Arg Lys Leu Leu Arg Lys Gly Ile Asp Pro Ala Thr His Arg Pro
           115                 120                 125
   atc aac gag acc aaa act tct caa gat tcg tct gat tct agt aaa aca     432
   Ile Asn Glu Thr Lys Thr Ser Gln Asp Ser Ser Asp Ser Ser Lys Thr
       130                 135                 140
   gag gac cct ctt gtc aag att ctc tct ttt ggt cct cag ctg gag aaa     480
   Glu Asp Pro Leu Val Lys Ile Leu Ser Phe Gly Pro Gln Leu Glu Lys
   145                 150                 155                 160
   ata gca aat ttc ggg gac gag aga att caa aag aga gtt gag tac tca     528
   Ile Ala Asn Phe Gly Asp Glu Arg Ile Gln Lys Arg Val Glu Tyr Ser
                   165                 170                 175
   gtt gtt gaa gaa aga tgt ctg gac ttg aat ctt gag ctt agg atc agt        576
   Val Val Glu Glu Arg Cys Leu Asp Leu Asn Leu Glu Leu Arg Ile Ser
               180                 185                 190
   cca cca tgg caa gac aag ctc cat gat gag agg aac cta agg ttt ggg        624
   Pro Pro Trp Gln Asp Lys Leu His Asp Glu Arg Asn Leu Arg Phe Gly
           195                 200                 205
   aga gtg aag tat agg tgc agt gcg tgc cgt ttt gga ttc ggg aac ggc        672
   Arg Val Lys Tyr Arg Cys Ser Ala Cys Arg Phe Gly Phe Gly Asn Gly
       210                 215                 220
   aag gag tgt agc tgt aat aat gtg aaa tgt caa aca gag gac agt agt        720
   Lys Glu Cys Ser Cys Asn Asn Val Lys Cys Gln Thr Glu Asp Ser Ser
   225                 230                 235                 240
   agc agc agt tat tct tca acc gac att agt agt agc att ggt tat gac        768
   Ser Ser Ser Tyr Ser Ser Thr Asp Ile Ser Ser Ser Ile Gly Tyr Asp
                   245                 250                 255
   ttc ttg ggt cta aac aac act agg gtt ttg gat ttt agc act ttg gaa        816
   Phe Leu Gly Leu Asn Asn Thr Arg Val Leu Asp Phe Ser Thr Leu Glu
               260                 265                 270
   atg aaa tga                                                            825
   Met Lys
<210> 44
   <211> 274
   <212> PRT
   <213> Arabidopsis thaliana
<400> 44
   Met Gly Arg Ser Pro Cys Cys Glu Lys Asp His Thr Asn Lys Gly Ala
   1               5                   10                  15
   Trp Thr Lys Glu Glu Asp Asp Lys Leu Ile Ser Tyr Ile Lys Ala His
               20                  25                  30
   Gly Glu Gly Cys Trp Arg Ser Leu Pro Arg Ser Ala Gly Leu Gln Arg
           35                  40                  45
   Cys Gly Lys Ser Cys Arg Leu Arg Trp Ile Asn Tyr Leu Arg Pro Asp
       50                  55                  60
   Leu Lys Arg Gly Asn Phe Thr Leu Glu Glu Asp Asp Leu Ile Ile Lys
   65                  70                  75                  80
   Leu His Ser Leu Leu Gly Asn Lys Trp Ser Leu Ile Ala Thr Arg Leu
                   85                  90                  95
   Pro Gly Arg Thr Asp Asn Glu Ile Lys Asn Tyr Trp Asn Thr His Val
               100                 105                 110
   Lys Arg Lys Leu Leu Arg Lys Gly Ile Asp Pro Ala Thr His Arg Pro
           115                 120                 125
   Ile Asn Glu Thr Lys Thr Ser Gln Asp Ser Ser Asp Ser Ser Lys Thr
       130                 135                 140
   Glu Asp Pro Leu Val Lys Ile Leu Ser Phe Gly Pro Gln Leu Glu Lys
   145                 150                 155                 160
   Ile Ala Asn Phe Gly Asp Glu Arg Ile Gln Lys Arg Val Glu Tyr Ser
                   165                 170                 175
   Val Val Glu Glu Arg Cys Leu Asp Leu Asn Leu Glu Leu Arg Ile Ser
               180                 185                 190
   Pro Pro Trp Gln Asp Lys Leu His Asp Glu Arg Asn Leu Arg Phe Gly
           195                 200                 205
   Arg Val Lys Tyr Arg Cys Ser Ala Cys Arg Phe Gly Phe Gly Asn Gly
       210                 215                 220
   Lys Glu Cys Ser Cys Asn Asn Val Lys Cys Gln Thr Glu Asp Ser Ser
   225                 230                 235                 240
   Ser Ser Ser Tyr Ser Ser Thr Asp Ile Ser Ser Ser Ile Gly Tyr Asp
                   245                 250                 255
   Phe Leu Gly Leu Asn Asn Thr Arg Val Leu Asp Phe Ser Thr Leu Glu
               260                 265                 270
   Met Lys
<210> 45
   <211> 918
   <212> DNA
   <213> Arabidopsis thalina
<220>
   <221> CDS
   <222> (30)..(839)
   <223> G255
<400> 45
   agcatcatca tcatcagaag aagagagtc atg gga aga tct cct tgc tgc gag      53
                                   Met Gly Arg Ser Pro Cys Cys Glu
                                   1               5
   aaa gaa cac atg aac aaa ggt gct tgg act aaa gaa gaa gat gag aga     101
   Lys Glu His Met Asn Lys Gly Ala Trp Thr Lys Glu Glu Asp Glu Arg
       10                  15                  20
   cta gtc tct tac atc aag tct cac ggt gaa ggt tgt tgg cga tct ctt     149
   Leu Val Ser Tyr Ile Lys Ser His Gly Glu Gly Cys Trp Arg Ser Leu
   25                  30                  35                  40
   cct aga gcc gct ggt ctc ctt cgc tgc ggt aaa agc tgc cgt ctt cgg     197
   Pro Arg Ala Ala Gly Leu Leu Arg Cys Gly Lys Ser Cys Arg Leu Arg
                   45                  50                  55
   tgg att aac tat ctc cga cct gat ctc aaa aga gga aac ttt aca cat     245
   Trp Ile Asn Tyr Leu Arg Pro Asp Leu Lys Arg Gly Asn Phe Thr His
               60                  65                  70
   gat gaa gat gaa ctt atc atc aag ctt cat agc ctc cta ggc aac aag     293
   Asp Glu Asp Glu Leu Ile Ile Lys Leu His Ser Leu Leu Gly Asn Lys
           75                  80                  85
   tgg tct ttg att gcg gcg aga tta cct gga aga aca gat aac gag atc     341
   Trp Ser Leu Ile Ala Ala Arg Leu Pro Gly Arg Thr Asp Asn Glu Ile
       90                  95                  100
   aag aac tac tgg aac aca cat ata aag agg aag ctt ttg agc aaa ggg     389
   Lys Asn Tyr Trp Asn Thr His Ile Lys Arg Lys Leu Leu Ser Lys Gly
   105                 110                 115                 120
   att gat cca gcc act cat aga ggg atc aac gag gca aaa att tct gat      437
   Ile Asp Pro Ala Thr His Arg Gly Ile Asn Glu Ala Lys Ile Ser Asp
                   125                 130                 135
   ttg aag aaa aca aag gac caa att gta aaa gat gtt tct ttt gtg aca      485
   Leu Lys Lys Thr Lys Asp Gln Ile Val Lys Asp Val Ser Phe Val Thr
               140                 145                 150
   aag ttt gag gaa aca gac aag tct ggg gac cag aag caa aat aag tat      533
   Lys Phe Glu Glu Thr Asp Lys Ser Gly Asp Gln Lys Gln Asn Lys Tyr
           155                 160                 165
   att cga aat ggg tta gtt tgc aaa gaa gag aga gtt gtt gtt gaa gaa      581
   Ile Arg Asn Gly Leu Val Cys Lys Glu Glu Arg Val Val Val Glu Glu
       170                 175                 180
   aaa ata ggc cca gat ttg aat ctt gag ctt agg atc agt cca cca tgg      629
   Lys Ile Gly Pro Asp Leu Asn Leu Glu Leu Arg Ile Ser Pro Pro Trp
   185                 190                 195                 200
   caa aac cag aga gaa ata tct act tgc act gcg tcc cgt ttt tac atg      677
   Gln Asn Gln Arg Glu Ile Ser Thr Cys Thr Ala Ser Arg Phe Tyr Met
                   205                 210                 215
   gaa aac gac atg gag tgt agt agt gaa act gtg aaa tgt caa aca gag      725
   Glu Asn Asp Met Glu Cys Ser Ser Glu Thr Val Lys Cys Gln Thr Glu
               220                 225                 230
   aat agt agc agc att agc tat tct tct att gat att agt agt agt aac      773
   Asn Ser Ser Ser Ile Ser Tyr Ser Ser Ile Asp Ile Ser Ser Ser Asn
           235                 240                 245
   gtt ggt tat gac ttc ttg ggt ttg aag aca aga att ttg gat ttt cga      821
   Val Gly Tyr Asp Phe Leu Gly Leu Lys Thr Arg Ile Leu Asp Phe Arg
       250                 255                 260
   agc ttg gaa atg aaa taa atgaatagta ttagattctt aatttgtagg             869
   Ser Leu Glu Met Lys
   265
   tctgataatg aatgttagat tcgcggccct ctagacaggc ctcgtaccg                918
<210> 46
   <211> 269
   <212> PRT
   <213> Arabidopsis thalina
<400> 46
   Met Gly Arg Ser Pro Cys Cys Glu Lys Glu His Met Asn Lys Gly Ala
   1 5 10 15
   Trp Thr Lys Glu Glu Asp Glu Arg Leu Val Ser Tyr Ile Lys Ser His
   20 25 30
   Gly Glu Gly Cys Trp Arg Ser Leu Pro Arg Ala Ala Gly Leu Leu Arg
   35 40 45
   Cys Gly Lys Ser Cys Arg Leu Arg Trp Ile Asn Tyr Leu Arg Pro Asp
   50 55 60
   Leu Lys Arg Gly Asn Phe Thr His Asp Glu Asp Glu Leu Ile Ile Lys
   65 70 75 80
   Leu His Ser Leu Leu Gly Asn Lys Trp Ser Leu Ile Ala Ala Arg Leu
   85 90 95
   Pro Gly Arg Thr Asp Asn Glu Ile Lys Asn Tyr Trp Asn Thr His Ile
   100 105 110
   Lys Arg Lys Leu Leu Ser Lys Gly Ile Asp Pro Ala Thr His Arg Gly
   115 120 125
   Ile Asn Glu Ala Lys Ile Ser Asp Leu Lys Lys Thr Lys Asp Gln Ile
   130 135 140
   Val Lys Asp Val Ser Phe Val Thr Lys Phe Glu Glu Thr Asp Lys Ser
   145 150 155 160
   Gly Asp Gln Lys Gln Asn Lys Tyr Ile Arg Asn Gly Leu Val Cys Lys
   165 170 175
   Glu Glu Arg Val Val Val Glu Glu Lys Ile Gly Pro Asp Leu Asn Leu
   180 185 190
   Glu Leu Arg Ile Ser Pro Pro Trp Gln Asn Gln Arg Glu Ile Ser Thr
   195 200 205
   Cys Thr Ala Ser Arg Phe Tyr Met Glu Asn Asp Met Glu Cys Ser Ser
   210 215 220
   Glu Thr Val Lys Cys Gln Thr Glu Asn Ser Ser Ser Ile Ser Tyr Ser
   225 230 235 240
   Ser Ile Asp Ile Ser Ser Ser Asn Val Gly Tyr Asp Phe Leu Gly Leu
   245 250 255
   Lys Thr Arg Ile Leu Asp Phe Arg Ser Leu Glu Met Lys
   260 265
<210> 47
   <211> 660
   <212> DNA
   <213> Arabidopsis thaliana
<220>
   <221> CDS
   <222> (48)..(521)
   <223> G1113
<400> 47
   aagctttatc tctttctttc tctcctctct atctttctct cacactc atg ggt ctt         56
                                                        Met Gly Leu
                                                        1
   cca aca gat ttc aaa gag ctt cag att cca ggt tac gta cta aaa aca        104
   Pro Thr Asp Phe Lys Glu Leu Gln Ile Pro Gly Tyr Val Leu Lys Thr
       5                   10                  15
   ctt tac gtc atc ggt ttc ttt aga gac atg gtc gat gct ctt tgt cct        152
   Leu Tyr Val Ile Gly Phe Phe Arg Asp Met Val Asp Ala Leu Cys Pro
   20                  25                  30                  35
   tac atc ggt cta cca agt ttt ctt gac cac aac gag acc tct cga tcc        200
   Tyr Ile Gly Leu Pro Ser Phe Leu Asp His Asn Glu Thr Ser Arg Ser
                   40                  45                  50
   gac ccg acc cga ctc gct ctc tcc acg tca gca act ctt gcc aac gag        248
   Asp Pro Thr Arg Leu Ala Leu Ser Thr Ser Ala Thr Leu Ala Asn Glu
               55                  60                  65
   tta atc ccg gtg gtt cgt ttc tcc gat ctt tta acc gat ccg gaa gat        296
   Leu Ile Pro Val Val Arg Phe Ser Asp Leu Leu Thr Asp Pro Glu Asp
           70                  75                  80
   tgc tgc acg gtt tgc tta tcc gat ttt gta tcc gac gat aag att aga        344
   Cys Cys Thr Val Cys Leu Ser Asp Phe Val Ser Asp Asp Lys Ile Arg
       85                  90                  95
   cag ctg ccg aag tgt gga cac gtg ttt cat cat cgt tgt tta gac cgt        392
   Gln Leu Pro Lys Cys Gly His Val Phe His His Arg Cys Leu Asp Arg
   100                 105                 110                 115
   tgg atc gtt gac tgt aat aag ata acg tgc ccg att tgt cgg aac cgg        440
   Trp Ile Val Asp Cys Asn Lys Ile Thr Cys Pro Ile Cys Arg Asn Arg
                   120                 125                 130
   ttc tta ccg gag gaa aag tcc acg ccg ttt gat tgg ggt act tca gat        488
   Phe Leu Pro Glu Glu Lys Ser Thr Pro Phe Asp Trp Gly Thr Ser Asp
               135                 140                 145
   tgg ttt aga gat gaa gtg gag agt acc aac taa taatgatggt tttactttta      541
   Trp Phe Arg Asp Glu Val Glu Ser Thr Asn
           150                 155
   ctttttactt ttttcacggt aatatttttc tactgtataa ttctttcttc caaactactg      601
   tataattcaa gtataagatt atgtaattgt gtatattagc atcaatcatc tttctttgt       660
<210> 48
   <211> 157
   <212> PRT
   <213> Arabidopsis thaliana
<400> 48
   Met Gly Leu Pro Thr Asp Phe Lys Glu Leu Gln Ile Pro Gly Tyr Val
   1               5                   10                  15
   Leu Lys Thr Leu Tyr Val Ile Gly Phe Phe Arg Asp Met Val Asp Ala
               20                  25                  30
   Leu Cys Pro Tyr Ile Gly Leu Pro Ser Phe Leu Asp His Asn Glu Thr
           35                  40                  45
   Ser Arg Ser Asp Pro Thr Arg Leu Ala Leu Ser Thr Ser Ala Thr Leu
       50                  55                  60
   Ala Asn Glu Leu Ile Pro Val Val Arg Phe Ser Asp Leu Leu Thr Asp
   65                  70                  75                  80
   Pro Glu Asp Cys Cys Thr Val Cys Leu Ser Asp Phe Val Ser Asp Asp
                   85                  90                  95
   Lys Ile Arg Gln Leu Pro Lys Cys Gly His Val Phe His His Arg Cys
               100                 105                 110
   Leu Asp Arg Trp Ile Val Asp Cys Asn Lys Ile Thr Cys Pro Ile Cys
           115                 120                 125
   Arg Asn Arg Phe Leu Pro Glu Glu Lys Ser Thr Pro Phe Asp Trp Gly
       130                 135                 140
   Thr Ser Asp Trp Phe Arg Asp Glu Val Glu Ser Thr Asn
   145                 150                 155
<210> 49
   <211> 1201
   <212> DNA
   <213> Arabidopsis thaliana
<220>
   <221> CDS
   <222> (148)..(996)
   <223> G398
<400> 49
   agaaggtttc tcttgtcctc catacactta gcacaactga taaatctttt gaggtaaaat      60
   cagctttaga tcaaggtttt tctagtcatc tctactcata aagatcaaag cttttgctat     120
   tctcattttc taccaagaga caatatc atg atg atg ggt aaa gag gat ttg ggt     174
                                 Met Met Met Gly Lys Glu Asp Leu Gly
                                 1               5
   tta agt ctt agc ttg gga ttt gca caa aac cat cct ctc cag cta aat       222
   Leu Ser Leu Ser Leu Gly Phe Ala Gln Asn His Pro Leu Gln Leu Asn
   10                  15                  20                  25
   ctt aaa ccc act tct tca cca atg tcc aat ctc cag atg ttt cca tgg       270
   Leu Lys Pro Thr Ser Ser Pro Met Ser Asn Leu Gln Met Phe Pro Trp
                   30                  35                  40
   aac caa acc ctt gtt tct tcc tca gat caa caa aag caa cag ttt ctt       318
   Asn Gln Thr Leu Val Ser Ser Ser Asp Gln Gln Lys Gln Gln Phe Leu
               45                  50                  55
   agg aaa atc gac gtg aac agc ttg cca aca acg gtg gat ttg gaa gag       366
   Arg Lys Ile Asp Val Asn Ser Leu Pro Thr Thr Val Asp Leu Glu Glu
           60                  65                  70
   gag aca gga gtt tcg tct cca aac agt acg atc tcg agc aca gtg agt       414
   Glu Thr Gly Val Ser Ser Pro Asn Ser Thr Ile Ser Ser Thr Val Ser
       75                  80                  85
   gga aag agg agg agt act gaa aga gaa ggt acc tcc ggt ggt ggt tgc       462
   Gly Lys Arg Arg Ser Thr Glu Arg Glu Gly Thr Ser Gly Gly Gly Cys
   90                  95                  100                 105
   gga gat gac ctt gac atc act cta gat aga tct tcc tca cgt gga acc       510
   Gly Asp Asp Leu Asp Ile Thr Leu Asp Arg Ser Ser Ser Arg Gly Thr
                   110                 115                 120
   tcc gat gaa gag gaa gat tac gga ggt gag act tgt agg aag aag ctt       558
   Ser Asp Glu Glu Glu Asp Tyr Gly Gly Glu Thr Cys Arg Lys Lys Leu
               125                 130                 135
   aga cta tcc aaa gat caa tcc gca gtt ctc gaa gac act ttc aaa gag       606
   Arg Leu Ser Lys Asp Gln Ser Ala Val Leu Glu Asp Thr Phe Lys Glu
           140                 145                 150
   cac aat act ctc aat ccc aaa cag aag ctg gct ttg gct aag aag cta       654
   His Asn Thr Leu Asn Pro Lys Gln Lys Leu Ala Leu Ala Lys Lys Leu
       155                 160                 165
   ggt tta aca gca aga caa gtg gaa gtg tgg ttc caa aac aga aga gca       702
   Gly Leu Thr Ala Arg Gln Val Glu Val Trp Phe Gln Asn Arg Arg Ala
   170                 175                 180                 185
   agg aca aag tta aag cag acc gaa gtg gat tgc gag tat ttg aaa aga       750
   Arg Thr Lys Leu Lys Gln Thr Glu Val Asp Cys Glu Tyr Leu Lys Arg
                   190                 195                 200
   tgt gtt gag aaa tta acg gaa gag aat cgg cgg ctc gag aaa gag gca       798
   Cys Val Glu Lys Leu Thr Glu Glu Asn Arg Arg Leu Glu Lys Glu Ala
               205                 210                 215
   gcg gaa cta aga gca tta aag ctt tca ccg cgg ttg tat ggt cag atg      846
   Ala Glu Leu Arg Ala Leu Lys Leu Ser Pro Arg Leu Tyr Gly Gln Met
           220                 225                 230
   agt cca ccg acc aca ctt ttg atg tgt cca tcg tgt gaa cgt gtg gcc      894
   Ser Pro Pro Thr Thr Leu Leu Met Cys Pro Ser Cys Glu Arg Val Ala
       235                 240                 245
   gga cca tcc tca tct aac cac aac cag cga tct gtc tca ttg agt cca      942
   Gly Pro Ser Ser Ser Asn His Asn Gln Arg Ser Val Ser Leu Ser Pro
   250                 255                 260                 265
   tgg ctc caa atg gcc cat ggg tca acc ttt gat gtg atg cgt cct agg      990
   Trp Leu Gln Met Ala His Gly Ser Thr Phe Asp Val Met Arg Pro Arg
                   270                 275                 280
   tct taa ctttaatgct gcttctatgg gttgtgtgtg ggtcattgta ctttttagat     1046
   Ser
   tattgactct cagctaatgt atccttaaaa gcctttttct acttttaaat ttactttaat   1106
   ctaattaaat tagttgtcca tgtcttcttg ataacaaaaa aatttataat tataaaaaaa   1166
   aaaaacagga taaaaaaaaa aaaaaaaaaa aaaaa                              1201
<210> 50
   <211> 282
   <212> PRT
   <213> Arabidopsis thaliana
<400> 50
   Met Met Met Gly Lys Glu Asp Leu Gly Leu Ser Leu Ser Leu Gly Phe
   1               5                   10                  15
   Ala Gln Asn His Pro Leu Gln Leu Asn Leu Lys Pro Thr Ser Ser Pro
               20                  25                  30
   Met Ser Asn Leu Gln Met Phe Pro Trp Asn Gln Thr Leu Val Ser Ser
           35                  40                  45
   Ser Asp Gln Gln Lys Gln Gln Phe Leu Arg Lys Ile Asp Val Asn Ser
       50                  55                  60
   Leu Pro Thr Thr Val Asp Leu Glu Glu Glu Thr Gly Val Ser Ser Pro
   65                  70                  75                  80
   Asn Ser Thr Ile Ser Ser Thr Val Ser Gly Lys Arg Arg Ser Thr Glu
                   85                  90                  95
   Arg Glu Gly Thr Ser Gly Gly Gly Cys Gly Asp Asp Leu Asp Ile Thr
               100                 105                 110
   Leu Asp Arg Ser Ser Ser Arg Gly Thr Ser Asp Glu Glu Glu Asp Tyr
           115                 120                 125
   Gly Gly Glu Thr Cys Arg Lys Lys Leu Arg Leu Ser Lys Asp Gln Ser
       130                 135                 140
   Ala Val Leu Glu Asp Thr Phe Lys Glu His Asn Thr Leu Asn Pro Lys
   145                 150                 155                 160
   Gln Lys Leu Ala Leu Ala Lys Lys Leu Gly Leu Thr Ala Arg Gln Val
                   165                 170                 175
   Glu Val Trp Phe Gln Asn Arg Arg Ala Arg Thr Lys Leu Lys Gln Thr
               180                 185                 190
   Glu Val Asp Cys Glu Tyr Leu Lys Arg Cys Val Glu Lys Leu Thr Glu
           195                 200                 205
   Glu Asn Arg Arg Leu Glu Lys Glu Ala Ala Glu Leu Arg Ala Leu Lys
       210                 215                 220
   Leu Ser Pro Arg Leu Tyr Gly Gln Met Ser Pro Pro Thr Thr Leu Leu
   225                 230                 235                 240
   Met Cys Pro Ser Cys Glu Arg Val Ala Gly Pro Ser Ser Ser Asn His
                   245                 250                 255
   Asn Gln Arg Ser Val Ser Leu Ser Pro Trp Leu Gln Met Ala His Gly
               260                 265                 270
   Ser Thr Phe Asp Val Met Arg Pro Arg Ser
           275                 280
<210> 51
   <211> 937
   <212> DNA
   <213> Arabidopsis thaliana
<220>
   <221> CDS
   <222> (120)..(797)
   <223> G395
<400> 51
   gcacaatccc actatccttc gcaagaccct tcctctatat aaggaagttc atttcatttg      60
   gagaggacac gctgacaagc tgactctagc agatctggta ccgtcgacaa ggaggaaga      119
   atg ccc tta gga gca gct acg gtt gtg gag gag gaa gag gag gag gag      167
   Met Pro Leu Gly Ala Ala Thr Val Val Glu Glu Glu Glu Glu Glu Glu
   1               5                   10                  15
   gaa gcg gtg cct agt atg tca gta tcg ccg ccg gat agt gta acg tcg      215
   Glu Ala Val Pro Ser Met Ser Val Ser Pro Pro Asp Ser Val Thr Ser
               20                  25                  30
   tcg ttt caa ttg gac ttt ggg att aaa agt tat ggt tat gag aga aga      263
   Ser Phe Gln Leu Asp Phe Gly Ile Lys Ser Tyr Gly Tyr Glu Arg Arg
           35                  40                  45
   agc aat aag aga gat att gat gat gaa gtg gag aga tca gcc tca aga      311
   Ser Asn Lys Arg Asp Ile Asp Asp Glu Val Glu Arg Ser Ala Ser Arg
       50                  55                  60
   gcc agc aac gaa gac aac gat gac gag aat gga tcc act agg aag aaa      359
   Ala Ser Asn Glu Asp Asn Asp Asp Glu Asn Gly Ser Thr Arg Lys Lys
   65                  70                  75                  80
   ctt aga ctc tcc aaa gac caa tct gct ttt ctt gaa gac agc ttc aaa      407
   Leu Arg Leu Ser Lys Asp Gln Ser Ala Phe Leu Glu Asp Ser Phe Lys
                   85                  90                  95
   gaa cac agt acc ctt aat cct aaa cag aag att gca ttg gcg aag cag      455
   Glu His Ser Thr Leu Asn Pro Lys Gln Lys Ile Ala Leu Ala Lys Gln
               100                 105                 110
   ttg aat ctt cgt cct cgt cag gtt gaa gtc tgg ttt caa aac aga cga       503
   Leu Asn Leu Arg Pro Arg Gln Val Glu Val Trp Phe Gln Asn Arg Arg
           115                 120                 125
   gcc agg aca aag ctg aag caa acg gaa gtg gac tgt gaa tac cta aag       551
   Ala Arg Thr Lys Leu Lys Gln Thr Glu Val Asp Cys Glu Tyr Leu Lys
       130                 135                 140
   aga tgc tgt gag tca cta acc gaa gaa aac cgg agg ctt caa aaa gag       599
   Arg Cys Cys Glu Ser Leu Thr Glu Glu Asn Arg Arg Leu Gln Lys Glu
   145                 150                 155                 160
   gtt aaa gaa ttg aga acc ttg aag act tcc aca ccc ttt tac atg caa       647
   Val Lys Glu Leu Arg Thr Leu Lys Thr Ser Thr Pro Phe Tyr Met Gln
                   165                 170                 175
   ctt ccg gcc act act ctc act atg tgc cct tct tgt gaa cgt gtt gcc       695
   Leu Pro Ala Thr Thr Leu Thr Met Cys Pro Ser Cys Glu Arg Val Ala
               180                 185                 190
   act tca gca gca cag ccc tcc acg tca gct gcc cac aac ctc tgt ttg       743
   Thr Ser Ala Ala Gln Pro Ser Thr Ser Ala Ala His Asn Leu Cys Leu
           195                 200                 205
   tcc acg tca tca ttg att ccg gtt aag cct cgg ccg gcc aaa caa gtt       791
   Ser Thr Ser Ser Leu Ile Pro Val Lys Pro Arg Pro Ala Lys Gln Val
       210                 215                 220
   tca tga aagcacctgc gaaatacagt ttgagcaaac gggcggccgc tctagacagg        847
   Ser
   225
   cctcgtaccg gatcctctag ctagagcttt cgttcgtatc atcggtttcg acaacgttcg     907
   tcaagttcaa tgacatcagt ttgattgcgc                                      937
<210> 52
   <211> 225
   <212> PRT
   <213> Arabidopsis thaliana
<400> 52
   Met Pro Leu Gly Ala Ala Thr Val Val Glu Glu Glu Glu Glu Glu Glu
   1               5                   10                  15
   Glu Ala Val Pro Ser Met Ser Val Ser Pro Pro Asp Ser Val Thr Ser
               20                  25                  30
   Ser Phe Gln Leu Asp Phe Gly Ile Lys Ser Tyr Gly Tyr Glu Arg Arg
           35                  40                  45
   Ser Asn Lys Arg Asp Ile Asp Asp Glu Val Glu Arg Ser Ala Ser Arg
       50                  55                  60
   Ala Ser Asn Glu Asp Asn Asp Asp Glu Asn Gly Ser Thr Arg Lys Lys
   65                  70                  75                      80
   Leu Arg Leu Ser Lys Asp Gln Ser Ala Phe Leu Glu Asp Ser Phe Lys
                   85                  90                  95
   Glu His Ser Thr Leu Asn Pro Lys Gln Lys Ile Ala Leu Ala Lys Gln
               100                 105                 110
   Leu Asn Leu Arg Pro Arg Gln Val Glu Val Trp Phe Gln Asn Arg Arg
           115                 120                 125
   Ala Arg Thr Lys Leu Lys Gln Thr Glu Val Asp Cys Glu Tyr Leu Lys
       130                 135                 140
   Arg Cys Cys Glu Ser Leu Thr Glu Glu Asn Arg Arg Leu Gln Lys Glu
   145                 150                 155                     160
   Val Lys Glu Leu Arg Thr Leu Lys Thr Ser Thr Pro Phe Tyr Met Gln
                   165                 170                 175
   Leu Pro Ala Thr Thr Leu Thr Met Cys Pro Ser Cys Glu Arg Val Ala
               180                 185                 190
   Thr Ser Ala Ala Gln Pro Ser Thr Ser Ala Ala His Asn Leu Cys Leu
           195                 200                 205
   Ser Thr Ser Ser Leu Ile Pro Val Lys Pro Arg Pro Ala Lys Gln Val
       210                 215                 220
   Ser
   225
<210> 53
   <211> 927
   <212> DNA
   <213> Arabidopsis thaliana
<220>
   <221> CDS
   <222> (37)..(861)
   <223> G393
<400> 53
   aaattttaaa ggcatatttt tttcatttcg actcag atg ggt ttt gat gat aca      54
                                           Met Gly Phe Asp Asp Thr
                                           1               5
   tgc aac aca ggt ctt gtt ctt gga tta ggt ccc tca cca att tca aat     102
   Cys Asn Thr Gly Leu Val Leu Gly Leu Gly Pro Ser Pro Ile Ser Asn
               10                  15                  20
   aat tac aat agt acc atc aga caa tcc tcc gtt tac aag ctc gag ccg     150
   Asn Tyr Asn Ser Thr Ile Arg Gln Ser Ser Val Tyr Lys Leu Glu Pro
           25                  30                  35
   tcg ttg act cta tgc ctc tcg ggc gat ccc tcg gtt acc gtg gtg acc     198
   Ser Leu Thr Leu Cys Leu Ser Gly Asp Pro Ser Val Thr Val Val Thr
       40                  45                  50
   gga gct gac cag cta tgc cgt cag acg tca tct cac agc gga gtc tct     246
   Gly Ala Asp Gln Leu Cys Arg Gln Thr Ser Ser His Ser Gly Val Ser
   55                  60                  65                  70
   tct ttc tca agc ggg agg gtg gtg aaa aga gag aga gac ggt ggc gaa     294
   Ser Phe Ser Ser Gly Arg Val Val Lys Arg Glu Arg Asp Gly Gly Glu
                   75                  80                  85
   gag tcg ccg gag gag gaa gag atg acg gag aga gtt ata agt gat tac     342
   Glu Ser Pro Glu Glu Glu Glu Met Thr Glu Arg Val Ile Ser Asp Tyr
               90                  95                  100
   cat gaa gat gaa gaa ggt att agt gct aga aaa aaa ctt agg ctt acg     390
   His Glu Asp Glu Glu Gly Ile Ser Ala Arg Lys Lys Leu Arg Leu Thr
           105                 110                 115
   aaa caa caa tct gct ctt ctt gag gaa agc ttc aag gat cat agc acc     438
   Lys Gln Gln Ser Ala Leu Leu Glu Glu Ser Phe Lys Asp His Ser Thr
       120                 125                 130
   ctt aat ccc aaa caa aag caa gtt ctg gct aga cag ctg aat cta agg      486
   Leu Asn Pro Lys Gln Lys Gln Val Leu Ala Arg Gln Leu Asn Leu Arg
   135                 140                 145                 150
   cct aga caa gtt gaa gta tgg ttt caa aat aga aga gcc agg aca aag      534
   Pro Arg Gln Val Glu Val Trp Phe Gln Asn Arg Arg Ala Arg Thr Lys
                   155                 160                 165
   ctg aag caa aca gaa gta gat tgt gag ttt ttg aag aag tgt tgt gaa      582
   Leu Lys Gln Thr Glu Val Asp Cys Glu Phe Leu Lys Lys Cys Cys Glu
               170                 175                 180
   aca tta gca gat gag aac ata aga ctt cag aaa gag att caa gaa ctc      630
   Thr Leu Ala Asp Glu Asn Ile Arg Leu Gln Lys Glu Ile Gln Glu Leu
           185                 190                 195
   aaa acc cta aaa ttg act cag ccc ttt tac atg cac atg cct gca tcg      678
   Lys Thr Leu Lys Leu Thr Gln Pro Phe Tyr Met His Met Pro Ala Ser
       200                 205                 210
   act cta acg aag tgt cct tct tgt gag aga atc ggc ggc ggc ggc ggg      726
   Thr Leu Thr Lys Cys Pro Ser Cys Glu Arg Ile Gly Gly Gly Gly Gly
   215                 220                 225                 230
   ggt aat gga gga gga ggt ggc ggc agc ggg gct acc gcg gtg att gta      774
   Gly Asn Gly Gly Gly Gly Gly Gly Ser Gly Ala Thr Ala Val Ile Val
                   235                 240                 245
   gat gga agt acg gcc aaa gga gct ttc tct atc tcc tca aag cct cac      822
   Asp Gly Ser Thr Ala Lys Gly Ala Phe Ser Ile Ser Ser Lys Pro His
               250                 255                 260
   ttc ttc aac cct ttt act aac cca tct gca gct tgt tga atagttaatt       871
   Phe Phe Asn Pro Phe Thr Asn Pro Ser Ala Ala Cys
           265                 270
   cgtttaattt tattacttaa aatattaatt ttcttttttt ttttgggtgg catttt        927
<210> 54
   <211> 274
   <212> PRT
   <213> Arabidopsis thaliana
<400> 54
   Met Gly Phe Asp Asp Thr Cys Asn Thr Gly Leu Val Leu Gly Leu Gly
   1               5                   10                  15
   Pro Ser Pro Ile Ser Asn Asn Tyr Asn Ser Thr Ile Arg Gln Ser Ser
               20                  25                  30
   Val Tyr Lys Leu Glu Pro Ser Leu Thr Leu Cys Leu Ser Gly Asp Pro
           35                  40                  45
   Ser Val Thr Val Val Thr Gly Ala Asp Gln Leu Cys Arg Gln Thr Ser
       50                  55                  60
   Ser His Ser Gly Val Ser Ser Phe Ser Ser Gly Arg Val Val Lys Arg
   65                  70                  75                  80
   Glu Arg Asp Gly Gly Glu Glu Ser Pro Glu Glu Glu Glu Met Thr Glu
                   85                  90                  95
   Arg Val Ile Ser Asp Tyr His Glu Asp Glu Glu Gly Ile Ser Ala Arg
               100                 105                 110
   Lys Lys Leu Arg Leu Thr Lys Gln Gln Ser Ala Leu Leu Glu Glu Ser
           115                 120                 125
   Phe Lys Asp His Ser Thr Leu Asn Pro Lys Gln Lys Gln Val Leu Ala
       130                 135                 140
   Arg Gln Leu Asn Leu Arg Pro Arg Gln Val Glu Val Trp Phe Gln Asn
   145                 150                 155                 160
   Arg Arg Ala Arg Thr Lys Leu Lys Gln Thr Glu Val Asp Cys Glu Phe
                   165                 170                 175
   Leu Lys Lys Cys Cys Glu Thr Leu Ala Asp Glu Asn Ile Arg Leu Gln
               180                 185                 190
   Lys Glu Ile Gln Glu Leu Lys Thr Leu Lys Leu Thr Gln Pro Phe Tyr
           195                 200                 205
   Met His Met Pro Ala Ser Thr Leu Thr Lys Cys Pro Ser Cys Glu Arg
       210                 215                 220
   Ile Gly Gly Gly Gly Gly Gly Asn Gly Gly Gly Gly Gly Gly Ser Gly
   225                 230                 235                 240
   Ala Thr Ala Val Ile Val Asp Gly Ser Thr Ala Lys Gly Ala Phe Ser
                   245                 250                 255
   Ile Ser Ser Lys Pro His Phe Phe Asn Pro Phe Thr Asn Pro Ser Ala
               260                 265                 270
   Ala Cys